(19) Europäisches Patentamt
European Patent Office
Office européen des brevets

(11) Publication number: **0 189 596**
**A1**

(12) **EUROPEAN PATENT APPLICATION**

(21) Application number: 85116616.5

(22) Date of filing: 27.12.85

(51) Int. Cl.[4]: C 07 D 307/80
C 07 D 307/85, C 07 D 333/52
C 07 D 405/06, C 07 D 407/06
C 07 D 411/06, C 07 D 417/06
A 61 K 31/34, A 61 K 31/38

(30) Priority: 28.12.84 JP 281269/84

(43) Date of publication of application:
06.08.86 Bulletin 86/32

(84) Designated Contracting States:
BE CH DE FR IT LI NL SE

(71) Applicant: SHIONOGI & CO., LTD.
12, Dosho-machi 3-chome Higashi-ku
Osaka 541(JP)

(72) Inventor: Maeda, Ryozo
4-4-14, Harinakano Higashisumiyoshi-ku
Osaka-shi Osaka(JP)

(72) Inventor: Osugi, Eiichi
4-8-49, Midoridai
Kawanishi-shi Hyogo(JP)

(72) Inventor: Fukioka, Toshihiro
1-983-6, Isokabe Kashiba-cho
Kitakatsuragi-gun Nara(JP)

(72) Inventor: Yonetani, Yukio
3-5-4, Chiyogaoka
Nara-shi Nara(JP)

(72) Inventor: Nakamura, Masuhisa
1-3-1-707, Nishimidorigaoka
Toyonaka-shi Osak(JP)

(74) Representative: Bruin, Cornelis Willem et al,
Octrooibureau Arnold & Siedsma Isartorplatz 5
D-8000 München 2(DE)

(54) **Benzofuran and benzothiophene derivatives.**

(57) Highly effective diuretic antihypertensives, *i.e.,* benzofuran or benzothiophene derivatives having a substituted propenoyl group at the 5-position, which are classified into loop diuretics with less adverse effects and can be administered orally at a daily dosage of 0.5-200 mg or parenterally of 0.01-50 mg.

EP 0 189 596 A1

# BENZOFURAN AND BENZOTHIOPHENE DERIVATIVES

## BACKGROUND OF THE INVENTION

### Field of the Invention

The present invention relates to novel benzofuran and benzothiophene derivatives having antihypertensive, diuretic, and uricosuric activities.

### Prior Art

All diuretic antihypertensives are classified, by the actions and the structures thereof, to diuretic thiazides, loop diuretics, and potassium-sparing diuretics such as antialdosterone-type compounds. The benzofuran- or benzothiophene-derivatives of the present invention can be reasonably classified into loop diuretics. Followings are the representatives of loop diuretic agents which are clinically used or are under research and development.

Ethacrynic acid: Edecril® (Nippon Merck-Banyu),

Chlorthialidone: Hygroton® (Fujisawa Pharmaceutical Co., Ltd./Ciba-Geigy Japan),

Mefruside: Baycaron® (Yositomi Pharmaceutical Ind.)

Furosemide: Lasix® (Hoechst)

Bumetanide: Lunetoron® (Sankyo Co., Ltd.)

Tienilic acid, or Ticrynafen: U.S. Patent No. 3,758,506 (C.E.R.P.H.A.),

Indacrinone and the derivatives: Japanese Unexamined Patent Publication Nos. 57-163338, 57-163339, 57-176920, 57-176968, 57-209246 (Merck),

Benzenesulfonamide derivatives substituted at 2, 3, and 4

positions: JPN Unexam. Pub. No. 58-124758 (Fujisawa Pharmaceutical Co., Ltd.),

5-Acyl-substituted-2,3-dihydrobenzofuran derivatives: JPN Unexam. Pub. No. 52-10261 (Merck).

The compounds of this invention are classified into the same as the last series of the compounds listed above, *i.e.*, 5-Acyl-substituted-2, 3-dihydrobenzofuran derivatives, in their essential structure, but different in their partial structure.

SUMMARY OF THE INVENTION

This invention provides new diuretic compounds which can be administered orally at a daily-dosage of 0.5-200 mg, preferably 1-100 mg or parenterally of 0.01-50 mg, preferably of 0.1-20 mg, and which have the following formula ( I ):

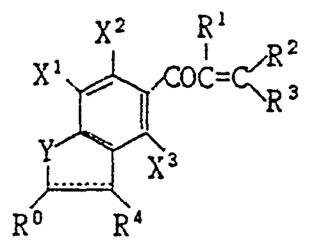

( I )

wherein $X^1$, $X^2$, and $X^3$ each is hydrogen, halogen, or $CH_3$; Y is an atom O or S; $R^1$ is hydrogen, alkyl, alkenyl, aryl, aralkyl, or alkoxycarbonyl; $R^2$ is $SR^5$, $OR^6$, or $NR^7R^8$,

wherein $R^5$ is hydrogen, alkyl, alkenyl, alkynyl, aryl, aralkyl, or carbamoylmethyl, $R^6$ is alkyl, $R^7$ and $R^8$ each is hydrogen, alkyl, alkenyl, cycloalkyl, aryl, furylmethyl, or acyl where $R^7$ and $R^8$ taken together with the adjacent

nitrogen atom may form pyrrolidino, piperidino, or morpholino or one of $R^7$ and $R^8$ is hydrogen and the other is $-C(O)R^{22}$ where $R^{22}$ is alkyl, substituted alkyl, alkylene or substituted alkylene;

$R^3$ is $SR^9$ or $S(O)R^{10}$,

wherein $R^9$ is hydrogen, alkyl, alkenyl, alkynyl, or aralkyl and $R^{10}$ is alkyl;

$R^4$ is hydrogen or alkyl; $R^0$ is CHO, $COCH_3$, $COOCH_2COOH$, CN, CH=NOH, $COOR^{17}$, $CH_2OR^{18}$, $CONR^{19}R^{20}$, or $CH_2OC(O)-CH_2R^{21}$,

wherein $R^{17}$ is hydrogen, alkali metal, or alkyl, $R^{18}$ is hydrogen, alkyl, or acyl, $R^{19}$ and $R^{20}$ each is hydrogen or alkyl, $R^{21}$ is hydrogen or lower alkyl where $R^{19}$ and $R^{20}$ may form pyrrolidino together with the adjacent nitrogen atom;

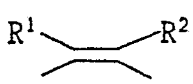 may be 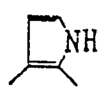 ; and,

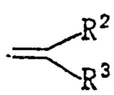 may be any one of the followings:

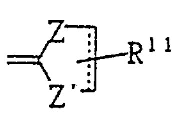 , 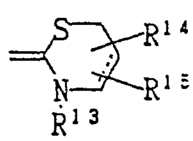 , and 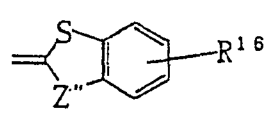

wherein Z is O, S, or NH, Z' is S or $N-R^{12}$, Z" is S, NH, or $N-CH_3$, $R^{11}$ is hydrogen, alkyl, alkoxy, carbonyl, or

methylene, $R^{12}$, $R^{13}$, $R^{14}$, and $R^{16}$ each is hydrogen or alkyl, $R^{15}$ is hydrogen or carbonyl, and the dotted line shows the presence or absence of a double bond.

DESCRIPTION OF THE PREFERRED EMBODIMENTS

The present invention is to provide novel benzofuran and benzothiophene derivatives. Compounds of the present invention, which may be the permissible salts thereof, are represented by the following formula ( I ):

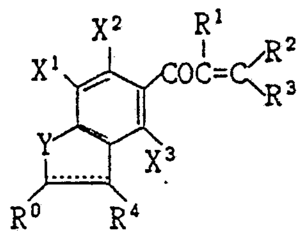

( I )

wherein $X^1$, $X^2$, and $X^3$ each is hydrogen, halogen, or $CH_3$; Y is an atom O or S; $R^1$ is alkyl, alkenyl, aryl, aralkyl, or alkoxycarbonyl; $R^2$ is $SR^5$, $OR^6$, or $NR^7R^8$,

wherein $R^5$ is hydrogen, alkyl, alkenyl, alkynyl, aryl, aralkyl, or carbamoylmethyl, $R^6$ is alkyl, $R^7$ and $R^8$ each is hydrogen, alkyl, alkenyl, cycloalkyl, aryl, furylmethyl, or acyl where $R^7$ and $R^8$ taken together with the adjacent nitrogen atom may form pyrrolidino, piperidino, or morpholino or one of $R^7$ and $R^8$ is hydrogen and the other is $-C(O)R^{22}$ where $R^{22}$ is alkyl, substituted alkyl, alkylene or substituted alkylene;

$R^3$ is $SR^9$ or $S(O)R^{10}$,

having the following formula (Ⅲ):

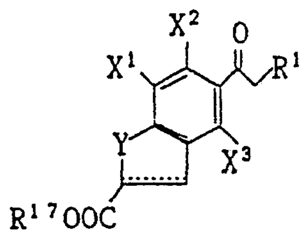

(Ⅲ)

wherein $X^1$, $X^2$, $X^3$, Y, $R^1$, and $R^{17}$ each has the same meaning as defined above, according to the processes explained in the reaction scheme shown later.

Each symbol used in the reaction schemes has the same meaning as defined above.

Abbreviations used in this specification are listed as follows:

| | |
|---|---|
| DMA | Dimethylacetamide |
| DME | Dimethoxyethane |
| DCC | 1,3-Dicyclohexylcarbodiimide |
| THF | Tetrahydrofuran |
| DMSO | Dimethyl sulfoxide |
| DMF | Dimethylformamide |
| p-TsOH | para-Toluenesulfonic acid |
| Me | Methyl |
| Et | Ethyl |
| MeOH | Methanol |
| EtOH | Ethanol |
| $Et_2O$ | Diethyl ether |

wherein $R^9$ is hydrogen, alkyl, alkenyl, alkynyl, or aralkyl and $R^{10}$ is alkyl;

$R^4$ is hydrogen or alkyl; $R^0$ is CHO, $COCH_3$, $COOCH_2COOH$, CN, CH=NOH, $COOR^{17}$, $CH_2OR^{18}$, $CONR^{19}R^{20}$, or $CH_2OC(O)-CH_2R^{21}$,

wherein $R^{17}$ is hydrogen, alkali metal, or alkyl, $R^{18}$ is hydrogen, alkyl, or acyl, $R^{19}$ and $R^{20}$ each is hydrogen or alkyl, $R^{21}$ is hydrogen or lower alkyl where $R^{19}$ and $R^{20}$ may form pyrrolidino together with the adjacent nitrogen atom;

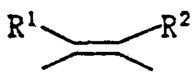 may be 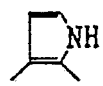 ; and,

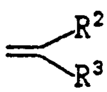 may be any one of the followings:

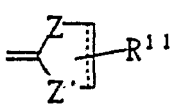 , 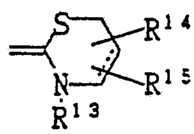 , and 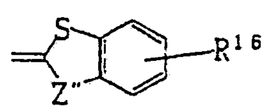

wherein Z is O, S, or NH, Z' is S or N-$R^{12}$, Z" is S, NH, or N-$CH_3$, $R^{11}$ is hydrogen, alkyl, alkoxy, carbonyl, or methylene, $R^{12}$, $R^{13}$, $R^{14}$, and $R^{16}$ each is hydrogen or alkyl, $R^{15}$ is hydrogen or carbonyl, and the dotted line shows the presence or absence of a double bond.

Compounds of the formula ( I ) can be prepared from benzofuran or benzothiophene derivatives as the starting materials

benzoyl. Alkylene or substituted alkylene means $C_2$-$C_4$ alkylene which may be substituted. $C_2$-$C_4$ alkylene includes methylene, ethylene, trimethylene, tetramethylene, and the like. Halogen represented by $X^1$, $X^2$, and $X^3$ includes fluorine, chlorine, bromine, and iodine.

Most of the starting materials used in the following Examples are disclosed in U. S. Patent No. 3,751,436 or J. Med. Chem. *24* (7), 865-873, 1981, or according to which the other can readily be prepared.

ϕ Phenyl

qu. Quantitatively

Compounds of this invention have antihypertensive and diuretic activities and can be used as diuretic antihypertensives in the treatment or prophylaxis of essential or renal hypertension, nephredema, cardiac or hepatic edema, gestosis, or the like diseases.

The compounds of this invention may be administered orally or parenterally (intravenously or intramuscularly) in a suitable form such as tablets, granules, fine granules, powders, capsules, injections or the like formulations. They can be admininstered orally at a single or divided doses of 0.5 - 200 mg a day, preferably 1 - 100 mg; or parenterally at 0.01 - 50 mg, preferably at 0.1 - 20 mg.

In the formula (I), alkyl means straight or branched chain $C_1$-$C_6$ alkyl such as methyl, ethyl, propyl, isopropyl, butyl, s-butyl, isobutyl, pentyl, isopentyl, or the like. Alkenyl means $C_2$-$C_6$ alkenyl such as vinyl 1-propenyl, 2-propenyl, 3-butenyl, 1,4-butadienyl, 3-pentenyl, and the like. Aryl means $C_6$-$C_{12}$ aryl such as phenyl, naphtyl, and the like. Aralkyl means $C_7$-$C_8$ aralkyl, for example, benzyl, phenethyl, and the like. Alkoxycarbonyl means $C_2$-$C_6$ alkoxycarbonyl such as methoxycarbonyl, ethoxycarbonyl, n-propoxycarbonyl, isopropoxycarbonyl, n-butoxycarbonyl, sec-butoxycarbonyl, tert-butoxycarbonyl, and the like. Alkynyl means $C_2$-$C_6$ alkynyl such as ethynyl, 2-propynyl, and the like. Cycloalkyl means $C_3$-$C_7$ cycloalkyl, for example, cyclopropyl, cyclobutyl, cyclopentyl, cyclohexyl, cycloheptyl. Acyl means $C_1$-$C_6$ alkanoyl (e.g. formyl, acetyl, propionyl, butyryl, valeryl, etc.) and

Reaction Scheme 1.
(Example Nos. 1 - 34)

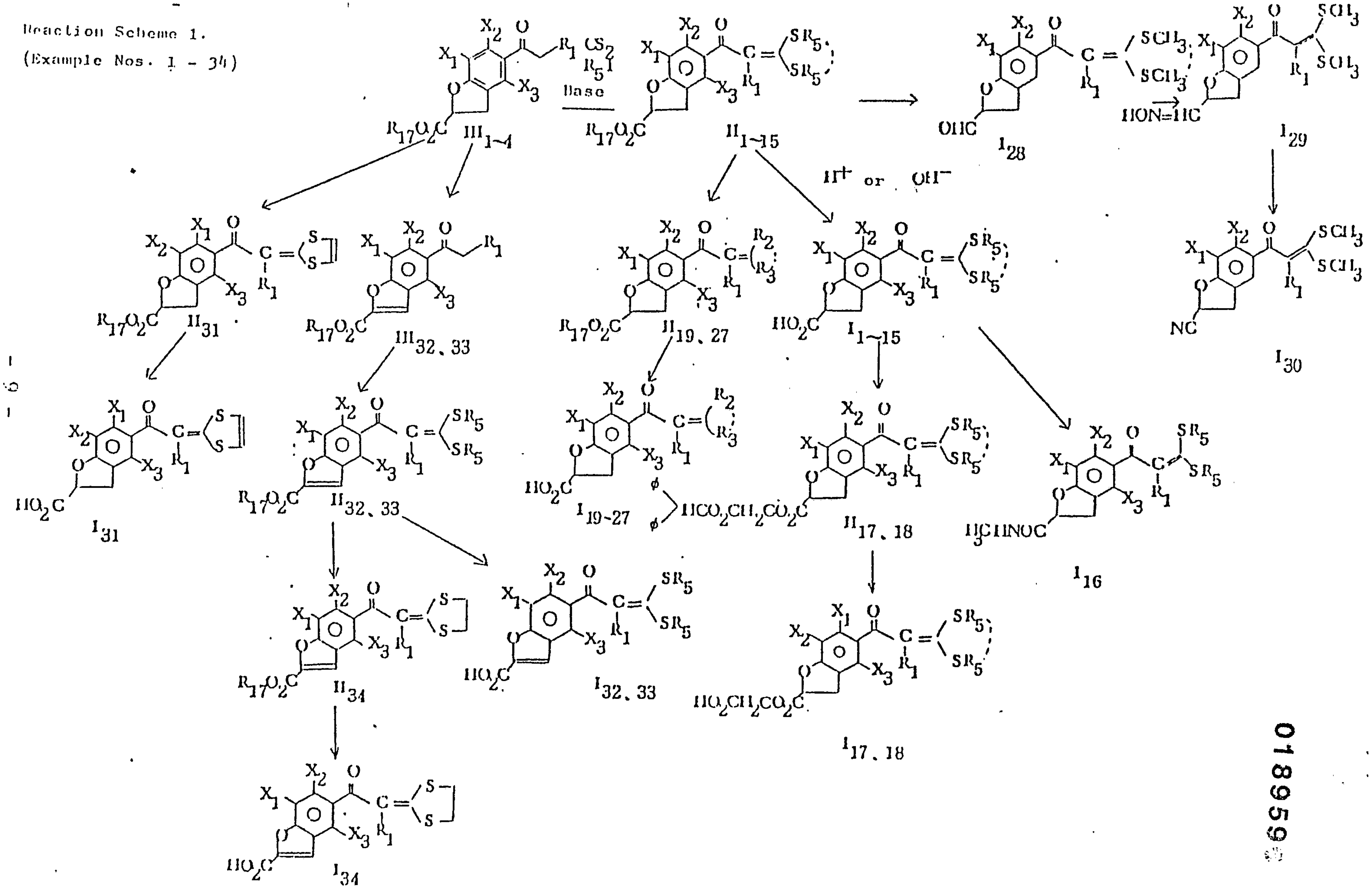

Reaction Scheme 2 (Example Nos. 35-43)

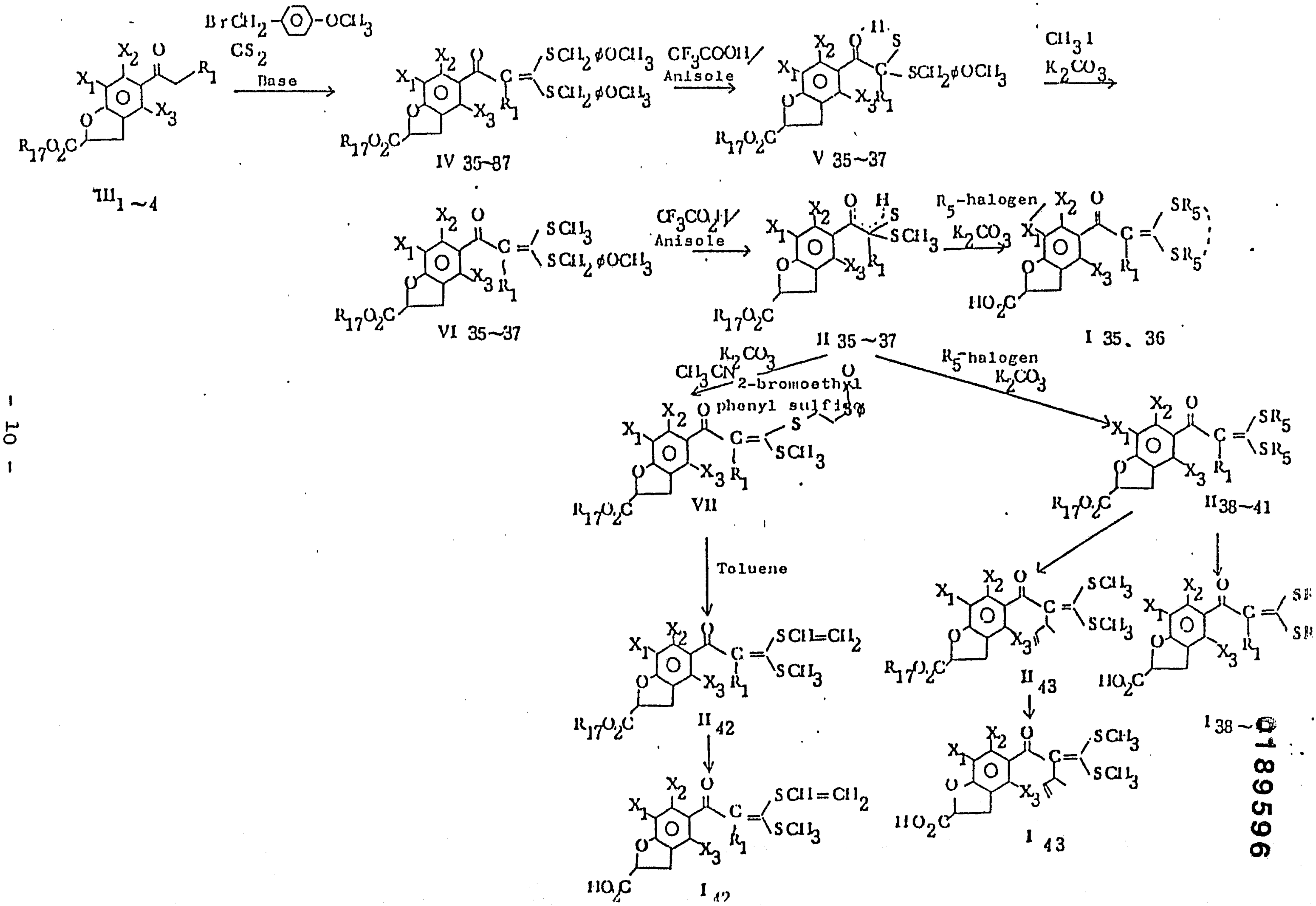


III 1~4

IV 35~87

V 35~37

VI 35~37

II 35~37

I 35, 36

VII

II 38~41

II 42

I 42

II 43

I 43

I 38~41

0189596

Reaction Scheme 3 (Example Nos. 44-50)

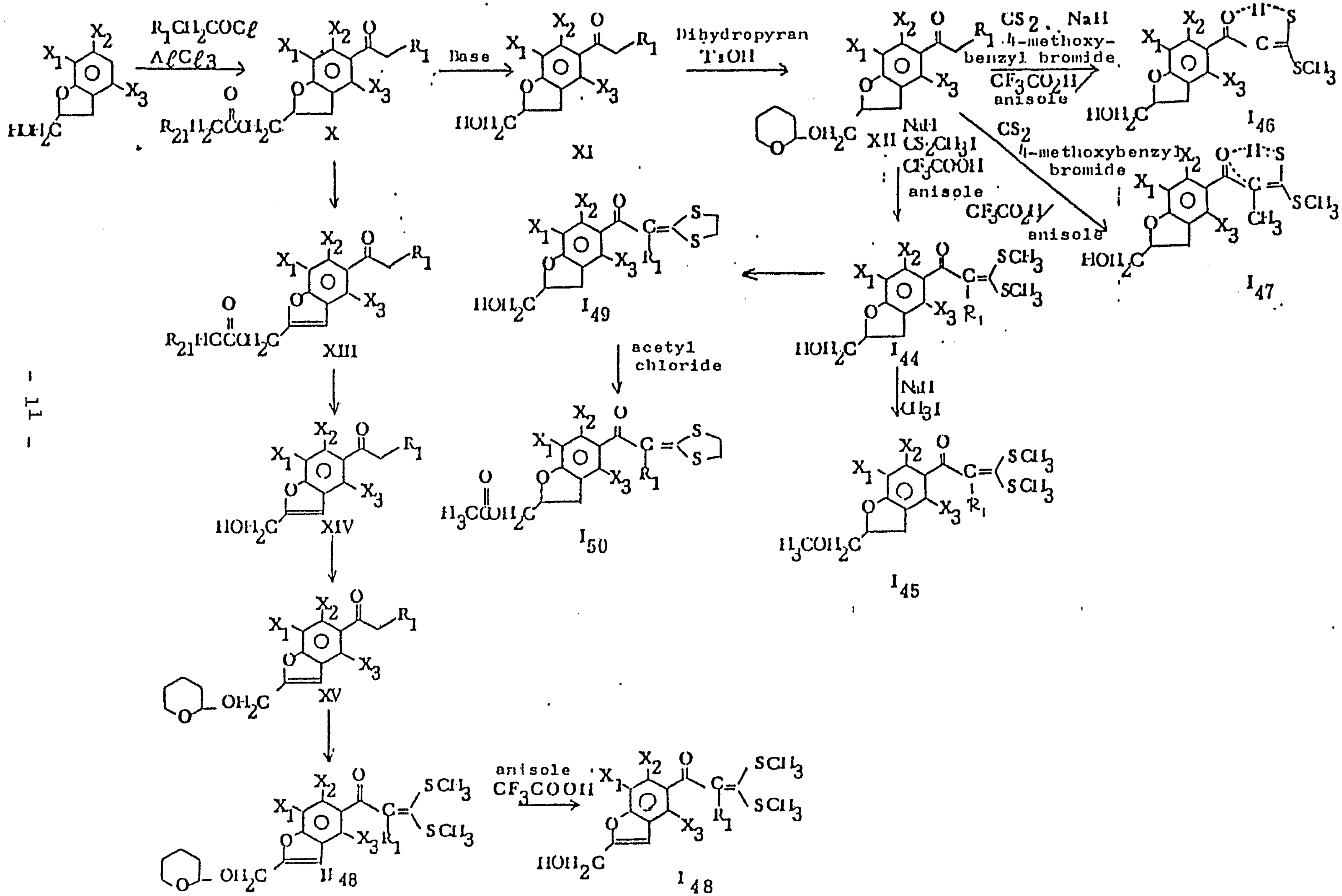

Reaction Scheme 4 (Example Nos. 51-53)

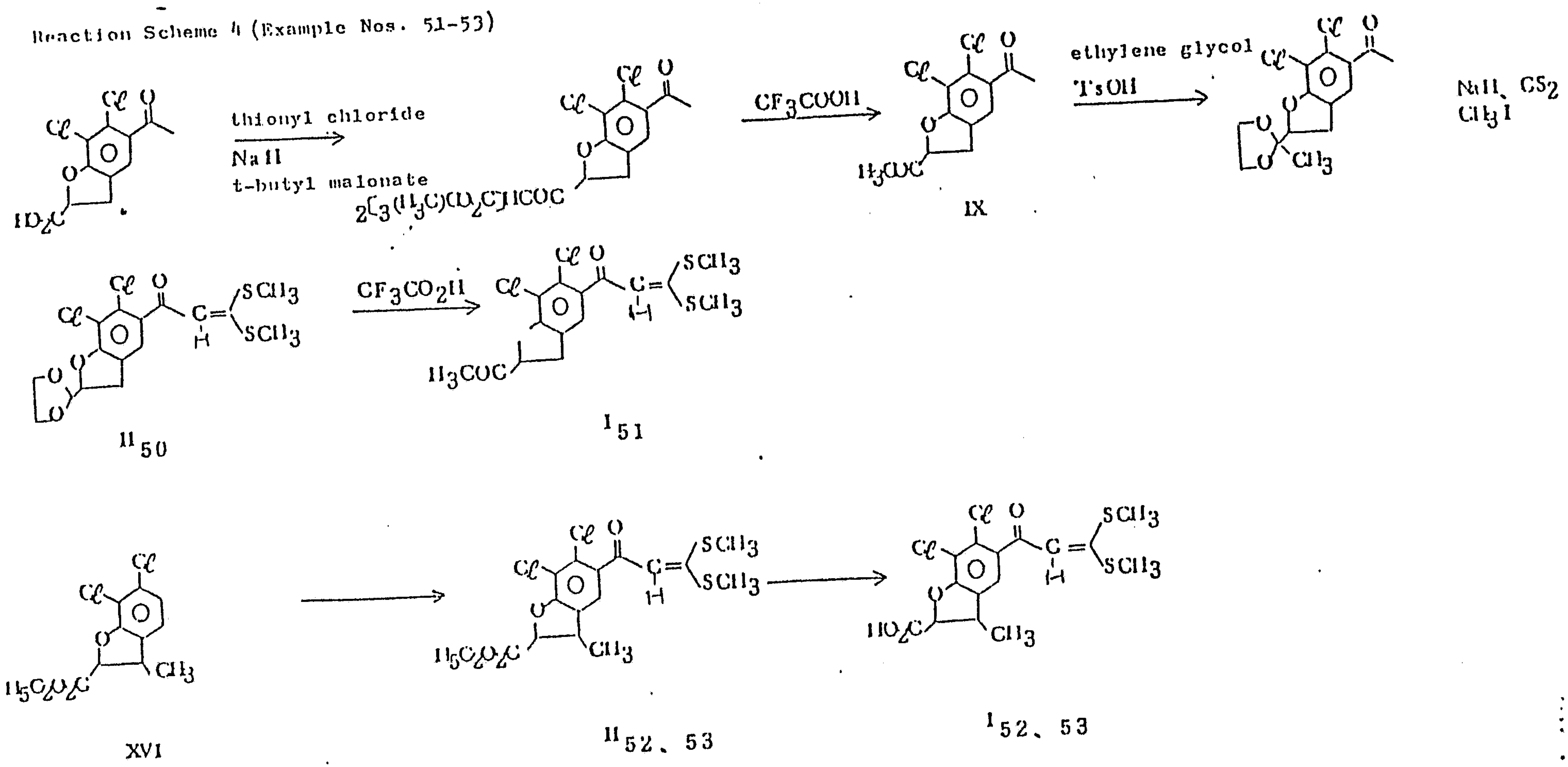


IX

II50

I51

XVI

II52、53

I52、53

0189596

Reaction Scheme 5 (Example No. 54)

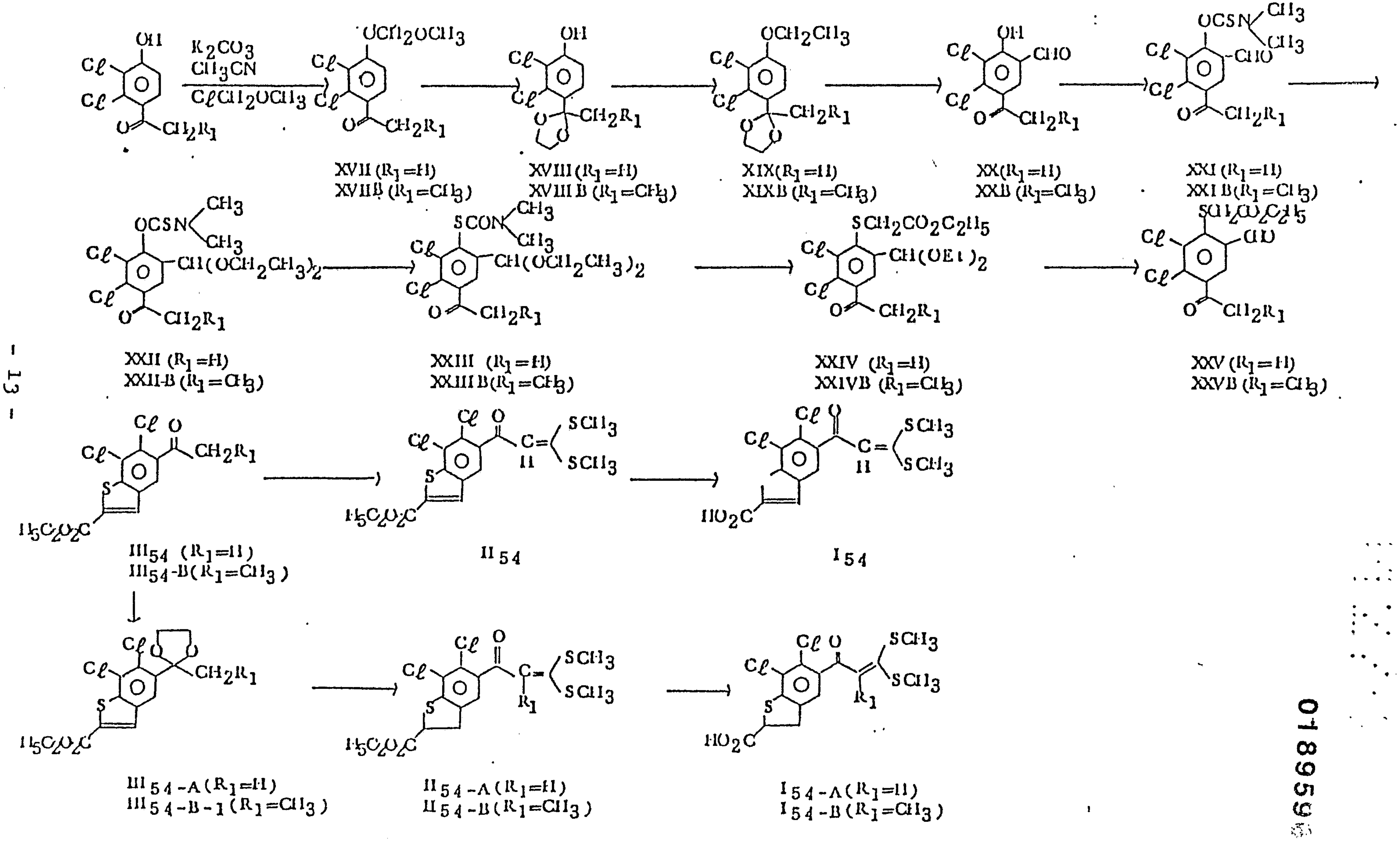


XVII ($R_1$=H)
XVIIB ($R_1$=$CH_3$)

XVIII ($R_1$=H)
XVIII B ($R_1$=$CH_3$)

XIX ($R_1$=H)
XIXB ($R_1$=$CH_3$)

XX ($R_1$=H)
XXB ($R_1$=$CH_3$)

XXI ($R_1$=H)
XXI B ($R_1$=$CH_3$)

XXII ($R_1$=H)
XXII-B ($R_1$=$CH_3$)

XXIII ($R_1$=H)
XXIII B ($R_1$=$CH_3$)

XXIV ($R_1$=H)
XXIVB ($R_1$=$CH_3$)

XXV ($R_1$=H)
XXVB ($R_1$=$CH_3$)

$III_{54}$ ($R_1$=H)
$III_{54}$-B ($R_1$=$CH_3$)

$II_{54}$

$I_{54}$

$III_{54}$-A ($R_1$=H)
$III_{54}$-B-1 ($R_1$=$CH_3$)

$II_{54}$-A ($R_1$=H)
$II_{54}$-B ($R_1$=$CH_3$)

$I_{54}$-A ($R_1$=H)
$I_{54}$-B ($R_1$=$CH_3$)

Reaction Scheme 6 (Example Nos. 55-71)

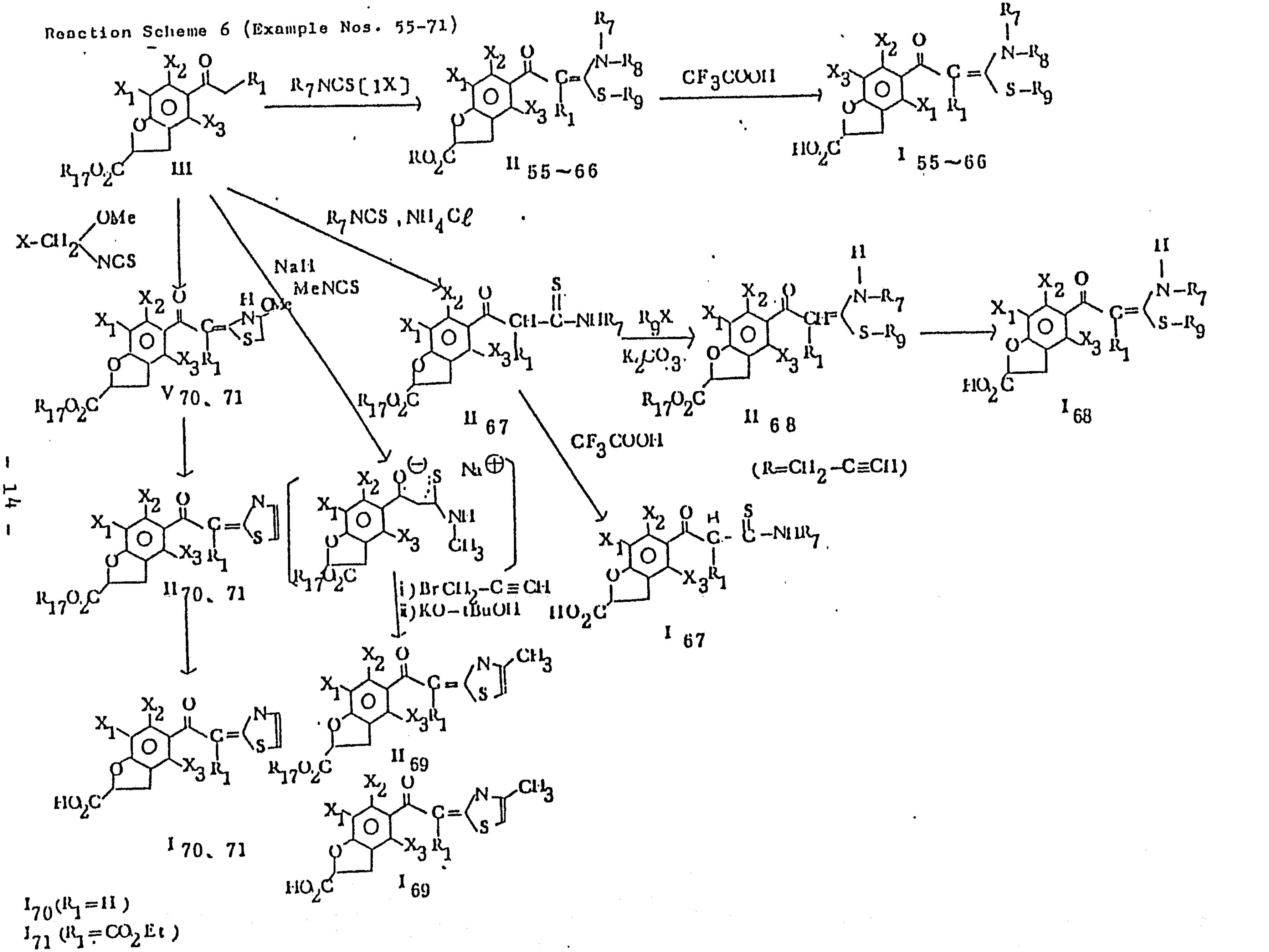

Reaction Scheme 7 (Example No. 72-79)

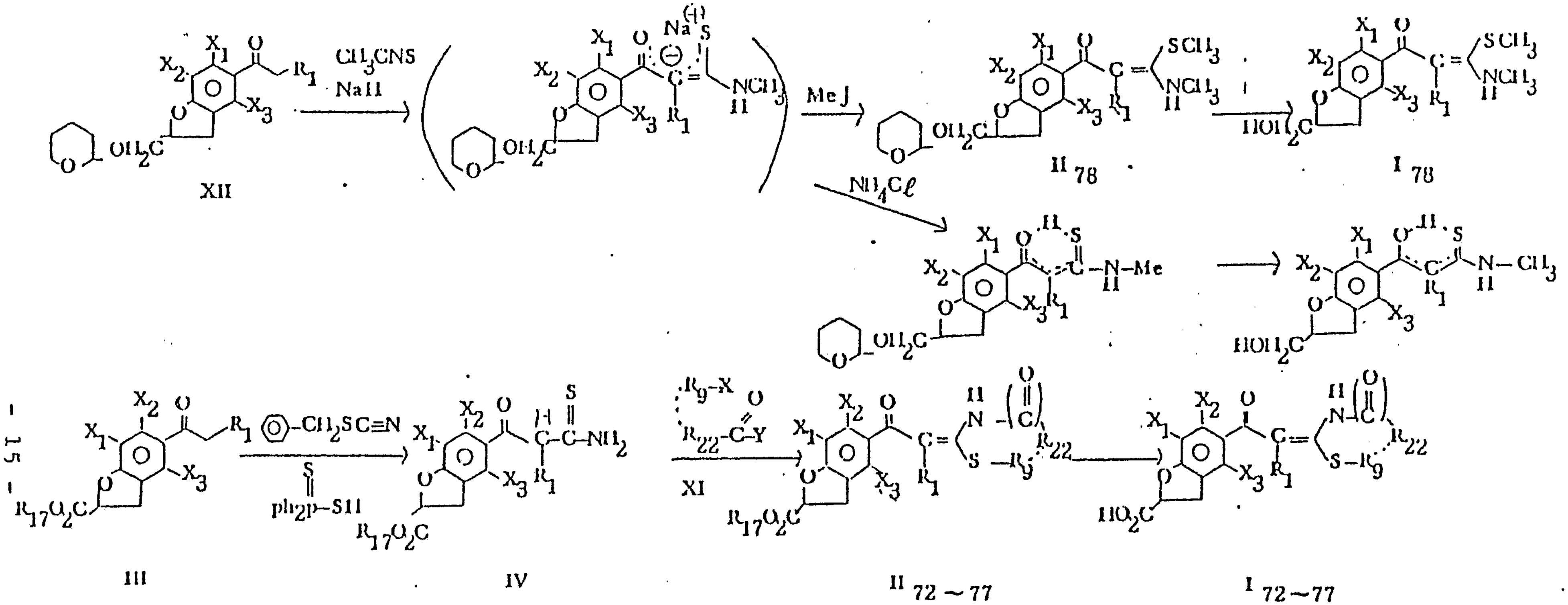


XII

II 78

I 78

III

IV

II 72 ~ 77

I 72 ~ 77

Reaction Scheme 8 (Example No. 80-86)

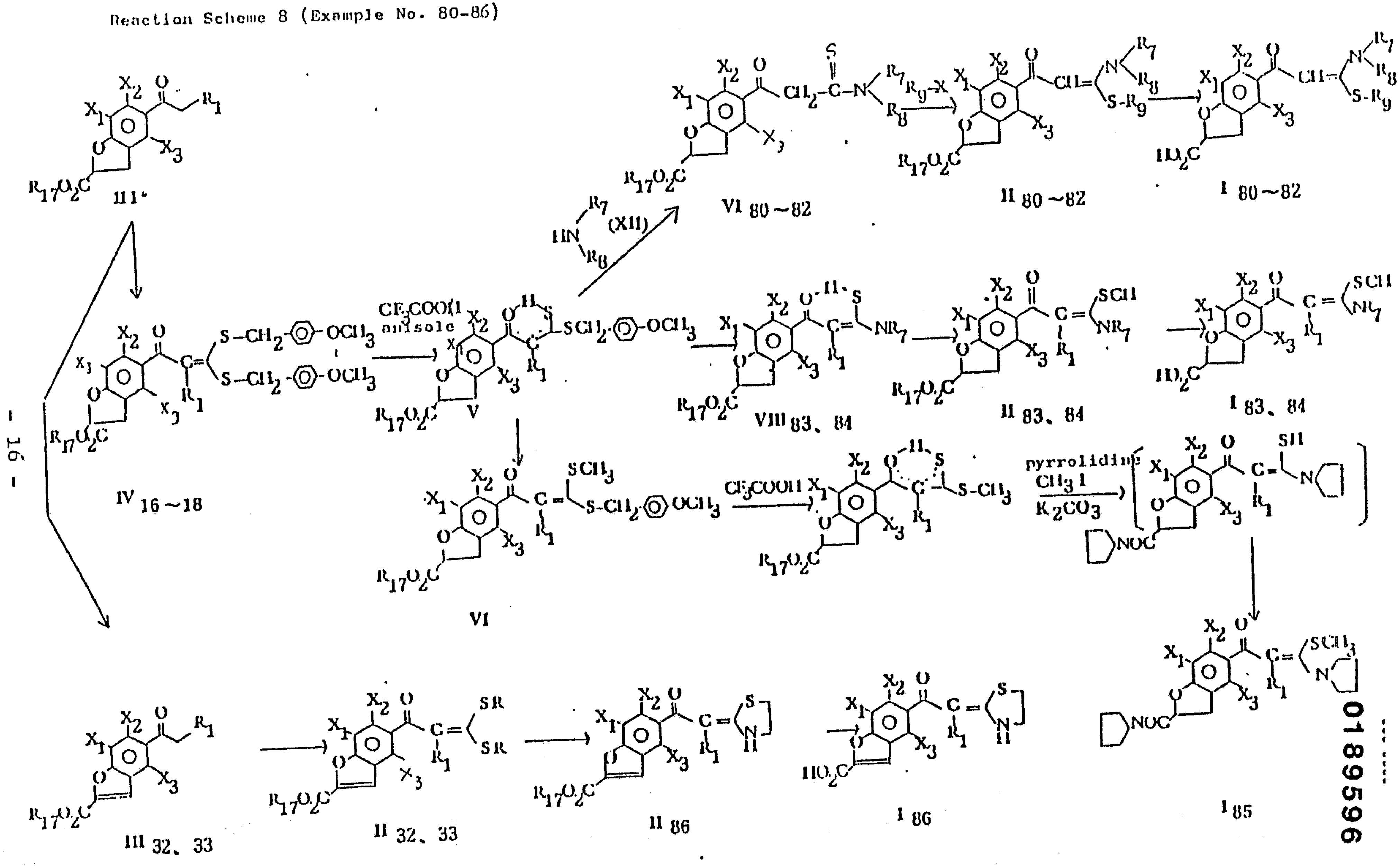


III', IV 16~18, V, VI 80~82, II 80~82, I 80~82, VIII 83、84, II 83、84, I 83、84, VI, I 85, III 32、33, II 32、33, II 86, I 86

Reaction Scheme 9 (Example Nos. 87-98)

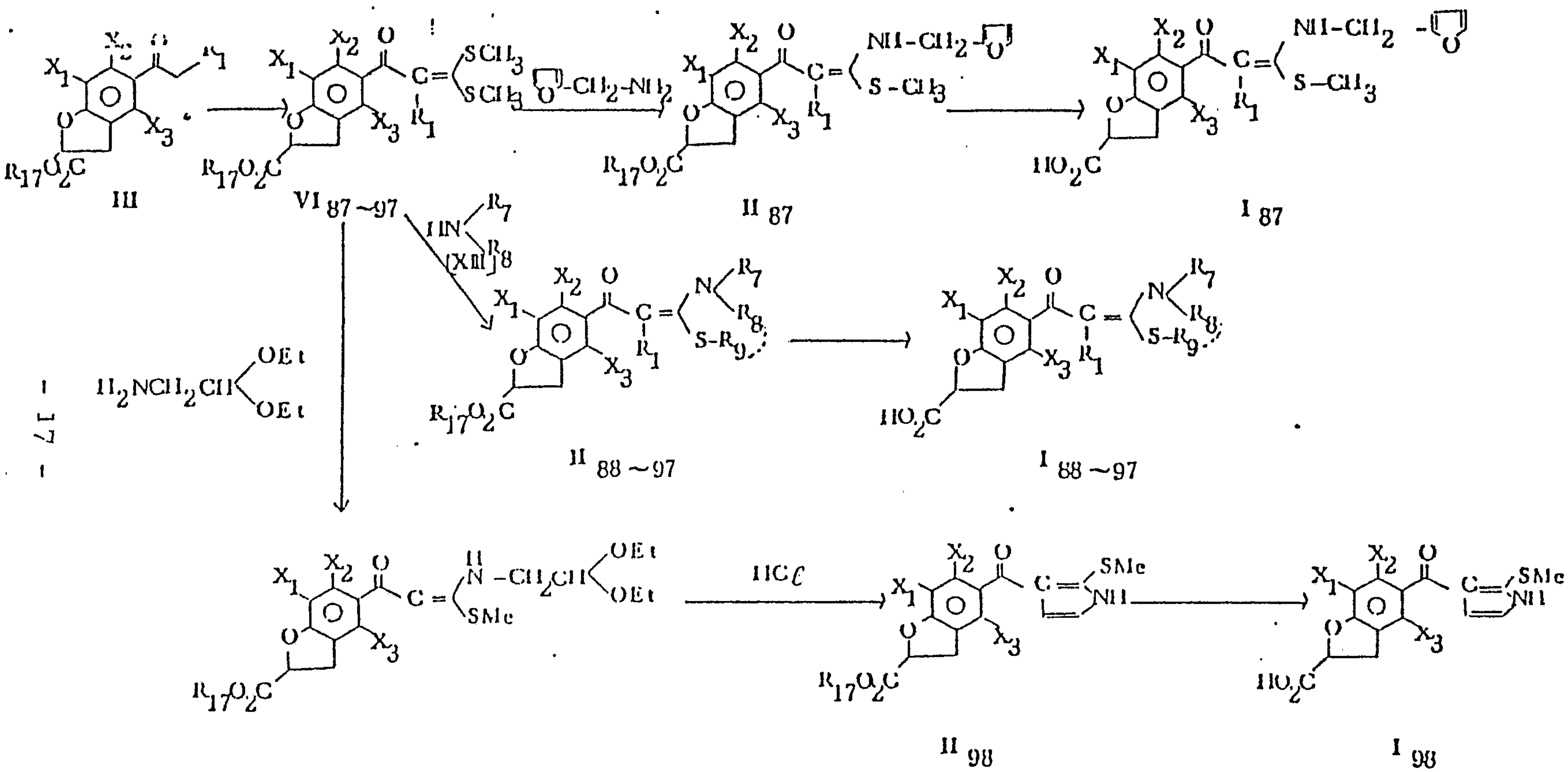


III, VI 87~97, II 87, I 87, II 88~97, I 88~97, II 98, I 98

Reaction Scheme 10 (Example Nos. 99-102)

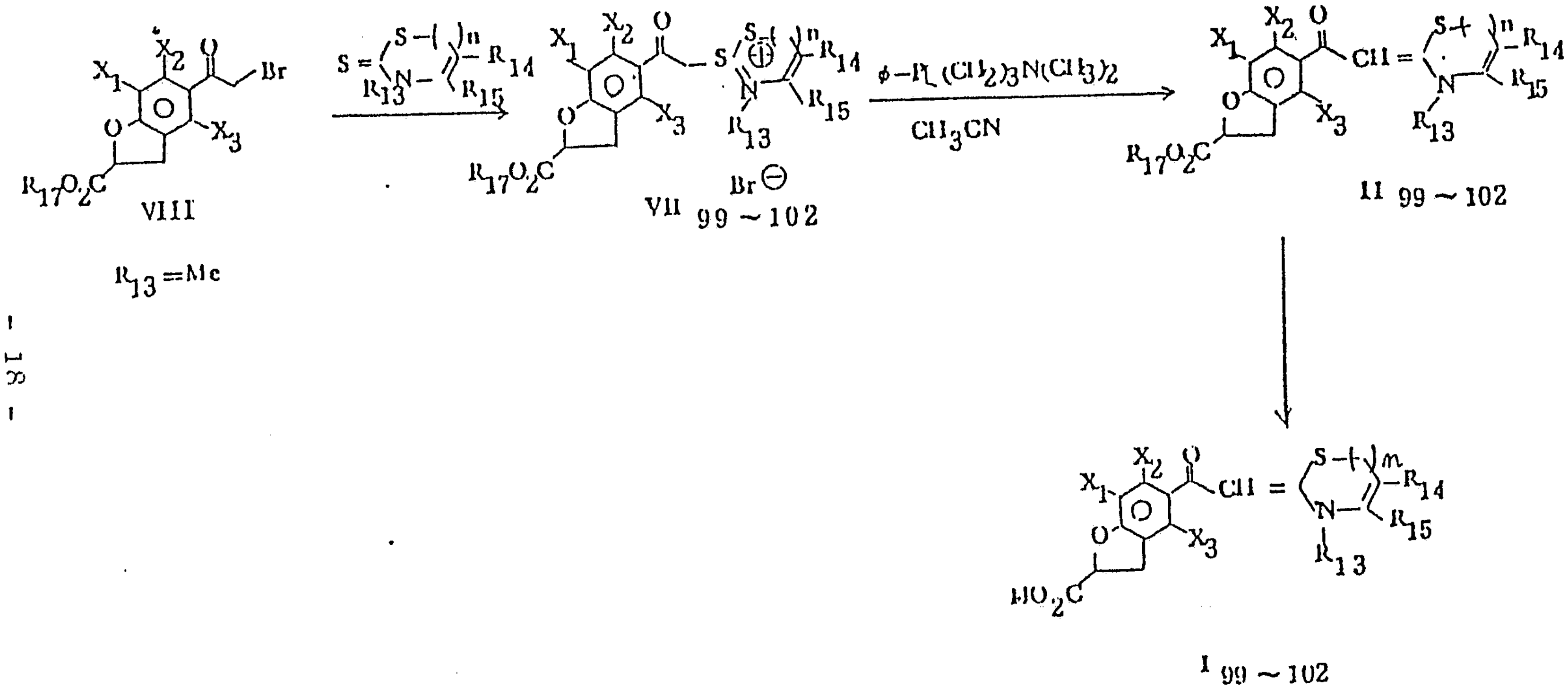


$R_{13}$ = Me

The following examples are provided to illustrate this invention in more detail.

## Example 1

Preparation of 6,7-dichloro-5-[2-methyl-3,3-bis(methylthio)-propanoyl]-2,3-dihydro-1-benzofuran-2-carboxylic acid $I_1$

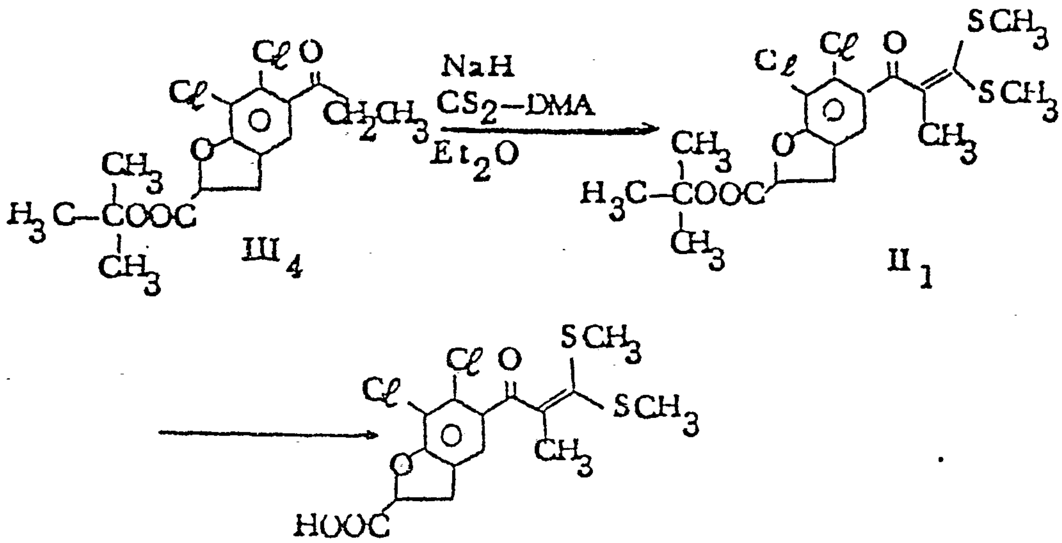


$III_4$ $II_1$

$I_1$

To a suspension of 2.03 g (55.5 mmol) of 65.6 % sodium hydride in 30 ml of dry ether is, under nitrogen flow while being stirred, added a solution of 8.0 g (23.3 mmol) of t-butyl 6,7-dichloro-5-propyonyl-2,3-dihydro-1-benzofuran-2-caboxylate $III_4$, 5.3 g (69.6 mmol) of carbon disulfide and 9.9 g (69.6 mmol) of iodomethane in 190 ml of dry ether, and then 4.8 ml of N,N-dimethylacetamide and the resulting mixture is allowed to react at room temperature for 72 hours. The reaction mixture is poured into ice-cold water and extracted three times with benzene. The benzene layers are combined, washed with water (four times), dried over magnesium sulfate and evaporated to give 13.2 g of a residue. This is chromatographed on a column of 160 g of silica gel (by Merck 70-230 mesh) with n-hexane/benzene (7/3) (F-1, 2 L), n-hexane/benzene (65/35) (F-2, 2 L), n-hexane/benzene (3/2) (F-3, 2 L), n-hexane/benzene (55/45) (F-4, 1 L), n-hexane/benzene (1/1) (F-5, 0.5 L), n-hexane/benzene (3/7) (F-6, 1 L), n-hexane/benzene (1/4) (F-7, 0.8 L), and benzene (F-8, 1 L) in order. From the last three fractions, i.e. F-6, F-7, and F-8 is obtained 9.4 g of compound $II_1$ as an oil, yield 90.3 %.

IR $\nu$ max ($CHCl_3$): 1740 ($C(O)-O-C-(CH_3)_3$), 1640 $cm^{-1}$.

NMR $\delta$ ppm ($CDCl_3$): 1.50 (9H,s), 2.23 (3H, s), 2.00 (3H, s), 2.35 (3H, s), 3.20-3.93 (2H, m), 5.10-5.45 (1H, m), 7.37-7.42 (1H, m).

To a solution of 7.7 g (17.1 mmol) of the compound $II_1$ in 80 ml of dry dichloromethane is added 2.7 g (20.2 mmol) of anhydrous aluminium chloride (powder) under ice-cooling while being stirred and the mixture is allowed to react for an hour and then for additional 2.5 hours at room temperature.

The reaction mixture is poured into ice-cold water, then combined with 6 ml of 10 % hydrochloric acid, and extracted three times with ether. The ether layers are combined, washed with water, dried over magnesium sulfate and evaporated to give 6.8 g of a residue. The residue is treated with a mixture of n-hexane-isopropyl ether to give crystals, mp. 124-128 °C, which are recrystallized from isopropyl ether to give 5.6 g of yellowish white crystals, yield 83.1 %, mp. 131-132 °C.

Anal. Calcd. (%) for $C_{16}H_{14}Cl_2O_4S_2$

: C 45.80 H 3.59, Cl 18.03, S 16.31,

Found (%): C 45.52, H 3.63, Cl 17.91, S 16.25.

IR $\nu$ max (Nujol): 2630, 1724, 1710, 1648, 1605 $cm^{-1}$.

Example 2-15

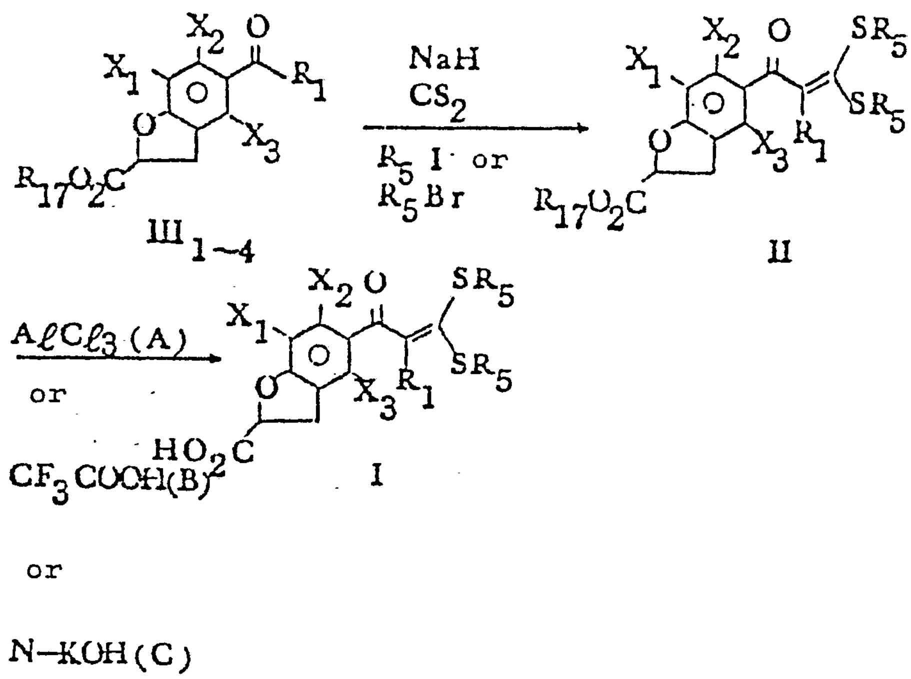


III 1~4 II I

The compounds ( $I_{2-15}$ ) are prepared in the same manner as in Example 1, whose physical constants and reaction conditions are shown in the following Table 1 (Nos. 1 and 2).

Table 1 ( No. 1 )

| Example No. | $R_{17}$ | $R_1$ | $X_1$ | $X_2$ | $X_3$ | III (mmol) | 65.0% NaH (mmol) | $CS_2$ (mmol) | $R_{51}$ or Br (mmol) | Solvent ml $Et_2O$ or Ether | DMA or DME | Temp | Time | $R_5$ | Yield (%) | NMR : $\delta^{CDCl_3}_{ppm}$ |
|---|---|---|---|---|---|---|---|---|---|---|---|---|---|---|---|---|
| 2 | tBu | $CH_3$ | Cl | Cl | H | 2.5 (7.2) | 0.64 (17.5) | 1.66 (21.8) | $(CH_3)_2CHI$ 3.70 (21.8) | Ether 30 | 1.5 | 25 ~ 27℃ | 72 | $(CH_3)_2CH-$ | 7.7 | 100(6H,d)130(6H,d)150(9H,S)227(3H,S)307~393(4H,m)510~540(1H,m)738(1H) |
| 3 | 〃 | 〃 | 〃 | 〃 | 〃 | 1.5 (4.3) | 0.38 (10.4) | 0.99 (13.0) | Br-Br 2.45 (13.0) | 〃 25 | 0.9 | 〃 | 148 | $-(CH_2)_2-$ | 32.9 | 148(9H,S)200(3H,S)307~370(6H,m)503~537(1H,m)692(1H) |
| 4 | 〃 | 〃 | 〃 | 〃 | 〃 | 3.0 (8.7) | 0.76 (20.8) | 2.0 (26.3) | Br-Br 5.30 (26.3) | 〃 30 | 1.8 | 〃 | 96 | $-(CH_2)_3-$ | 7.7 | 150(9H,S)247~200(5H,m)273~393(6H,m)505~543(1H,m)698~708(1H) |
| 5 | $C_2H_5$ | 〃 | 〃 | 〃 | 〃 | 4.0 (12.0) | 1.10 (30.1) | 2.90 (38.1) | $\phi CH_2Br$ 6.50 (38.0) | 〃 40 | 2.6 | 〃 | 48 | $\phi CH_2-$ | 34.7 | 130(t,3H)205(S,3H)317~356(2H,m)380(S,2H)403(2H,S)429(2H,q)517~556(1H,m)687~747(11H) |
| 6 | tBu | H | 〃 | 〃 | 〃 | 4.2 (12.7) | 1.12 (30.6) | 2.90 (38.1) | $CH_3I$ 5.40 (38.2) | 〃 70 | 2.6 | 〃 | 72 | $CH_3-$ | 70.7 | |
| 7 | 〃 | 〃 | 〃 | 〃 | 〃 | 2.4 (7.2) | 0.64 (17.4) | 1.68 (22.1) | EtI 3.40 (21.8) | 36 | 1.5 | 〃 | 72 | $C_2H_5-$ | 81.3 | 137(6H,t)148(9H,S)277~367(6H,m)502~537(1H,m)720(1H) |
| 8 | $C_2H_5$ | $\phi$ | 〃 | 〃 | 〃 | 1.8 (4.7) | 0.42 (11.5) | 1.08 (14.2) | $CH_3I$ 2.02 (14.2) | DME 16 | 0.98 | 〃 | 24 | $CH_3-$ | 72.1 | 127(3H,t)220(6H,S)333~363(2H,m)422(q,2H)513~542(1H,m)712~748(6H) |
| 9 | 〃 | $\phi$-$CH_2$ | 〃 | 〃 | 〃 | 0.3 (0.8) | 0.07 (1.8) | 0.18 (2.3) | $CH_3I$ 0.33 (2.3) | 〃 5 | 0.16 | 〃 | 72 | $CH_3-$ | 24.0 | 128(3H,t)197(3H,S)238(S,3H)307~348(2H,m)375(2H,S)425(2H,q)512~540(1H,m)658(1H)720(5H) |
| 10 | 〃 | 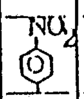 | 〃 | 〃 | 〃 | 1.0 (2.4) | 0.32 (6.0) | 0.56 (7.4) | 1.04 (7.3) | 〃 7 | 0.5 | 〃 | 24 | $CH_3-$ | 84.0 | 130(3H,t)222(3H,S)227(3H,S)313~392(2H,m)425(2H,q)520~547(1H,m)740(1H)757~817(4H,d-d) |
| 11 | | $CH_3$ | H | Cl | H | 0.99 (3.5) | 0.29 (7.7) | 0.67 (8.8) | 1.24 (8.8) | Ether 19 | 0.72 | 〃 | 72 | $CH_3-$ | 17.3 | 130(t,3H)200(3H,S)221(3H,S)234(3H,S)317~373(2H,m)425(2H,q)525(1H,d-d)687(1H,S)752(1H,m) |
| 12 | | $CH_3$ | H | H | Cl | 1.20 (4.2) | 0.34 (9.3) | 0.81 (10.6) | 1.51 (10.6) | 〃 23 | 0.9 | 〃 | 216 | $CH_3$ | 17.6 | 198(3H,S)220(3H,S)233(3H,S)130(3H,t)320~380(2H,m)425(q,2H)525(1H,d-d)677(1H,d)753(1H) |
| 13 | $C_2H_5$ | $CH_3$ | H | H | H | 2.52 (10.2) | 0.82 (22.3) | 1.93 (25.4) | 3.62 (25.4) | 〃 54 | 2.2 | 〃 | 72 | $CH_3$ | 8.4 | 130(t,3H)210(3H,S)217(3H,S)235(3H,S)320~377(2H,m)426(2H,q)525(d-d,1H)71~78(m,2H)690(1H) |
| 14 | $C_2H_5$ | $CH_3$ | $CH_3$ | Cl | H | 1.50 (5.1) | 0.41 (11.1) | 0.96 (12.6) | 1.80 (12.6) | 〃 27 | 1.1 | 〃 | 96 | $CH_3$ | 8.8 | 128(3H,t)197(3H,t)221(3H,S)228(S,3H)233(3H,S)321~375(2H,m)425(2H,q)525(1H,d-d)735(1H,S) |
| 15 | tBut | $CH_3$ | $CH_3$ | $CH_3$ | H | 0.61 (2.1) | 0.185 (4.6) | 0.40 (5.3) | 0.75 (5.3) | THF 10 | 0.4 | 〃 | 72 | $CH_3$ | 37.0 | 148(9H,S)220(3H,S)234(3H,S)(244(S)250(S)6H)310~370(2H,m)508(1H,d-d)635(1H,S)715(1H) |



0189596

Table 1 ( No. 2 )

| Example No. | Amount Used II, y (mmol) | AlCl₃(A) CF₃CO₂H (B) N-KOH(C) | (%) | Recrystal From | m.p. ℃ | Molecular formula | C | H | Cl | S | N | I R | N M R |
|---|---|---|---|---|---|---|---|---|---|---|---|---|---|
| 2 | 0.23 | B | 83.3 | hexane/ isopropyl ether | 121~ 122 | $C_{19}H_{22}Cl_2O_4S_2$ | 50.78<br>50.70 | 4.93<br>4.78 | 15.78<br>15.94 | 14.27<br>14.28 | | 2630, 2540, 1715, 1661, 1611 | |
| 3 | 0.64 | B | 88.4 | acetone | 231~ 232(d) | $C_{15}H_{12}Cl_2O_4S_2$ | 46.04<br>45.89 | 3.09<br>3.05 | 18.12<br>17.85 | 16.39<br>16.12 | | 2650, 2560, 1753, 1710, 1616, 1603 | |
| 4 | 0.55 | B | 82.8 | acetone | 221~ 222 | $C_{16}H_{14}Cl_2S_2O_4$ | 47.41<br>47.15 | 3.48<br>3.60 | 17.50<br>17.39 | 15.82<br>15.62 | | 2680, 2585, 2490, 1750, 1608, 1570 | |
| 5 | 0.70 | C | 49.5 | ether/ isopropyl ether | 128~ 130 | $C_{27}H_{22}Cl_2O_4S_2$ | 59.45<br>59.55 | 4.07<br>3.94 | 13.00<br>13.03 | 11.76<br>11.68 | | 2720, 2645, 2560, 1737, 1712, 1647, 1611 | |
| 6 | 8.80 | A | 83.4 | dioxane/ ether | 258~ 260(d) | $C_{14}H_{12}Cl_2O_4S_2$ | 44.33<br>44.37 | 3.19<br>3.27 | 18.70<br>18.55 | 16.91<br>16.89 | | 2700, 2570, 2480, 1745, 1607, 1560 | [2.48(S) 2.53(S) 6H] 3.10~3.93 (2H, m) 5.31~5.58 (1H, m) 6.40 (1H, S) 7.35 (1H, S) |
| 7 | 2.60 | A | 62.1 | acetone | 175~ 177 | $C_{16}H_{16}Cl_2O_4S_2 \cdot 1/4H_2O$ | 46.66<br>46.60 | 4.02<br>3.94 | 17.22<br>17.40 | 15.57<br>15.57 | | 2680, 2560, 2470, 1750, 1611, 1553 | (1.32; 1.35 6H (×2)) 2.87~3.30 (m, 4H) 3.47~3.80 (m, 2H) 7.30 (1H, S) 5.33~6.00 (2H, m) ($D_2O$ 1H) 6.52 (1H, S) |
| 8 | 1.60 | C | 55.1 | ether/ isopropyl ether | 183~ 185 | $C_{20}H_{16}Cl_2O_4S_2$ | 52.75<br>52.69 | 3.54<br>3.76 | 15.57<br>15.61 | 14.08<br>13.86 | | 2640, 2540, 1735, 1710, 1662, 1605 | |
| 9 | 0.091 (0.2) | C | 84.7 | hexane/ isopropyl ether | 138~ 139 | $C_{21}H_{18}Cl_2O_4S_2$ | 53.73<br>53.67 | 3.81<br>4.02 | 15.11<br>15.25 | 13.66<br>13.53 | | 2660, 2580, 1740, 1713, 1653, 1603 | |
| 10 | 0.165 (0.3) | C | 64.1 | ether/ isopropyl ether | 134~ 136 | $C_{20}H_{15}Cl_2NO_3S_2$ | 48.00<br>47.93 | 3.02<br>3.01 | 14.17<br>13.92 | 12.82<br>12.65 | 2.80<br>2.80 | 3460~3360, 1770, 1739, 1682, 1605, 1514 | |
| 11 | 0.23 | C | 38.0 | isopropyl ether | 129~ 130 | $C_{15}H_{15}ClO_4S_2$ | 50.20<br>50.30 | 4.21<br>4.13 | 9.88<br>9.99 | 17.87<br>17.85 | | 3300~2400, 1714, 1665, 1618, 1580 | 1.98 (S, 3H) 2.18 (3H, S) 2.37 (3H, S) 3.25~3.84 (2H, m) 5.42 (1H, d-d) 6.91 (1H, S) 7.57 (1H) |
| 12 | 0.28 | C | 84.1 | hexane/ isopropyl ether | 96~ 97 | $C_{15}H_{15}ClO_4S_2$ | 50.06 | 4.17 | 9.68 | 17.55 | | 3000~2000, 1740, 1652, 1600, 1590 | 1.97 (3H, S) 2.18 (3H, S) 2.36 (3H, S) 3.26~3.85 (2H, m) 6.50 (1H, S) 6.84 (1H, d) 7.55 (1H) |
| 13 | 0.30 | C | 65.3 | isopropyl ether | 137~ 138 | $C_{15}H_{16}O_4S_2$ | 55.54<br>55.24 | 4.97<br>4.94 | | 19.76<br>19.72 | | 3400~2000, 1737, 1642, 1602, 1438 | 2.07 (S, 6H) 2.34 (3H, S) 3.15~3.80 (2H, m) 5.35 (1H, d-d) 6.94 (1H, d) 7.05~7.20 (2H, m) |
| 14 | 0.17 | C | 51.3 | hexane/ isopropyl ether | 129~ 130 | $C_{16}H_{17}ClO_4S_2$ | 51.54<br>51.45 | 4.59<br>4.52 | 9.50<br>9.61 | 17.19<br>17.12 | | 3300~2400, 1748, 1645, 1592 | |
| 15 | 0.35 | A | 79.7 | ethanol | 246~ 248 | $C_{16}H_{18}O_4S_2$ | 56.78<br>56.49 | 5.36<br>5.22 | | 18.95<br>18.74 | | 3200~2200, 1746, 1562 | |

Example 16

6.7-Dichloro-5-[2-(1,3-dithiolan-2-ylidene)-propyonyl]-2,3-dihydro-1-benzofuran-2-N-methylformamide

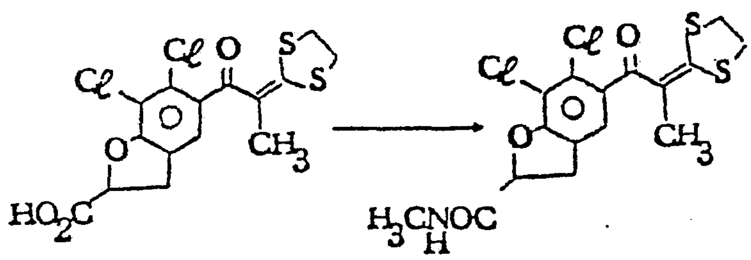

$I_3$ $I_{16}$

In 5 ml of dichloromethane, 0.250 g (0.6 mmol) of the compound($I_3$), 0.138 g (0.7 mmol) of 1,3-dicyclohexyl carbodiimide (D.C.C.) and a large excess amount of methylamine are allowed to react at room temperature for 20 hours. The reaction product is purified by chromatography on a Lober column (Type B) with a mixture of chloroform-benzene-ethyl acetate (3/1/1) to give 0.21 g of the compound ($I_{16}$); yield 46.5 %, mp. 230-233 °C (dec.), which is recrystallized from ethyl acetate to give 0.091 g of grayish crystals, yield 34.9 %, mp. 232-234 °C (dec.).

Anal. Calcd. (%) for $C_{16}H_{15}Cl_2NO_3S_2$

: C 47.53 H 3.74, Cl 17.54, N 3.46, S 15.86,

Found (%): C 47.54, H 3.69, Cl 17.81, N 3.55, S 15.90.

IR $\nu$ max(Nujol): 3315, 3310, 1654, 1615, 1603 $cm^{-1}$.

NMR $\delta$ ppm ($CDCl_3$): 2.00(3H,s), 2.88(3H,d), 3.22-3.73(6H,m), 5.17-5.45(1H,m), 6.60(1H,br), 6.97(1H).

## Example 17

6,7-Dichloro-5-[3,3-bis(methylthio)-2-propenoyl]-2,3-dihydro-1-benzofuran-2-carboxyacetic acid

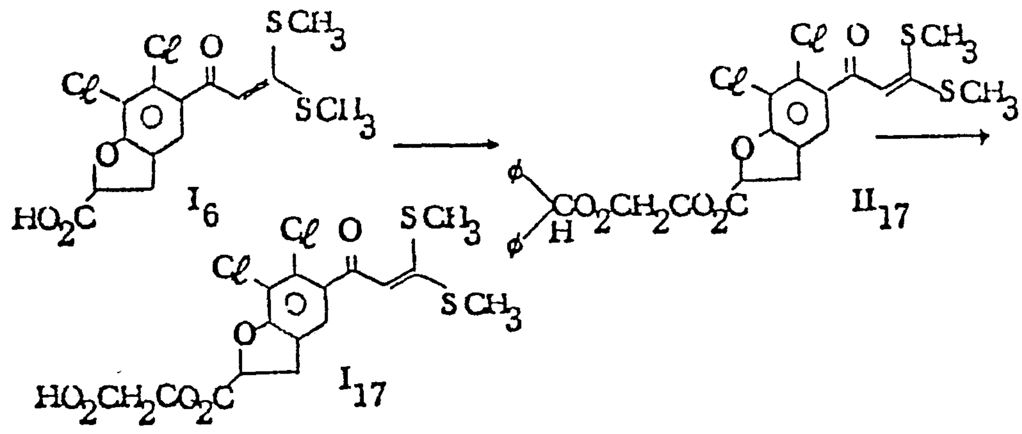

$I_6$ $II_{17}$ $I_{17}$

To 0.44 g (1.2 mmol) of the compound $I_6$ (prepared from Example 6) are added 0.263 g (1.3 mmol) of 1,3-dichlorohexylcarbodiimide and 5 ml of dry dioxane, and the mixture is stirred at room temperature for 2 hours. The mixture is combined with 0.337 g (1.4 mmol) of diphenylmethyl glycolate and allowed to react for further 72 hours. The reaction product is purified by liquid chromatography on a Lober column (Type B) with a mixture of benzene-ethyl acetate (10/1) to give 0.345 g of the compound $II_{17}$, yield 49.3 %.

NMR $\delta$ ppm ($CDCl_3$): 2.47 (3H, s), 2.53 (3H, s), 3.25-3.75 (2H, m), 4.83 (2H, s), 5.30-5.57 (1H, m), 6.43(1H, s), 6.92 (1H, s), 7.22-7.40 (11H, m).

To 0.34 g (0.6 mmol) of the compound $II_{17}$ are added 0.68 ml of anisole and 0.68 ml of trifluoroacetic acid. The mixture is allowed to react at room temperature for 5/6 hours while being stirred. The solvent is removed by evaporation and the residue is treated with n-hexane to give 0.243 g of the compound $I_{17}$, yield 98.8 %, mp. 170-173 °C. This is recrystallized from ether-acetone to give 0.22 g of grayish white crystals, yield 89.4 %, mp. 172-174 °C.

Anal. Calcd. (%) for $C_{16}H_{14}Cl_2O_6S_2$

: C 43.94 H 3.23, Cl 16.22, S 14.66,

Found (%): C 43.78, H 3.38, Cl 15.98, S 14.37.

IR $\nu$ max(Nujol): 3090, 1765, 1742, 1615, 1590 $cm^{-1}$.

Example 18

6,7-Dichloro-5-(2-methyl-1,3-dithiolan-2-ylpropyonyl)-2,3-dihydro-1-benzofuran-2-carboxyacetic acid

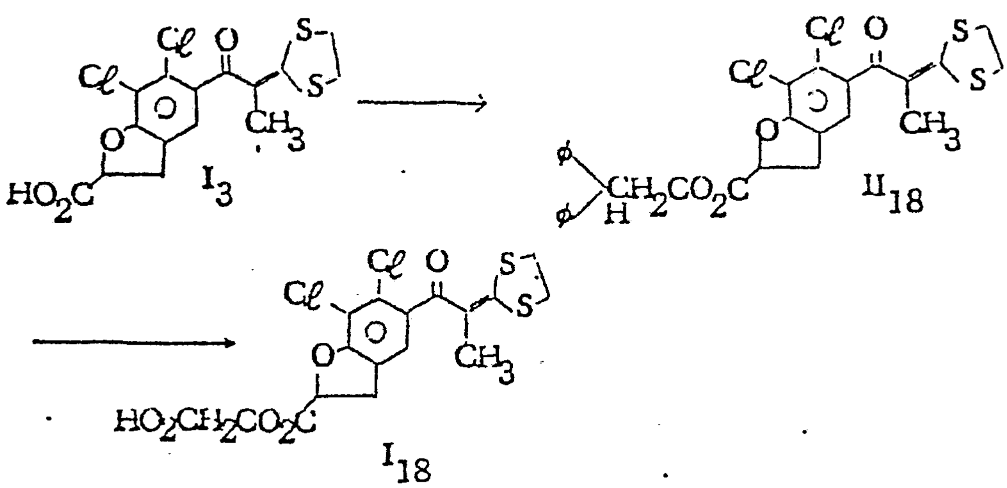

$I_3$ $II_{18}$ $I_{18}$

To 0.50g (1.3 mmol) of the compound $I_3$ (prepared in Example 1) are added 0.277 g (1.3 mmol) of 1,3-dicyclohexylcarbodiimido, 0.60 g (2.5 mmol) of diphenylmethyl glycolate and 5 ml of dioxane, and the mixture is allowed to react and then worked up in the same manner as in Example 17 to give 0.415 g of the compound $II_{18}$, yield 52.8 %.

NMR $\delta$ ppm ($CDCl_3$): 1.95 (3H, s), 3.07-3.67 (6H, m), 4.73 (2H, s), 5.18-5.47 (1H, m), [6.83(s), 6.80 (s) 2H], 7.23 (10H).

A mixture of 0.40 g (0.6 mmol) of the compound $II_{18}$ with 0.8 ml of anisole and 0.8 ml of trifluoroacetic acid is treated in the same manner as in Example 1 to give 0.292 g of the compound $I_{18}$,

yield 100 %, mp. 200-203 °C, which is recrystallized from ethyl acetate to give 0.260 g of the grayish white crystals, yield 89.0 %, mp. 202-204 °C.

Anal. Calcd. (%) for $C_{17}H_{14}Cl_2O_6S_2$

: C 45.44 H 3.14, Cl 15.78, S 14.27,

Found (%): C 45.26, H 3.36, Cl 15.59, S 14.09.

IR: $\nu$ max(Nujol) 3040, 2670, 2570, 1768, 1740, 1715, 1612, 1602.

## Example 19

6,7-Dichoro-5-[2-(1,3-dithiolan-2-ylidene)acetyl]-2,3-dihydro-1-benzofuran-2-carboxylic acid.

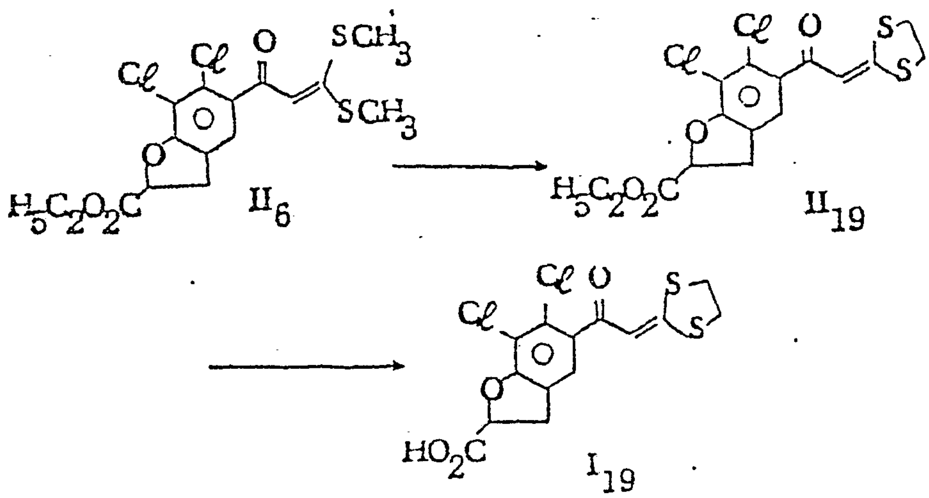

The compound $II_6$ (prepared in Example 6) (0.60 g, 1.5 mmol) is allowed to react with 0.208 g (2.2 mmol) of ethanedithiol in 10 ml of toluene for 24 hours on an oil bath (140-145 °C) while being stirred. The reaction product is chromatographed on a Lober column (Type B) with a n-hexane/ethyl acetate (7/3) mixture to give 0.288 g of the compound $II_{19}$ (oil), yield 48.0 %.

NMR $\delta$ ppm ($CDCl_3$): 1.30 (3H, t), 3.13-3.77 (6H, m), 4.25 (2H, q), 5.17-5.55 (1H, m), 6.95 (1H, s), 7.17-7.40 (1H).

To 0.52 g (1.3 mmol) of the compound $II_{19}$ are added 2 ml of

ethanol, 2 ml of dioxane and 2 ml (2 mmol) of 1 N sodium hydroxide, and the mixture is allowed to react at room temperature for 30 minutes to give 0.487 g of the compound $I_{19}$, yield 100%, mp, 215-218 °C. This is recrystallized from acetone to give 0.42 g of grayish white crystals, yield 86.8 %, mp. 217-218 °C.

Anal. Calcd. (%) for $C_{14}H_{10}Cl_2O_4S_2$

: C 44.57 H 2.67, Cl 18.80, S 17.00,

Found (%): C 44.38, H 2.62, Cl 19.03, S 16.77.

IR $\nu$ max (Nujol): 2720, 2560, 2470, 1743, 1610. 1580 $cm^{-1}$.

Example 20

6,7-Dichloro-5-[2-methyl-3,3-bis(methylthio)propenoyl]-2,3-dihydro-1-benzofuran-2-carboxylic acid monosulfoxide

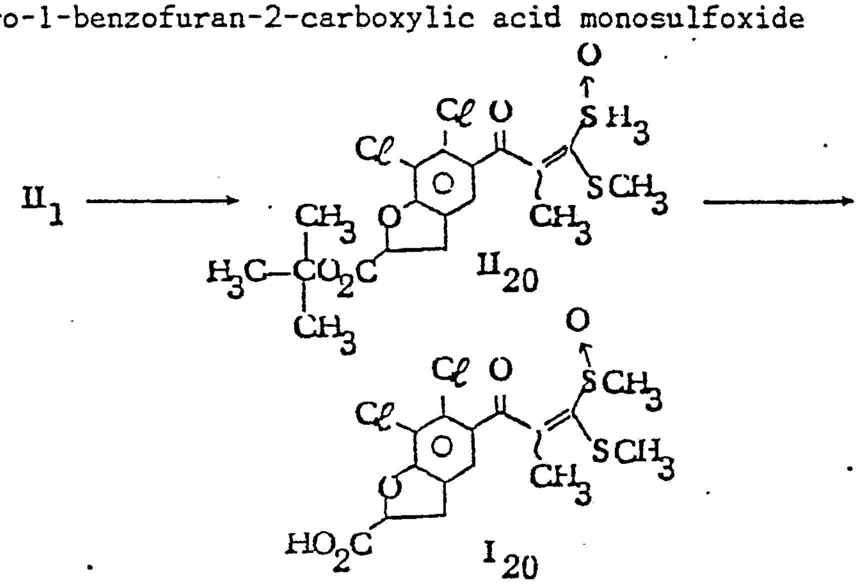
$II_1$
$II_{20}$
$I_{20}$

To a solution of 0.67 g (1.5 mmol) of the compound $II_1$ (prepared in Example 1) in 11.2 ml of dry dichloromethane is added in small portions 0.26 g (1.5 mmol) of m-chloroperbenzoic acid in a 40 minute period under flowing nitrogen while being stirred at an internal temperature of -7 to -5 °C. The resulting mixture is allowed to react at the same temperature for 15 minutes and then at room temperature for 40 minutes. The product is chromatographed on a Lober (Type B) column with a chloroform/benzene/ethyl acetate (3/1/1) mixture to give 0.20 g of the

compound $II_{26}$ as an oil, yield 28.8 %.

NMR $\delta$ ppm ($CDCl_3$): 1.50 (9H, s), 2.20 (3H, s), 2.33 (3H, s), 2.70 (3H, s), 3.10-3.93 (2H, m), 5.13-5.43 (1H, m), 7.60 (1H).

In the same manner as in Example 1, 0.20 g (0.4 mmol) of the compound $II_{20}$ is treated with trifluoroacetic acid and the ether-soluble matter is recrystallized from ethyl acetate to give 0.55 g of grayish white crystals, yield 31.1 %, mp. 197-199 °C (dec.).

Anal. Calcd. (%) for $C_{15}H_{14}Cl_2O_5S_2$

: C 44.01 H 3.45, Cl 17.33, S 15.67,

Found (%): C 44.02, H 3.54, Cl 17.35, S 15.76.

IR: $\nu$ max(Nujol) 2600, 2470, 1720, 1670, 1605.

## Examples 21-27

Compounds $I_{21-27}$ and their intermediates $II_{21-27}$ are prepared in the same manner as in Example 19 or 20, whose physical constants and reaction conditions are shown in the following Table 2 (Nos. 1 and 2).

Table 2 ( No. 1 )

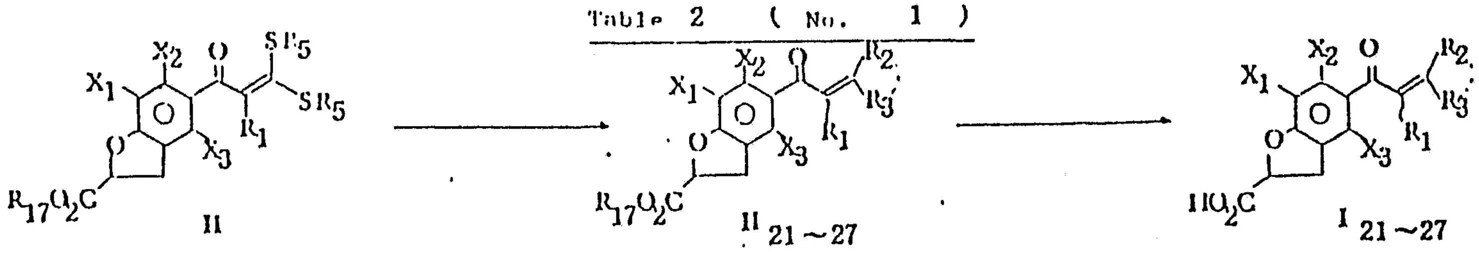

II

II 21~27

I 21~27

| Example Nos. | II R17 | R1 | R5 | X1 | X2 | X3 | Amount Used g (mmol) II | Reagent | Solvent | Temp. | Time | 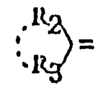 | Yield (%) | NMR |
|---|---|---|---|---|---|---|---|---|---|---|---|---|---|---|
| 21 | tBu | $CH_3$ | $CH_3$ | Cℓ | Cℓ | H | 1.60 (3.6) | 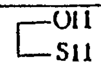 0.292 (3.7 | Toluene 16 mℓ | 140~145° | 24 | 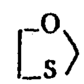 | 42.9 | 1.50(9H, S) 1.83(3H, S) 3.10~3.90(4H, m) 4.53(2H, t) 5.05~5.40(m, 1H) 6.88~7.00(1H) |
| 22 | 〃 | 〃 | 〃 | 〃 | 〃 | 〃 | 2.00 (4.5) | 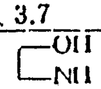 0.326 (5.3) | 12 | 〃 | 28 | 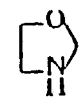 | 16.8 | 1.47(9H, S) 1.62(3H, S) 3.07~3.63(2H, m) 3.85(2H, t) 4.55(2H, t) 5.05~5.35(1H, m) 6.85~6.98(1H) 10.4~9.00(1H) |
| 23 | 〃 | 〃 | 〃 | 〃 | 〃 | 〃 | 1.20 (2.7) | 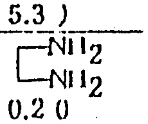 0.20 (3.3) | 6 | 〃 | 20 | 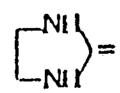 | 35.2 | 1.45(12H, S) 3.03~3.97(6H, m) 5.20~5.65 (1H, m) 6.98(1H) 7.18(br, 1H) 9.70(br, 1H) DMSO |
| 24 | $CH_3$ | 〃 | 〃 | 〃 | 〃 | 〃 | 2.0 (4.9) | HCl gas | MeOH 20 mℓ | -50° | 7 | 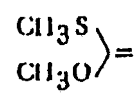 | 8.3 | 1.83(3H, S) 2.13(8H, S) 3.17~3.76(5H, m) 3.83(3H, S) 5.18~5.55(1H, m) 6.80~6.92(1H) |
| 25 | 〃 | 〃 | 〃 | 〃 | 〃 | 〃 | 0.50 (1.2) | φ SH 0.5 (4.5) | Toluene 5 | 140~145° | 12 | 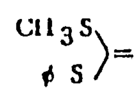 | 72.9 | [1.82(S) 2.30(S) 3H] [2.22(S) 2.28(S) 3H] 3.08~3.73(2H, m) [3.77, 3.80(3H)] 5.12~5.52 (1H, m) 7.07~7.47(6H, m) |
| 26 | $CH_3$ | 〃 | 〃 | 〃 | 〃 | 〃 | 0.65 (1.6) | 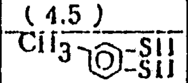 0.52 (3.3) | Toluene 6.5 | 140~145° | 20 | 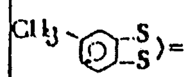 | 15.4 | 2.02(3H, S) 2.40(3H, S) 3.23~3.73(2H, m) 3.82(3H, S) 5.22~5.48(1H, m) 6.82~7.58(4H) |
| 27 | $C_2H_5$ | H | 〃 | 〃 | 〃 | 〃 | 0.23 (0.6) | HCℓ gas | EtOH 20 | 25° | 3.5 | 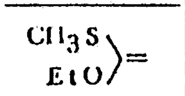 | 69.9 | [1.07(t) 1.12(t) 3H] 2.35(3H, S) 3.13~3.17 (2H, m) 4.00(q−q, 2H) 4.27(q, 2H) 1.32 (t, 3H) 5.13~5.48(1H, m) 5.73(1H, S) 6.87~7.00 (1H) |

C139596

Example 28

6,7-Dichloro-5-[3,3-bis(methylthio)-2-propenoyl]-2,3-dihydro-1-benzofuran-2-carboxaldehyde

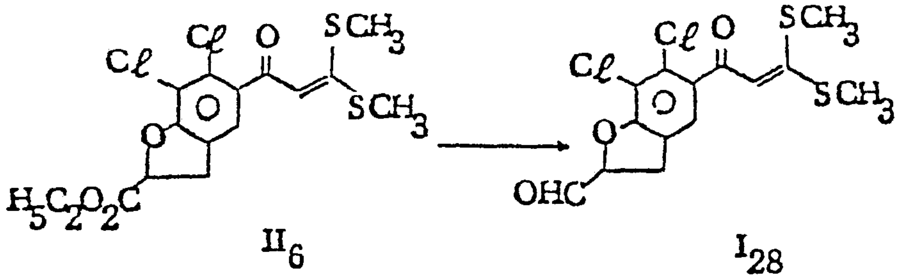

$II_6$ $I_{28}$

To a cooled solution (-78 °C) of 0.55 g of the compound $II_6$ (prepared in Example 6) in 6 ml of dry tetrahydrofuran (THF) is added 0.39 ml of a solution of 70 % sodium bis(2-methoxyethoxy)-aluminium hydride in toluene in 10 minutes while being stirred under nitrogen atmosphere. The mixture is allowed to react for 30 minutes. The reaction mixture is combined with 10 % hydrochloric acid and extracted with benzene (3 times). The benzene layer is washed with water, dried over dry magnesium sulfate and evaporated to give a residue, which is chromatographed on a Lober column (Type B) with chloroform/benzene/ethyl acetate (3/1/1) to give 0.40 g of the compound $I_{28}$, yield 81.6 %, mp. 160-163 °C.

This is recrystallized from acetone to give 0.350 g of grayish white crystals, yield 71.4 %, mp. 163-165 °C.

Anal. Calcd. (%) for $C_{14}H_{12}Cl_2O_3S_2$

: C 46.28 H 3.33, Cl 19.52, S 17.65,

Found (%): C 46.05, H 3.54, Cl 19.59, S 17.49.

IR $\nu$ max (Nujol): 2710, 1733, 1625, 1603 $cm^{-1}$.

NMR $\delta$ ppm (DMSO $d_6$): 2.50 (s), 2.55 (s), 5.43-5.73 (1H, m), 3.37-3.68 (2H, m), 6.43 (1H,s), 7.42 (1H), 9.70 (1H, s).

Table 2 (No. 2)

| Example No. | Amount Used g (mmol) II | CF$_3$CO$_2$H (A) / KOH (B) | Temp | Time | Recrystal from | m.p. | Yield % | molecular formula | Elementary Analysis (%) Calcd. / Found C | H | Cℓ | N | S | I R |
|---|---|---|---|---|---|---|---|---|---|---|---|---|---|---|
| 21 | 0.64<br>(1.5) | A | 25 | 0.5 | Dioxane/<br>ether | 253~<br>255(d) | 80.3 | $C_{15}H_{12}C\ell_2O_5S$ | 48.01<br>47.99 | 3.22<br>3.44 | 18.90<br>18.84 | | 8.55<br>8.40 | 2685, 2560, 2480, 1740,<br>1610, 1570, |
| 22 | 0.42<br>(1.0) | A | 〃 | 1 | — | 241~<br>242(d) | 55.9 | $C_{15}H_{13}C\ell_2NO_5$ | 50.29<br>49.99 | 3.66<br>3.76 | 19.80<br>20.07 | 3.91<br>3.88 | | 3295, 2480, 1730, 1620,<br>1610 |
| 23 | 0.58<br>(1.4) | A | 〃 | 2/3 | acetone | 273~<br>276(d) | 73.9 | $C_{15}H_{14}C\ell_2N_2O_4$ | 50.44<br>50.49 | 3.95<br>4.08 | 19.85<br>19.78 | 7.84<br>7.59 | | 3465, 3330, 2460, 1730,<br>1600 |
| 24 | 0.15<br>(0.4) | B | 〃 | 1 | ether/<br>hexane | 105~<br>107 | 34.5 | $C_{15}H_{14}C\ell_2O_5S$ | 47.75<br>47.79 | 3.74<br>4.04 | 18.80<br>18.71 | | 8.50<br>8.30 | 2720~2370, 1682(sh1728,<br>1710)1608 |
| 25 | 0.42<br>(0.9) | B | 〃 | 1 | isopropy<br>ether | 76~<br>78 | 29.5 | $C_{20}H_{16}C\ell_2O_4S_2$ | 52.75<br>52.91 | 3.54<br>3.70 | 15.57<br>15.31 | | 14.08<br>14.00 | 2690, 2590, 1743, 1714,<br>1655, 1605 |
| 26 | 0.15<br>(0.3) | B | 〃 | 3 | acetone | 215~<br>217(d) | 66.0 | $C_{20}H_{14}C\ell_2O_4S_2 \cdot \frac{1}{2}H_2O$ | 51.95<br>52.25 | 3.16<br>3.28 | 15.34<br>15.29 | | 13.87<br>13.65 | 3380, 2720, 2620, 2540,<br>1708, 1747, 1605, 1594 |
| 27 | 0.395<br>(1.0) | B | 〃 | 1 | ether/<br>petroleum<br>ether | 135~<br>136 | 67.9 | $C_{15}H_{14}C\ell_2O_5S$ | 47.75<br>47.55 | 3.74<br>3.75 | 18.80<br>18.98 | | 8.50<br>8.29 | 3230, 1762, 1693, 1610 |

## Example 29

6,7-Dichloro-5-[3,3-bis(methylthio)-2-propenoyl]-2,3-dihydro-1-benzofuran-2-carboxaldoxime

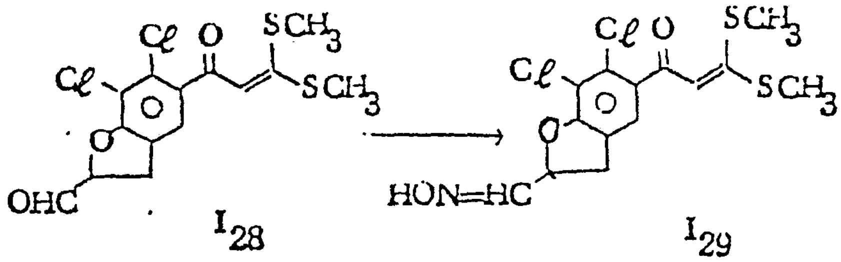

$I_{28}$ $I_{29}$

A mixture of 0.18 g (0.5 mmol) of the compound $I_{28}$ (prepared in Example 28), 0.118 g (1.5 mmol) of pyridine, 0.069 g (1 mmol) of hydroxylamine hydrochloride ($NH_2OH \cdot HCl$), 2 ml of methanol and 4 ml of water is allowed to react at room temperature for 1.5 hours under stirring. The precipitated crystals are collected by filtration to give 0.170 g of the product $I_{29}$, yield 90.9 %, mp. 185-186 °C. This is recrystallized from acetone to give 0.130 g of grayish white crystals, yield 69.5 %, mp. 188-189 °C.

Anal. Calcd. (%) for $C_{14}H_{13}Cl_2NO_3S_2$

: C 44.45 H 3.46, Cl 18.75, N 3.70, S 16.95,

Found (%): C 44.25, H 3.60, Cl 18.60, N 3.60, S 16.84.

IR $\nu$ max (Nujol): 3360, 1615, 1606 $cm^{-1}$.

NMR $\delta$ ppm (DMSO $d_6$): 2.50 (s), 2.60 (s) (shaded by DMSO signal), 3.10-3.73 (2H, m), 5.37-5.77 (1H, m), 6.42 (1H, s), 7.40 (1H), 7.57 (1H, d), 11.35 (1H, s).

## Example 30

6,7-Dichloro-5-[3,3-bis(methylthio)-2-propenoyl]-2,3-dihydro-1-benzofuran-2-carboxynitrile

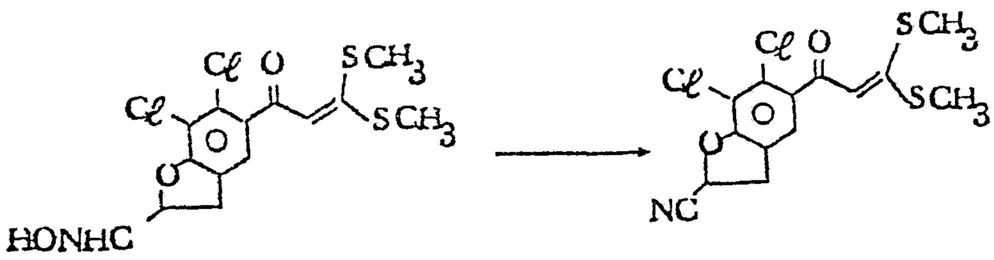

$I_{29}$ $I_{30}$

To a solution of 0.34 g (0.9 mmol) of the compound $I_{2}$, (prepared in Example 29) and 0.08 g (1.0 mmol) of pyridine in a mixture of 5 ml of ether and 5 ml of tetrahydrofuran is added 0.158 g (0.9 mmol) of phenylchlorosulfate (Ph-O-SOCl) [ω: E. Bissingene JACS 70 2664 (1948)] at 0 °C under stirring. The mixture is allowed to react in accordance with the method as disclosed in J. G. Krause et. al. Synthesis 502 (1975). The reaction mixture is purified by liquid chromatography on a Lober column (Type B) chloroform/benzene/ethyl acetate (3/1/1) to give 0.130 g of the compound $I_{30}$, yield 40.2 %, mp. 205-209 °C. This is recrystallized from acetone to give 0.100 g of grayish white crystals, yield 31.0 %, mp. 209-210 °C.

Anal. Calcd. (%) for $C_{14}H_{11}Cl_2NO_2S_2$

: C 46.67 H 3.08, Cl 19.68, N 3.89, S 17.80,

Found (%): C 46.41, H 3.15, Cl 19.62, N 4.02, S 17.96.

IR ν max (Nujol): 1622, 1605 $cm^{-1}$.

NMR δ ppm (DMSO $d_6$): 2.50(s), 2.58 (s) (shaded by DMSO signal), 3.57-3.90 (2H, m), 6.05 (1H, s), 6.42 (1H, m), 7.48 (1H).

Example 31

6,7-Dichloro-5-[2-(1,3-dithiol-2-ylidene)propynoyl]-1-benzofuran-2-carboxylic acid.

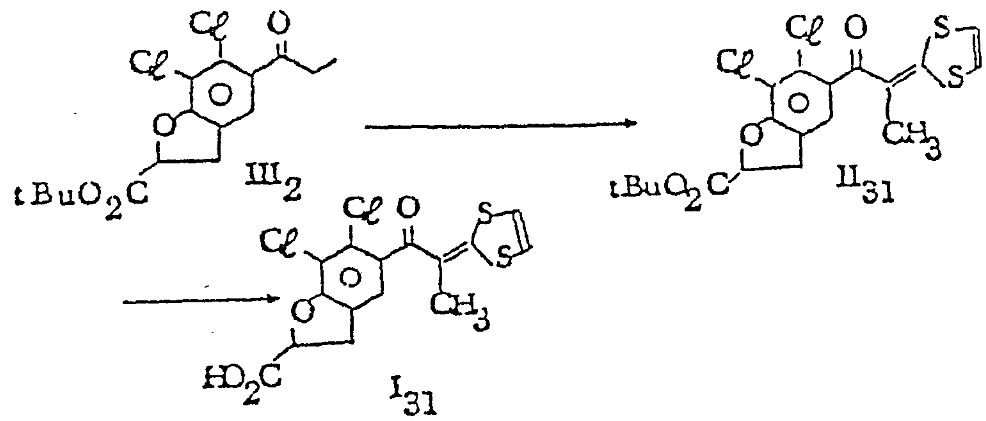

$III_2$ $II_{31}$ $I_{31}$

To a suspension of 0.225 g (6.2 mmol) of 65.6 % sodium hydride in 5 ml of dry acetonitrile is added a solution of 1.50 g (4.3 mmol) of the compound $III_2$ in 5 ml of acetonitrile under nitoregen flow while being stirred at room temperature. Subsequently, 1 ml of N,N-dimethylacetoamide is added to the mixture and the resulting mixture is allowed to react for 1.5 hours and then 1.44 g (5.2 mmol) of 2-methylthio-1,3-dithioliodide [L. Russell Melky et. al. JOC 39, 2456 (1974)] is added to the reaction mixture and this mixture is allowed to react for 6 hours. The reaction product is purified by liquid chromatography on a Lober column (Type B) with a mixture of n-hexane/ethyl acetate (7/3) to give 0.4 g of a crude product, yield 20.6 %. This is treated with ether to give 0.284 g of the compound $II_{31}$, yield 14.7 %, 175-177 °C.

NMR $\delta$ ppm ($CDCl_3$): 1.50(9H, s), 3.07-3.73 (2H, m), 5.08-5.37 (1H, m), 6.87-7.13 (3H, m).

To 0.41 g (0.9 mmol) of the compound $II_{31}$ is added 4.1 ml of trifluoroacetic acid and the mixture is allowed to react at room temperature for an hour under stirring.

The solvent is evaporated and the residue is treated with n-hexane to give 0.36 g of the compound $I_{31}$, yield 100 %, mp. 266-270 °C (dec.).

Anal. Calcd. (%) for $C_{18}H_{10}Cl_2O_4S_2$

: C 46.28 H 2.59, Cl 18.22, S 16.47,

Found (%): C 46.19, H 2.78, Cl 18.24, S 16.35.

IR: ν max (Nujol) 2650, 2560, 1712 (sh 1750),1608, 1583.

Example 32

6,7-Dichloro-5-[3,3-bis(methylthio)-2-propenoyl]-1-benzofuran-2-carboxylic acid.

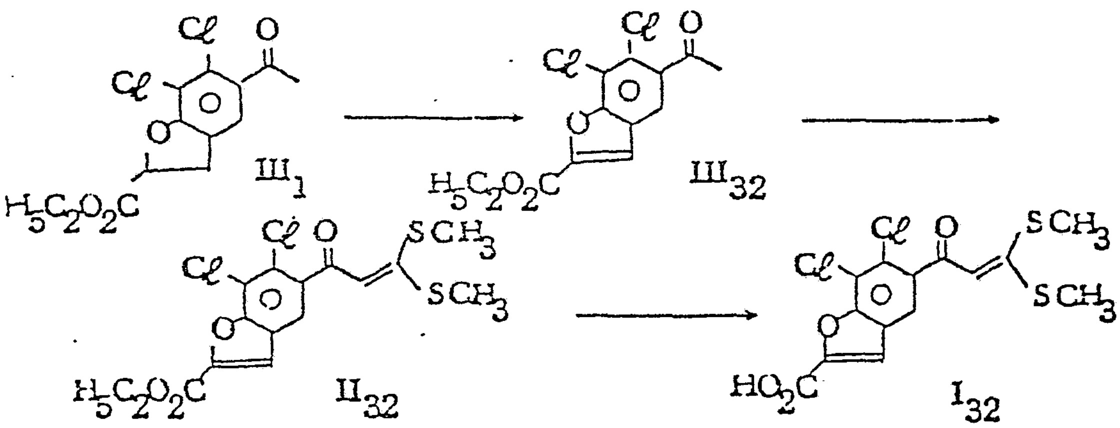

$III_1$ $III_{32}$ $II_{32}$ $I_{32}$

A mixture of 1.50 g (5.0 mmol) of ethyl 5-acetyl-6,7-dichloro-2,3-dihydro-1-benzofuran-2-carboxylate $III_1$ (William. Hoffman. et. al., J. Med. Chem.24 865 (1981)), 0.025 g (0.1 mmol) of benzoperoxide, 0.9 g (5.1 mmol) of N-bromosuccinimide and 50 ml of carbon tetrachloride is allowed to react according to the method disclosed in the above-mentioned literature.

The reaction product is treated in 0.6 ml (5.2 ml) of 1,5-diazabicyclo[4.3.0]none-5-ene and 12.5 ml of dimethyl sulfoxide, mentioned in the same literature, to give 1.20 g of the compound $III_{32}$, yield 80.5 %, mp. 120-122 °C.

NMR δ ppm ($CDCl_3$): 1.45(3H, t), 2.67 (3H, s), 4.47 (2H, q), 7.53 (1H, s), 7.73 (1H, s).

From 1.20 g (4.0 mmol) of the compound $III_{32}$ is prepared 0.66 g of the compound $II_{32}$, with treating in the same manner mentioned in Example 1, yield 40.9 %, mp.184-185 °C.

NMR $\delta$ ppm ($CDCl_3$): 1.42(3H, t), 2.50 (3H, s), 2.57 (3H, s), 4.45 (2H, q), 6.40 (1H, s), 7.50 (1H, s), 7.68 (1H, s).

According to Example 1, 0.30 g of (0.7 mmol) of the compound $II_{32}$ is hydrolyzed with alkali to give 0.279 g of the product $I_{32}$, mp. 266-271 °C (dec.). This is recrystallized from acetone to give 0.258 g of grayish white crystals, yield 92.4 %, mp. 268-271 °C.

Anal. Calcd. (%) for $C_{14}H_{10}Cl_2S_4$

: C 44.57, H 2.67, Cl 18.80, S 17.00,

Found (%): C 44.40, H 2.83, Cl 18.83, S 16.70.

IR $\nu$ max (Nujol): 2700, 2600, 2520, 1700, 1627, 1608 $cm^{-1}$.

Example 33

6,7-Dichloro-5-[2-methyl-3,3-bis(methylthio)propenoyl]-1-benzofuran-2-carboxylic acid

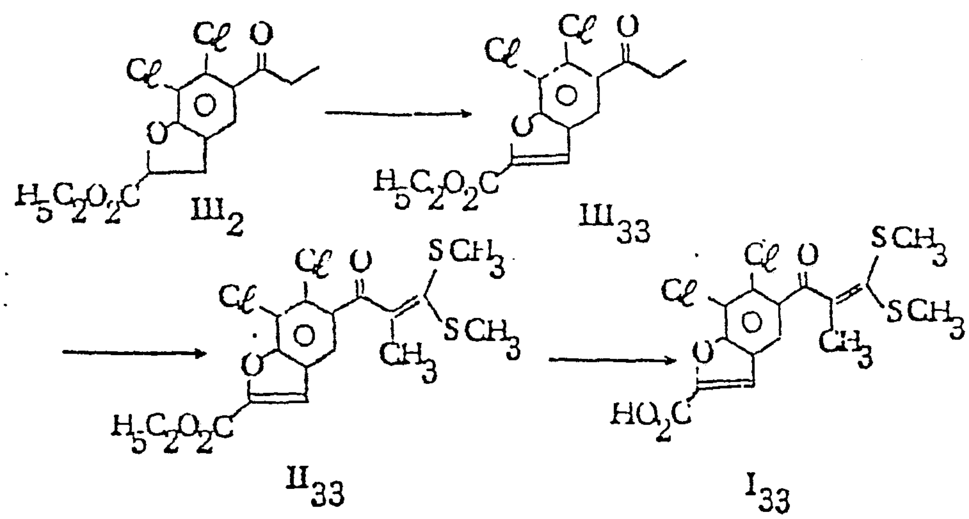

$III_2$ $III_{33}$ $II_{33}$ $I_{33}$

In the same manner as in Example 32 is treated 3.0 g (9.5 mmol) of the compound $III_2$ to give 2.24 g of the compound $III_{33}$, yield 75.6 %, mp. 107-108 °C.

NMR δ ppm ($CDCl_3$): 1.23 (3H, t), 1.43 (3H, t), 2.90 (2H, q), 4.45 (2H, q), 7.53 (1H, s), 7.63 (1H, s).

In the same manner as in Example 1 is treated 1.70 g (5.4 mmol) of the compound $III_{33}$ to give 0.50 g of the compound $II_{33}$, yield 22.1 %.

NMR δ ppm ($CDCl_3$): 1.43 (3H, t), 1.88 (3H, s), 2.30 (3H, s), 2.37 (3H, s), 4.45 (2H, q), 7.52 (1H, s), 7.72 (1H, s).

In the same manner as in Example 1 is hydrolyzed 0.095 g (0.2 mmol) of the compound $II_{33}$ to give 0.084 g of the compound $I_{33}$, yield 95.5 %, mp. 216-218 °C (dec.).

This is recrystallized from ethyl acetate to give 0.074 g of grayish white crystals, yield 84.1 %, mp. 218-219 °C.

Anal. Calcd. (%) for $C_{15}H_{12}Cl_2O_4S_2$

: C 46.04 H 3.09, Cl 18.12, S 16.39,

Found (%): C 45.98, H 3.28, Cl 18.19, S 16.18.

IR: ν max (Nujol) 2710, 2600, 2500, 1714, 1692, 1625, 1604.

## Example 34

6,7-Dichloro-5-[2-(1,3-dithiolan-2-yliden)acetyl]-1-benzofuran-2-carboxylic acid

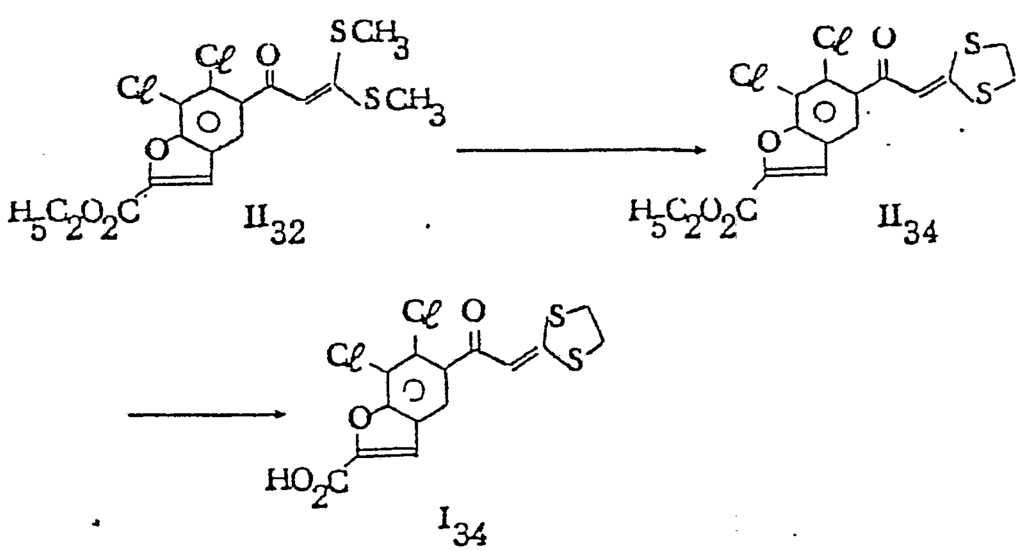

$II_{32}$ $II_{34}$ $I_{34}$

In the same manner as in Example 19 is treated 0.350 g (0.9

mmol) of the compound $II_{32}$ (prepared in Example 32) to give 0.120 g of the compound $II_{34}$, yield 34.5 %, mp. 235-237 °C.

NMR $\delta$ ppm ($CDCl_3$): 1.45(3H, t), 3.52 (4H, br), 4.50 (2H, q), 6.93 (1H, s), 7.58 (1H, s), 7.72 (1H, s).

In the same manner as mentioned in Example 32 is hydrolyzed 0.120 g (0.3 mmol) of the compound $II_{34}$ to give 0.112 g of the compound $I_{34}$, yield 100 %, mp. 300-305 °C (dec.). This is recrystallized from methyl ethyl ketone to give 0.105 g of grayish white crystals, yield 93.8 %, mp. 305-309 °C (dec.).

Anal. Calcd. (%) for $C_{14}H_8Cl_2O_4S_2$

: C 44.81 H 2.15, Cl 18.90, S 17.09,

Found (%): C 44.91, H 2.40, Cl 18.91, S 16.89.

IR $\nu$ max (Nujol): 2570, 1714, 1611, 1580 $cm^{-1}$.

Example 35

6, 7-Dichloro-5-[2-methyl-3-mercapto-3-(methylthio)-propanoyl-2,3-dihydro-1-benzofuran-2-carboxylic acid

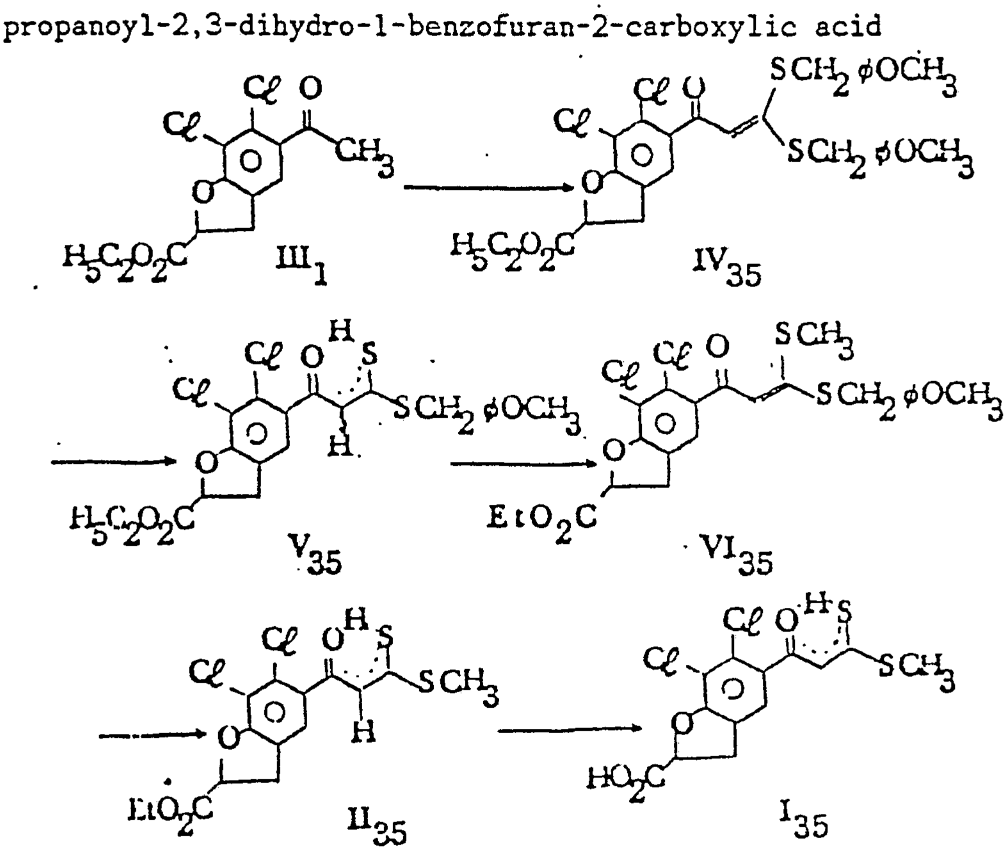

$III_1$ $IV_{35}$ $V_{35}$ $VI_{35}$ $II_{35}$ $I_{35}$

The compound $III_1$ (7.0 g, 23.1 mmol) is allowed to react with 2.10 g (56.9 mmol) of 65 % sodium hydride, 14 g (69.6 mmol) of 4-methoxyl-benzylbromide, 5.3 g (69.6 mmol) of carbon disulfide, 90 ml of dry ether, 4.7 ml of N,N-dimethylacetoamide and a catalytic amount of potassium iodide in the same manner as mentioned in Example 1 to give 8.1 g of the compound $IV_{35}$, yield 56.6 %.

NMR $\delta$ ppm ($CDCl_3$): 1.28(3H, t×2), 3.08-3.83 (8H, m), 4.03-4.43 (6H, m), 5.12-5.40 (1H, m), 6.50 (1H, s), 6.63-7.37 (1H).

To 5.50 g (8.9 mmol) of the compound $IV_{36}$ are added 11 ml of anisole and 11 ml of trifluoroacetic acid, and the mixture is allowed to reacted under stirring at room temperature for 2 hours. The solvent is evaporated and the resulting residue is chromatographed on a 40 g silical-gel column with a mixture of n-hexane/benzene (7/3), with n-hexane/benzene (7/3) (F-1, 400 ml), n-hexane/benzene (1/1) (F-2, 200 ml), n-hexane/benzene (2/3) (F-3, 200 ml) and benzene (F-4, 500 ml) as eluates in order. From the last fraction, 3.93 g of the compound $V_{36}$ is recovered, yield 88.8 %.

NMR $\delta$ ppm ($CDCl_3$): 1.30(3H, t), 3.17-3.70 (2H, m), 4.08-4.43 (4H, m), 5.18-5.47 (1H, m), 6.57 (1H, s), 6.70-7.43 (5H), 14.94 (1H, s).

To a suspension of 1.9 g (3.8 mmol) of the compound $V_{36}$ and 0.79 g (5.7 mmol) of powdery potassium carbonate in 10 ml of N,N-dimethylformamide, under nitrogen flow while being stirred at room temperature is added 1.08 g (7.6 mmol) of methyl iodide, the mixture is allowed to react for 2 hours. Insoluble material is filtered off and benzene is added to the filtrate. The benzene solution is washed with water (4 times), dried over anhydrous magnesium sulfate and evaporated to give a residue which is

treated by high performance liquid chromatography on a Lober column (Type B) with a benzene/ethyl acetate (10/1) mixture to give 1.80 g of the compound $VI_{36}$, yield 92.0 %,

NMR $\delta$ ppm ($CDCl_3$): 1.30(3H, t), 2.50 (3H, s), 3.10-3.70 (2H, m), 3.78 (3H, s), 4.07-4.47 (4H, m), 5.17-5.45 (1H, m), 6.43, 6.57 (1H, s×2), 6.73-7.40 (5H).

To 1.81 g (3.5 mmol) of the compound $VI_{36}$ are added 3.6 ml of anisole and 3.6 ml of trifluoroacetic acid, and the mixture is allowed to react at room temperature for 2 hours under stirring. Toluene is added to the reaction mixture and then the resulting mixture is evaporated to give a residue. The residue is chromatographed on a column of 18 g of silica-gel with n-hexane/benzene (7/3) mixture, with n-hexane/benzene (7/3) (F-1, 600ml), n-hexane/benzene (1/1) (F-2, 200ml), and n-hexane/benzene (2/3) (F-3, 400ml) as eluents in order. From the last fraction i.e. F-3, 1.30 g of the compound $II_{36}$ is recovered, yield 93.8 %.

IR $\nu$ max ($CHCl_3$): 1755, 1730, 1608, 1585 $cm^{-1}$.

NMR $\delta$ ppm ($CDCl_3$): 1.30(3H, t), 2.62 (3H, s), 3.12-3.95 (2H, m), 4.25 (2H, q), 5.22-5.65 (1H, m), 6.60 (1H, s), 7.28 (1H), 14.95 (1H,s).

To 0.24 g (0.6 mmol) of the compound $II_{36}$ are added 3 ml of ethanol and 1.2 ml of 1N potassium hydroxide, the mixture is allowed to react at room temperature for an hour. The solution is evaporated to give a residue to which is added 10 % hydrochloric acid to adjust to pH 3. The precipitated crystalline solid is collected by filtration, washed with water, and dried to give 0.213 g of the compound $I_{36}$, yield 95.5 %, mp. 182-185 °C (dec.). This is recrystallized from ethanol to give 0.113 g of yellow crystals, yield 5o.5%, mp. 185-186 °C (dec.).

IR $\nu$ max (Nujol): 3040, 2750, 2660, 2540, 1723 (sh 1708),

1608, 1584 $cm^{-1}$.

Anal. Calcd. (%) for $C_{13}H_{10}Cl_2O_4S_2$

: C 42.75 H 2.76, Cl 19.41, S 17.56,

Found (%): C 42.84, H 2.88, Cl 19.37, S 17.40.

Examples 36 and 37

The compounds $I_{36}$, $I_{37}$ and their intermediates are prepared in the same manner as in Example 35, whose physical constants and reaction conditions are shown in the following Table 3 (Nos. 1 to 5).

Table 3 ( No. 1 )

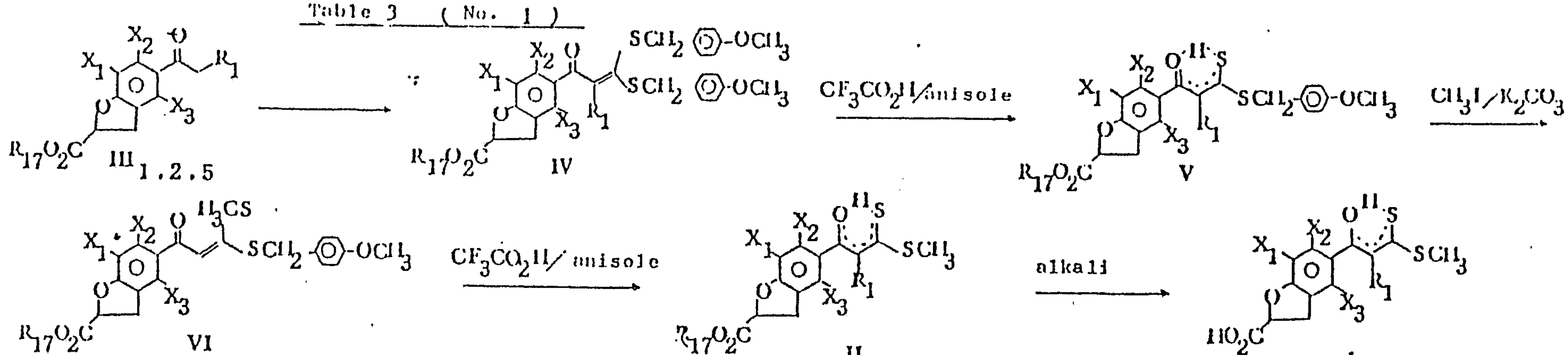


$II_{36\sim37}$ $I_{36\sim37}$

| Example Nos. | (III) R1 | R17 | X1 | X2 | X3 | Amount Used g (mmol) (III) | NaH | CS2 | R5Br $CH_3O$-⊙-$CH_2Br$ | Solvent ml ether DME | DMA | Temp | Time | Yield (%) | NMR $\delta^{CDCl_3}_{ppm}$ (IV) |
|---|---|---|---|---|---|---|---|---|---|---|---|---|---|---|---|
| 36 | $CH_3$ | $C_2H_5$ | Cl | Cl | H | 4.0 (12.6) | 1.11 (30.3) | 2.87 (37.7) | 7.50 (37.3) | 40 | 2.59 | 25 ~ 27° | 96 | 9.4 ※v (9.9%) | 1.32(3H,t)2.07(S,3H)3.27~3.60(2H,m)3.77(8H,S) 4.00(2H,S)4.25(2H,q)5.17~5.45(1H,m) 6.57~7.37(9H,m) |
| 37 | H | $CH_3$ | Cl | Cl | H | 3.40 (13.8) | 1.04 (28.2) | 3.30 (43.3) | 8.0 (39.8) | 33 | 2.35 | 〃 | 〃 | 32.5 | 3.20~3.93(11H,m)4.12(2H,S)4.20(2H,S) 5.13~5.50(1H,m)6.53(1H,S)6.67~7.37(9H) |

Table 3 ( No. 2 )

| Example Nos. | Amount Used g (mmol) (IV) | $CF_3CO_2H$ | anisole | Temp | Time | Yield (%) | NMR $\delta^{CDCl_3}_{ppm}$ (V) |
|---|---|---|---|---|---|---|---|
| 36 | 0.55 (0.9) | 1.1 ml | 1.1 ml | 25° | 2 | 69.5 | 1.30(t,3H)1.62(d)1.93(S)3H 6.67~7.43(5H,m) 3.17~3.70(2H,m)3.78(3H,S) 4.07~4.17(4H,m)4.90~5.53(1H,m) |
| 37 | 2.71 (4.5) | 5.4 | 5.4 | 〃 | 2 | 85.7 | 3.20~3.90(8H,m)4.43(2H,S)5.20~5.65(1H,m) 6.53(S,1H)6.72~7.37(5H,m)14.93(1H,S) |

Table 3 (No. 3)

| Example Nos. | Amount Used g (mmol) (V) | $K_2CO_3$ | $CH_3J$ | Solvent | Temp. | Time | Yield (%) | NMR $\delta^{CDCl_3}_{ppm}$ (VI) |
|---|---|---|---|---|---|---|---|---|
| 36 | 0.2 (0.6) | 0.162 (1.2) | 0.247 (1.7) | DME 3 ml | 25° | 6 | 64.9 | |
| 37 | 1.86 (9.8) | 1.06 (7.7) | 1.09 (7.7) | MeCN 6 | 25° | 1 | 94.2 | 2.47 (3H, S) 3.07~3.83 (8H, m) 4.10 (1H, S) 4.22 (1H, S) 5.17~5.45 (1H, m) 6.40 (S) 6.53 (S) (1H) 6.70~7.40 (5H, m) |

Table 3 (No. 4)

| Example Nos. | Amount Used g (mmol) (VI) | $CF_3CO_2H$ | anisole | Temp. | Time | Yield (%) | NMR $\delta^{CDCl_3}_{ppm}$ (II) |
|---|---|---|---|---|---|---|---|
| 36 | 0.18 (0.3) | 0.36 ml | 0.36 ml | 25° | 2 | 71.9 | 1.32 (t, 3H) [1.62 (d) 1.98 (S), 3H) [2.58 (S) 2.65 (S), 3H] 3.13~3.90 (2H, m) 4.26 (2H, q) 5.05~5.53 (1H, m) 6.83 (1H) 7.02~7.23 (1H, m) |
| 37 | 1.75 (3.5) | 3.5 | 3.5 | 25 | 2 | 90.2 | 2.65 (3H, S) 3.13~3.77 (2H, m) 3.82 (3H, S) 5.23~5.52 (1H, m) 6.62 (1H, S) 7.30 (1H) 14.95 (1H, S) |

- 44 -

Table 3 (No. 5)

| Example Nos. | Amount Used g (mmol) (II) | KOH | Solvent | Temp | Time | Yield (%) | Re-crystal from | mp (dec) | Calcd. Found ($C_{14}H_{12}Cl_2O_4S_2$) C | H | Cl | S | IR : $\nu^{Nujol}_{max}$ |
|---|---|---|---|---|---|---|---|---|---|---|---|---|---|
| 36 | 0.1 (0.2) | 0.041 (0.7) | ethanol · water | 25 | 1 | 53.8 | ether | 175 ~ 177° | 44.33<br>44.25 | 3.19<br>3.24 | 18.70<br>18.59 | 16.91<br>16.63 | 3100 (br), 2640, 2540, 1716, 1610, 1565 |

Example 38

6,7-Dichloro-5-[3-(chlorothio)-3-(methylthio)-2-propenoyl]-2,3-dihydro-1-benzofuran-3-carboxylic acid

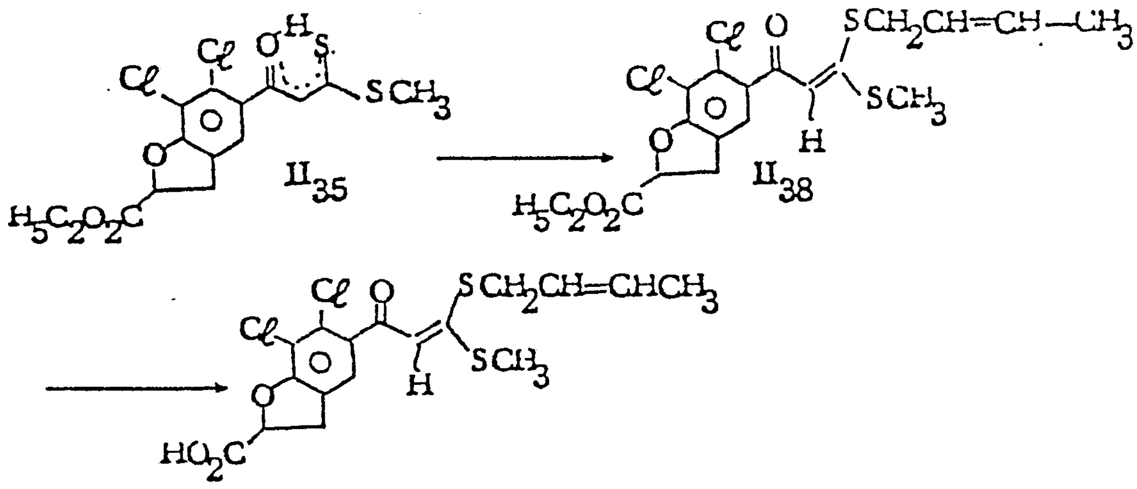

$II_{35}$ $II_{38}$

$I_{38}$

To 0.56 g (1.4 mmol) of ethyl 6,7-chloro-5-[3-(mercapto)-3-(methylthio)-2-propenoyl]-2,3-dihydro-1-benzofuran-2-carboxylate (prepared in Example 35) are added 0.387 g (2.8 mmol) of dry potassium carbonate, 0.250g (1.9 mmol) of crotyl bromide and 5 ml of N,N-dimethylformamide, and the mixture is allowed to react at room temperature for an hour while being stirred. Insoluble material is filtered off and benzene is added to the filtrate. The filtrate is washed with water, dried over anhydrous magnesium sulfate and evaporated. The residue is purified by high performance liquid chromatography on a Lober column (Type B) with a benzene/ethyl acetate (10/1) mixture to give 0.578 g of the oily compound $II_{38}$, yield 90.7 %.

NMR $\delta$ ppm ($CDCl_3$): 1.32(3H, t), 1.72 (3H, d), 2.50 (3H, s), 3.10-3.90 (4H, m), 4.43 (2H, q), 5.20-6.03 (3H, m), 6.55 (1H, s), 7.27 (1H).

To 0.185 g (0.4 mmol) of the compound $II_{38}$ are added 0.82 ml (0.8 mmol) of 1N potassium hydroxide and 2 ml of ethanol. The

mixture is allowed to react at room temperature for an hour. The solvent is evaporated to give a residue, to which ether is added. The mixture is adjusted to pH 3 with 10% hydrochloric acid while being stirred under ice-cooling. The ether layer is separated, washed with water, dried over dry magnesium sulfate and then evaporated to give 0.14 g of the objective product $I_{38}$, yield 80.9 %, mp. 166-169 °C. This is recrystallized from ethyl acetate to give 0.10 g of grayish white crystals $I_{38}$, yield 57.8 %, mp. 175-176 °C.

Anal. Calcd. (%) for $C_{17}H_{16}Cl_2O_4S_2$

: C 48.69 H 3.85, Cl 16.91, S 15.29,

Found (%): C 48.52, H 3.85, Cl 16.98, S 15.12.

IR $\nu$ max (Nujol): 2700, 2580, 2480, 1746, 1608 $cm^{-1}$.

Examples 39 - 41

The compounds are prepared in the same manner as in Example 38, whose physical constants and reaction conditions are shown in Table 4 (Nos. 1 and 2).

Table 4 (No. 1)

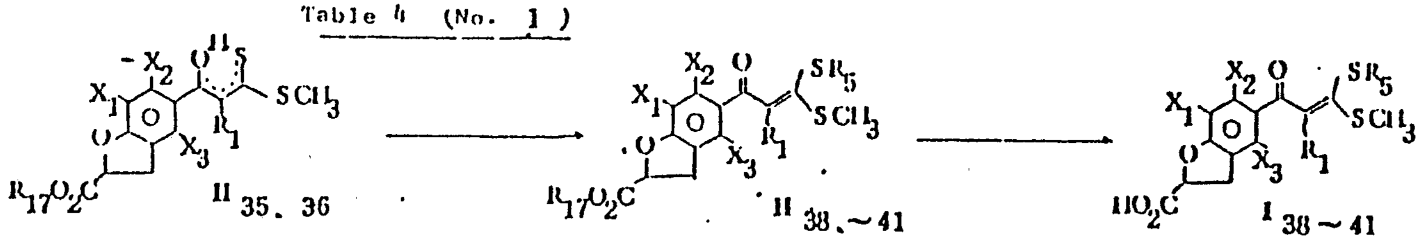

II 35, 36 → II 38 ~ 41 → I 38 ~ 41

| Example Nos. | $R_{17}$ | $R_1$ | $X_1$ | $X_2$ | $X_3$ | Amount Used g (mmol) (II 35, 36) | $R_5Br$ | $K_2CO_3$ | Solvent DMF | Temp. | Time | Yield (%) | Recrystal from | m.p. | N M R |
|---|---|---|---|---|---|---|---|---|---|---|---|---|---|---|---|
| 39 | $C_2H_5$ | $CH_3$ | Cℓ | Cℓ | H | 0.33 (0.8) | $C_2H_5Br$ 0.106 (1.0) | 0.168 (1.2) | DMF 7 ml | 25 | 5 | 58.6 | — | — | [0.95(t)1.25(t)3H]1.32(3H,t)2.27(3H,S) [2.36(S)2.02(S)3H][2.58(q)2.83(q)2H] 3.17~3.95(2H,m)4.30(q,2H)5.23~5.57(1H,m) 7.50~7.33(1H) |
| 40 | 〃 | H | 〃 | 〃 | 〃 | 0.16 (0.4) | $BrCH_2C \equiv CH$ 0.07 (0.6) | 1.10 (0.8) | 2 ml | 25 | 1 | 88.0 | — | — | 1.30(3H,t)2.28~2.47(1H,m)2.52(3H,S)3.15~3.95(2H,m)4.25(2H,q)5.18~5.45(1H,m) [6.33(S)6.62(S)1H]7.22(1H) |
| 41 | $CH_3$ | H | 〃 | 〃 | 〃 | 0.267 (0.7) | $C\ell CH_2CONH_2$ 0.098 (1.0) | 0.198 (1.4) | 4 ml | 25 | 5 | 33.8 | | 174 ~ 175° | 2.53(shaded by DMSO)3.20~3.47(2H,m)3.73 3.78 (5H,S~S)5.45~5.73(1H,m)6.43(1H,S) 7.17(1H,br)7.40(1H)7.58(1H,br) |

Table 4 (No. 2)

| Example Nos. | Amount Used (II) | KOH | Solvent $H_2O$-ethanol | Temp. | Time | Recrystal from | m.p. | Yield % | Molecular formula | C | H | Cℓ | S | N | IR |
|---|---|---|---|---|---|---|---|---|---|---|---|---|---|---|---|
| 39 | 0.33 (0.8) | 0.085 (1.5) | 3.6 | 25 | 1 | ether/hexane | 125 ~ 126 | 58.3 | $C_{16}H_{16}C\ell_2O_4S_2$ | 47.18<br>47.04 | 3.96<br>4.02 | 17.41<br>17.46 | 15.74<br>15.57 | —<br>— | 3020, 1763, 1630, 1600 |
| 40 | 0.145 (0.3) | 0.028 (0.5) | 2.5 | 25 | 1 | ethyl acetate | 135 ~ 136 (dec.) | 29.4 | $C_{16}H_{12}C\ell_2O_4S_2$ | 47.65<br>47.80 | 3.00<br>3.12 | 17.58<br>17.78 | 15.90<br>15.95 | —<br>— | 3285, 1736, 1700, 1627, 1603 |
| 41 | | | | | | | | | $C_{16}H_{15}C\ell_2NO_5S_2$ | 44.01<br>43.72 | 3.47<br>3.56 | 16.25<br>16.27 | 14.70<br>14.66 | 3.21<br>3.11 | 3430, 3365, 3290, 3185, 1755, 1682, 1625, 1603 |

Example 42

6,7-Dichloro-5-[3-(methylthio)-3-(vinylthio)-2-propenoyl]-2,3-dihydro-1-benzofuran-2-carboxylic acid

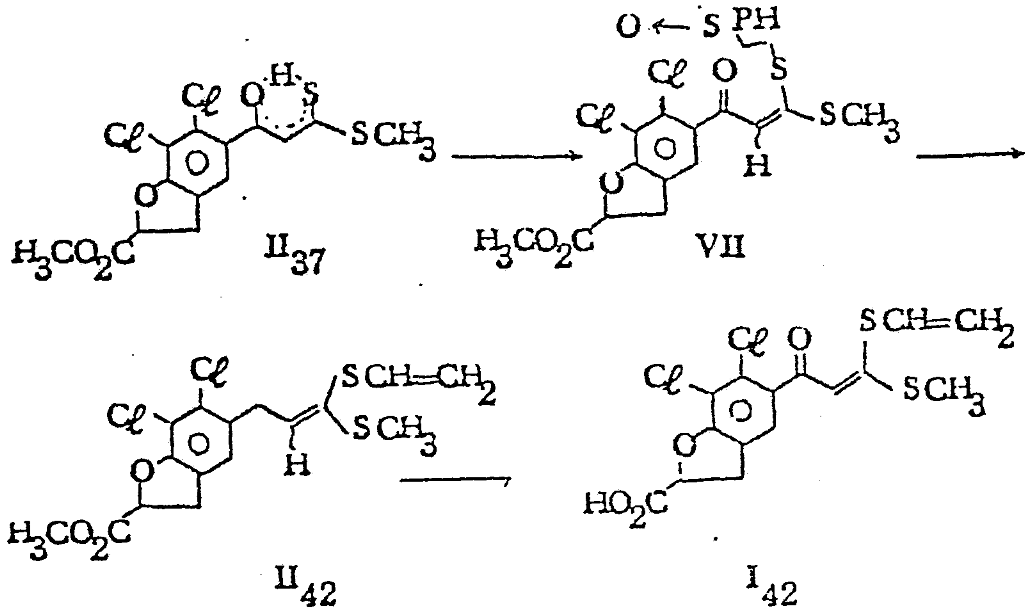

$II_{37}$ VII $II_{42}$ $I_{42}$

To 0.30 g (0.8 mmol) of the compound $II_{37}$ (prepared in Example 37) are added 0.221 g (1.6 mmol) of powdery potassium carbonate and 3 ml of acetonitrile while being stirred at room temperature, and 0.276 g (1.2 mmol) of 2-bromoethyl phenyl sulfide is added thereto. The mixture is allowed to react for 16 hours and then treated in the same manner as in Example 38 to give 0.4 g of the compound VII, yield 95.2 %.

NMR $\delta$ ppm ($CDCl_3$): 2.47(3H, s), 3.13-3.68 (6H, m), 3.80 (3H, s), 5.17-5.55 (1H, m), 6.32, 6.43 (1H, s-s), 7.18-7.72 (6H, m).

To 0.4 g (0.8mmol) of the compound VII is added 8 ml of toluene and the mixture is allowed to react on an oil bath (135-140 °C) for 16 hours while being stirred. The reaction product is treated by high performance liquid chromatography on a Lober column (Type A) with dichloromethane to give 0.213 g of the compound $II_{42}$, yield 69.8 %. mp. 101-102 °C.

NMR $\delta$ ppm ($CDCl_3$): 2.47, 2.52 (3H, s-s), 5.20-5.93 (3H, m),

6.33-7.13 (2H, m), 7.30 (1H).

The above product is hydrolyzed with an alkali in the same manner as in Example 1 without purification to give 0.170g of the compound $I_{42}$, yield 88.1 %, mp. 160-168 °C, which is recrystallized from ethyl acetate to give 0.133 g of grayish white crystals, yield 68.9 %, mp. 170-172 °C.

Anal. Calcd. (%) for $C_{18}H_{12}Cl_2O_2S_2$

: C 46.04 H 3.09, Cl 18.12, S 16.39,

Found (%): C 45.83, H 3.13, Cl 18.05, S 16.29.

IR $\nu$ max(Nujol): 2940, 2560, 1704 (3h,745), 1632, 1605cm$^{-1}$.

NMR $\delta$ ppm ($Me_2CO$ d=6): 2.58(3H,s), 3.23-4.10(2H,m), 5.30-6.30(4H,m), 6.57-7.23(2H,m), 7.35(1H).

## Example 43

6,7-Dichloro-5-[2-(1-methylallyl)-3,3-bis(methylthio)-propenoyl]-2,3-dihydro-1-benzofuran-2-carboxylic acid

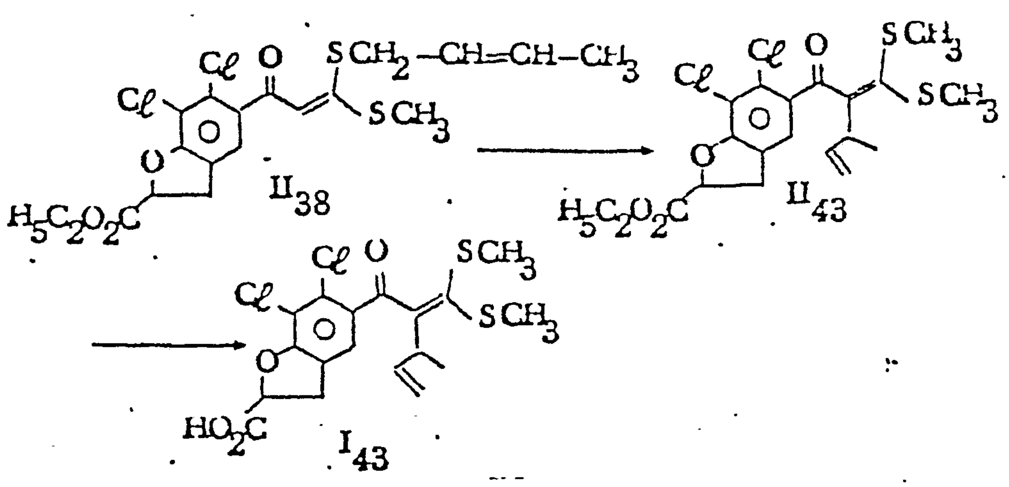
$II_{38}$ $II_{43}$ $I_{43}$

Under a reduced pressure (4/100-8/100 mmHg), 0.32 g (0.7 mmol) of the compound $II_{38}$ is heated on an oil bath (200 °C) for 5 minutes. The reaction product is chromatographed on a Lober column (Type A) with dichloromethane to give 0.173 g [containing 0.056 g

(17.5 %) of Ⅱ$_{34}$ remaining unchanged] of an oily material, to which 0.10 g (0.7 mmol) of powdery potassium carbonate, 0.085 g (1.4 mmol) of iodomethane and 2 ml of N,N-dimehtylacetoamide are added. The resulting mixture is allowed to react at room temperature for 14 hours while being stirred. The reaction product is chromatographed on a Lober column (Type A) with dichloromethane as an eluent to give 0.115 g of the compound Ⅱ$_{43}$ as an oil, yield 43.7 %.

NMR δ ppm ($CDCl_3$): 1.18-1.43 (6H, m), 2.00 (3H, s), 2.35 (3H, s), 3.15-3.72 (3H, m), 4.27 (2H, q), 4.87-6.23 (4H, m), 7.38 (1H).

To 0.115 g (0.2 mmol) of the compound Ⅱ$_{43}$ are added 0.5 ml of 1N potassium hydroxide and 1 ml of ethanol, and the mixture is hydrolyzed to give 0.10 g of the objective product I$_{43}$, yield 91.7 %, mp. 155-159 °C. This is recrystallized from a mixture of isopropyl ether/n-hexane to give 0.056 g of grayish white crystals, yield 51.4 %, mp. 162-164 °C.

Anal. Calcd. (%) for $C_{18}H_{18}Cl_2O_4S_2 \cdot 1/4 H_2O$

: C 49.37 H 4.26, Cl 16.20, S 14.65, $H_2O$ 1.03

Found (%): C 49.49, H 4.18, Cl 16.47, S 14.48, $H_2O$ 0.80

IR ν max (Nujol): 3260 (br), 1760, 1605, 1598 $cm^{-1}$.

## Example 44

6, 7-Dichloro-5-[3,3-bis(methylthio)-2-propenoyl]-2, 3-dihydro-1-benzofuran-2-yl methanol

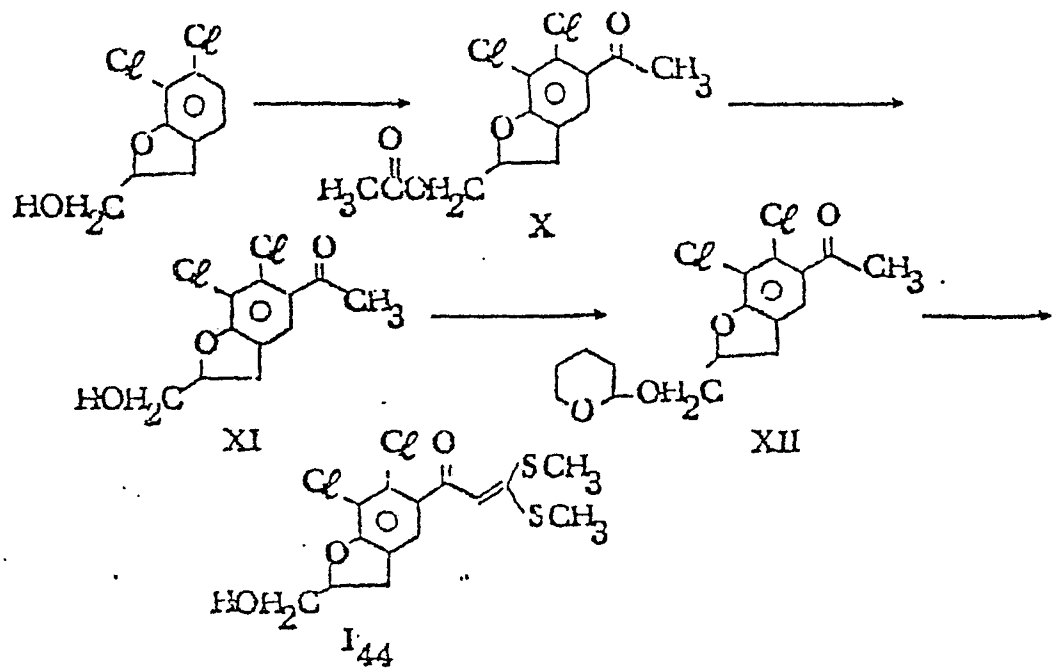

X

XI

XII

$I_{44}$

To a solution of 4.0 g (18.3 mmol) of 6,7-dichloro-2,3-dihydro-1-benzofuran-2-yl methanol (W. F. Hoffmann et.al. J. Med. Chem. 24, 865-873 (1981)) and 3.70 g (47.1 mmol) of acetyl chloride in 40 ml of dichloromethane is added in small portions 7.3 g of dry aluminium chloride over a 0.5 hour duration while being stirred under ice-cooling, and the mixture is allowed to react at room temperature for an hour. The reaction product is chromatographed on a Lober column (Type B) to give 4.98 g of the compound $X_{44}$, yield 89.9 %, mp. 90-93 °C. This is recrystallized from ethyl acetate to give 4.98 g of grayish white crystals, yield 89.9 %, mp. 93-94 °C.

Anal. Calcd. (%) for $C_{13}H_{12}Cl_2O_4$

: C 51.50 H 3.99, Cl 23.39,

Found (%): C 51.36, H 3.98, Cl 23.26.

IR: $\nu$ max (Nujol) 1735, 1685 $cm^{-1}$.

NMR $\delta$ ppm ($CDCl_3$): 2.07 (s,3H), 2.60 (3H,s), 2.83-3.70 (2H, m), 4.32 (2H, d), 4.97-5.45 (1H,m), 7.32 (1H).

To 4.78 g (15.8 mmol) of the compound X ₄₄ are added 20ml of methanol and 20 ml of 1N potassium hydroxide, and the mixture is allowed to react for an hour. The solvent is removed by evaporation and the residue is dissolved in dichloromethane. The dichloromethane layer is washed with water (2 times), dried over anhydrous magnesium sulfate, and decolored by chromatography on 4 g of silical-gel to give 3.51 g of the compound X I ₄₄, yield 85.2 %, mp. 102-105 °C. This is recrystallized to give grayish white crystals, mp. 105-106 °C.

Anal. Calcd. (%) for $C_{11}H_{10}Cl_2O_3$

: C 50.60 H 3.86, Cl 27.16,

Found (%): C 50.39, H 3.80, Cl 26.96.

IR: $\nu$ max (Nujol) 3500(br), 1677 $cm^{-1}$.

NMR: 2.58 (3H,s), 2.83-3.63 (3H, m-$D_2O$ 2H), 3.70-4.18 (2H, m), 4.85-5.33(1H,m), 7.28(1H).

To a solution of 3.30 g (12.6 mmol) of the compound X I and 2.13 g (25.3 mmol) of dihydropyrane in 35 ml of chloroform is added a catalytic amount of p-toluenesulfonic acid, and the mixture is allowed to react at room temperature for 3 hours while being stirred. Chloroform is evaporated and the residue is dissoved in ether, poured into an amnonia (2 ml)-ice mixture. The ether layer is separated, dried over anhydrous magnesium sulfate and evaporated to give 4.39 g of the compound X II, yield 100 %.

NMR: $\delta$ ppm 1.58 (6H,br), 2.60(3H,s), 2.93-4.13 (6H,m), 4.62(1H,br), 4.95-5.43(1H,m), 7.35(1H).

In the same manner as in Example 1 is treated 2.30 g (6.7 mmol) of the compound X II. To the reaction mixture are added 5 ml

of anisole and 5 ml of trifluoroacetic acid and the mixture is allowed to react at room temperature while bing stirred. The reaction product is treated by chromatography on a Lober column, crystallized from n-hexane, and recrystallized from ethyl acetate to give 0.47 g of pale yellow crystalline solid, yield 19.3 %, mp. 120-121 °C.

Anal. Calcd. (%) for $C_{14}H_{14}Cl_2O_3S_2$

: C 46.03 H 3.86, Cl 19.41, S 17.56

Found (%): C 45.84, H 4.03, Cl 19.46. S 17.52

NMR $\delta$ ppm ($CDCl_3$): 2.03 (1H,br), 2.50(3H,s), 2.53(3H,s), 3.12-340(2H,m), 3.95(2H,br-t,$D_2$0t), 4.85-5.32(1H,m), 6.48(1H,s), 7.27(1H).

Example 45

3,3-Bis(methylthio)-1-[6,7-dichloro-2-methoxymethyl-2,3-dihydro-1-benzofruan-5-yl]-acrylaldehyde

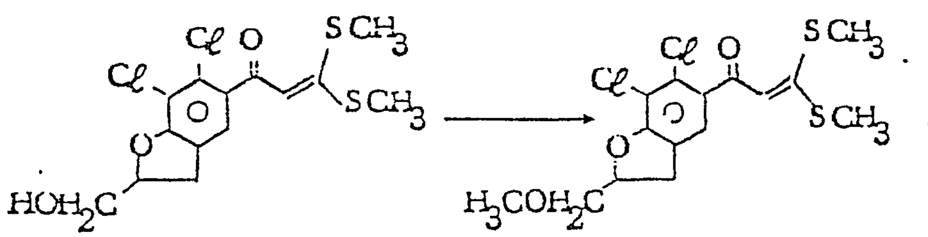

$I_{44}$ $I_{45}$

To a suspension of 0.037 g of 65 % sodium hydride in DMF is added a solution of 0.36 g (1 mmol) of the compound $I_{44}$ in 2 ml DMF under nitrogen atmosphere at room temperature while being stirred. Subsequently, an excess amount of methyl iodide added thereto and the mixture is allowed to react for 2 hours. The reaction product is chromatographed on a Lober column to give 0.065 g of the compound $I_{45}$, yield 17.4 %, mp. 132-134 °C, which is recrystallized from isopropyl ether-hexane to give 0.05 g of pale yellow crystalline product, yield 13.5 %, 135-136 °C.

Anal. Calcd. (%) for $C_{15}H_{16}Cl_2O_3S_2$

: C 47.49 H 4.56, Cl 18.69, S 16.91

Found (%): C 47.38, H 4.30, Cl 18.85, S 16.86.

NMR $\delta$ ppm ($CDCl_3$): [2.48(s), 2.53(s), 6H], 3.10-3.50 (5H,m), 3.63(2H,d), 4.87-5.37(1H,m), 6.47(1H,s), 7.25(1H).

Example 46

6,7-Dichloro-5-[3-mercapto-3-(methylthio)-2-propenoyl]-2,3-dihydro-1-benzofuran-2-yl-methanol

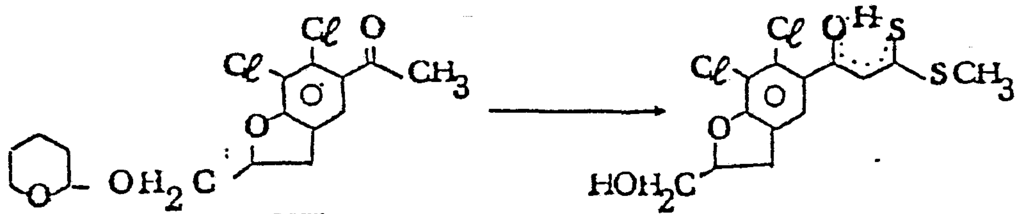

XII $I_{46}$

Using 3.43 g (9.9 mmol) of the compound XII, 2.27 g (29.8 mmol) of carbon disulfide, 6.0 g (29.8 mmol) of 4-methoxybenzylbromide, 0.88 g (23.8 mmol) of 65 % sodium hydride, 1.98 ml of N, N-dimethylacetoamide and 10 ml of ether, the reaction is made in the same manner as in Example 35. A portion (1.6 g) of 2.7 g of the reaction product [the remains (1.1 g) are used in Example 49] is allowed to react with 3.2 ml of anisole and 4 ml of trifluoroacetic acid for 1.5 hours and then to react with methyl iodide in the presence of anhydrous potassium carbonate in acetonitrile for 0.5 hours. The product is treated with a trifluoroacetic acid/anisole mixture and purified by using a Lober column to give 0.24 g (yield 11.7%) of the compound $I_{46}$, m.p. 100-102°C. This is recrystallized from isopropyl ether to give 0.20 g (yield 9.7%) of pale yellow crystals, m.p. 101-102°C.

Anal. Calcd. (%) for $C_{13}H_{12}Cl_2O_3S_2$

: C 44.45 H 3.44, Cl 20.19, S 18.25

Found (%): C 44.21, H 3.48, Cl 20.37, S 18.18.

IR $\nu$ max (Nujol): 3400 (br), 1595, 1609 $cm^{-1}$.

NMR $\delta$ ppm ($CDCl_3$): 2.02 (br, 1H, disappeared by addition of

$D_2O$), 2.63 (3H, s), 2.92-3.43(2H, m), 3.67-4.10(2H, m), 4.82-5.38 (1H, m), 6.62 (1H, s), 7.48 (1H), 14.97 (1H, s).

Example 47

6,7-Dichloro-5-[2-methyl-3-mercapto-3-(methylthio)propenoyl]-2,3-dihydro-1-benzofuran-2-yl-methanol

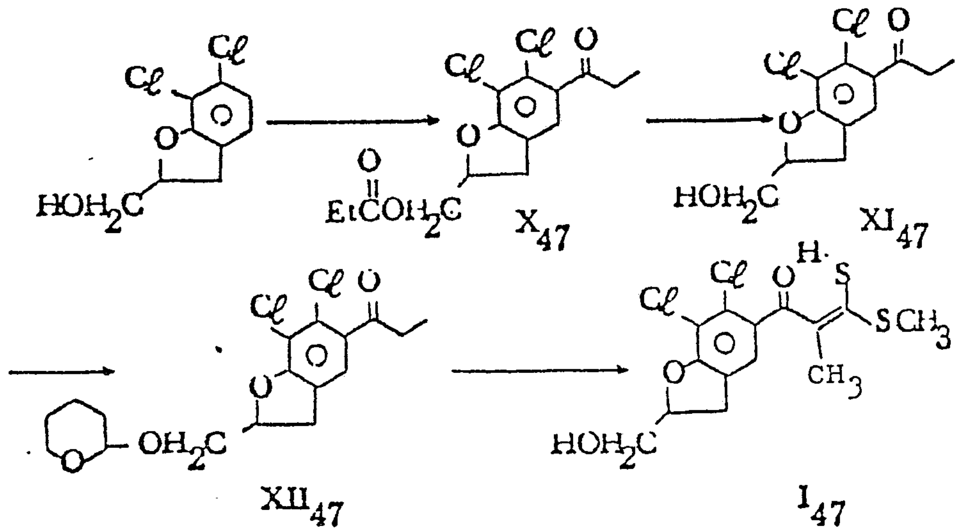

$X_{47}$ $XI_{47}$ $XII_{47}$ $I_{47}$

6,7-Dichloro-2,3-dihydro-1-benzofuran-2-yl-methanol (6.0 g, 27.4 mmol) is allowed to react with 7.6 g (82.1 mmol) of propyonyl chloride (82.1 mmol), 11.0 g (82.5 mmol) of anhydrous aluminium chloride, and 30 ml of dichloromethane in the same manner as in Example 44 to give 7.10 g (yield 82.7%) of the compound $X_{47}$, m.p. 49-50°C.

Anal. Calcd. (%) for $C_{15}H_{16}Cl_2O_4$

: C 54.39 H 4.87, Cl 21.41,

Found (%): C 54.19, H 4.92, Cl 21.37.

IR $\nu$ max (Nujol): 1742, 1697, 1607 $cm^{-1}$.

NMR $\delta$ ppm ($CDCl_3$): 1.10 (t, 3H), 1.17 (t, 3H), 2.35(2H, q), 2.92 (2H, q), 3.10-3.70 (2H, m), 4.30 (2H, d), 4.93-5.43 (1H, m), 7.17 (1H).

The compound $X_{47}$ (7.1 g, 21.4 mmol) is treated with 14 ml of ethanol and 26 ml of 1N-potassium carbonate in the same manner as in Example 44 to give 5.40 g (yield 91.5%) of the compound

X I $_{47}$, m.p. 94-95°C.

Anal. Calcd. (%) for $C_{12}H_{12}Cl_2O_3$

: C 52.38  H 4.40, Cl 25.77,

Found (%): C 52.15, H 4.45, Cl 25.63.

IR $\nu$ max (Nujol): 3510, 3430, 1682, 1654, 1604 $cm^{-1}$.

NMR $\delta$ ppm ($CDCl_3$): 1.15 (3H, t), 2.70-3.40 (7H, m), 3.55-4.17 (2H, m), 4.83-5.15 (1H, m), 7.17 (1H).

The compound X I $_{47}$ (5.0 g, 18.2 mmol) is treated with 3.05g (36.3 mmol) of dihydropyrane in the same manner as in Example 44 to give 6.53 g (yield 100%) of the compound X II $_{47}$.

NMR $\delta$ ppm ($CDCl_3$): 1.18 (3H, t), 1.63 (6H, br), 2.73-4.17 (8H, m), 4.67 (1H, br), 4.93-5.40 (1H, m), 7.20 (1H).

The compound X II $_{47}$ (4.0 g, 11.1 mmol) is allowed to react with 2.54 g (33.4 mmol) of carbon disulfide and 6.72 g (33.4 mmol) of 4-methoxybenzyl bromide in the same manner as in Example 46 to give the finally objective compound, which is recrystallized from water/isopropyl ether to give 0.060 g (yield 1.4%) of the compound I $_{47}$ as pale yellow crystals, m.p. 85-87°C.

Anal. Calcd. (%) for $C_{14}H_{14}Cl_2O_3S_2$ 1/4$H_2O$

: C 45.47, H 3.95, Cl 19.18, S 17.34, $H_2O$ 1.22,

Found (%): C 45.54, H 3.94, Cl 19.18, S 17.38, $H_2O$ 1.04.

IR $\nu$ max (Nujol): 3360, 1685, 1603 $cm^{-1}$.

NMR $\delta$ ppm ($CDCl_3$): [1.62 (d), 2.00 (s), 3H], [2.57 (s), 2.63 (s), 3H], 3.03-3.58 (3H, m), 3.70-3.95 (2H, m), 4.76-5.43 (1H, m).

Example 48

6,7-Dichloro-5-[2-methyl-3,3-bis(methylthio)propenoyl]-1-benzofuran-2-yl-methanol

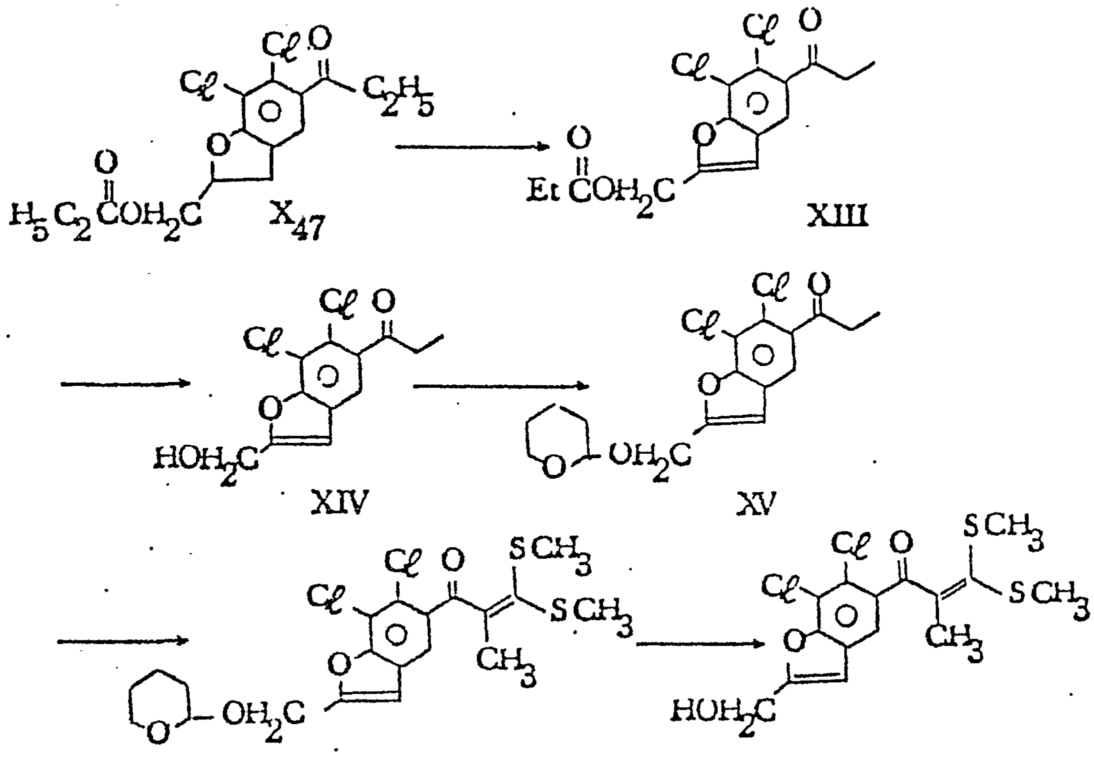

X$_{47}$ XIII XIV XV

II$_{48}$ I$_{48}$

In the same manner as in Example 33, 3.75 g (11.3 mmol) of the compound X$_{47}$ (Example 47) is treated to give 2.95 g (yield 79.2%) of the compound XIII.

NMR $\delta$ ppm ($CDCl_3$): 1.17, 1.22 (6H, t×2), 2.40 (2H, q), 2.93 (2H, q), 5.22 (2H, s), 6.78 (1H, s), 7.47 (1H, s).

In the same manner as in Example 33, 2.95 g (9.0 mmol) of the compound XIII is treated to give 2.40 g (yield 98.0%) of the compound XIV as grayish white crystals, m.p. 93-95°C.

Anal. Calcd. (%) for $C_{12}H_{10}Cl_2O_3$

: C 52.77, H 3.69, Cl 25.96,

Found (%): C 52.48, H 3.76, Cl 25.77.

IR $\nu$ max (Nujol): 3250, 1704 $cm^{-1}$.

In the same manner as in Example 33, 2.20 g (8.1 mmol) of the compound X IV is treated with dihydropyrane to give 2.30 g (yield 79.9%) of the compound X V.

NMR δ ppm ($CDCl_3$): 1.22 (3H, t), 1.70 (6H, m), 2.95 (2H, q), 3.25-4.33 (2H, m), 4.50-5.00 (3H, m), 6.73 (1H, s), 7.45 (1H, s).

In the same manner as in Example 1, 2.10 g (5.9 mmol) of the compound X V is allowed to react for 18 hours to give 2.10 g (yield 77.2%) of the compound $II_{48}$.

NMR δ ppm ($CDCl_3$): 1.67 (6H, m), 1.90 (3H, s), 2.27 (3H, s), 2.35 (1H, s), 3.33-4.17 (2H, m), 6.42 (3H, m), 6.73 (1H, s), 7.62 (1H, s).

In the same manner as in Example 44, 0.65 g (1.4 mmol) of the compound $II_{48}$ is treated with anisole/trifluoroacetic acid to give 0.430 g (yield 80.8%) of the compound $I_{48}$, m.p. 120-122°C. This is recrystallized from isopropyl ether to give 0.352 g (yield 66.2%) of pale yellow crystals, m.p. 122-123°C.

Anal. Calcd. (%) for $C_{15}H_{14}Cl_2O_3S_2$

: C 47.75, H 3.74, Cl 18.79, S 17.00,

Found (%): C 47.63, H 3.81, Cl 18.65, S 16.88.

IR ν max (Nujol): 3580, 3420, 1651, 1606 $cm^{-1}$.

NMR δ ppm ($CDCl_3$): 1.88 (3H, s), 2.28 (3H, s), 2.35 (3H, s), 2.98 (1H, t), 4.75 (2H, d, $D_2O$, s), 6.62 (1H, s), 4.78 (1H, s).

Example 49

6,7-Dichloro-5-[2-(1,3-dithioran-2-ylidene)acetyl]-2,3-dihydro-1-benzofuran-2-yl-methanol

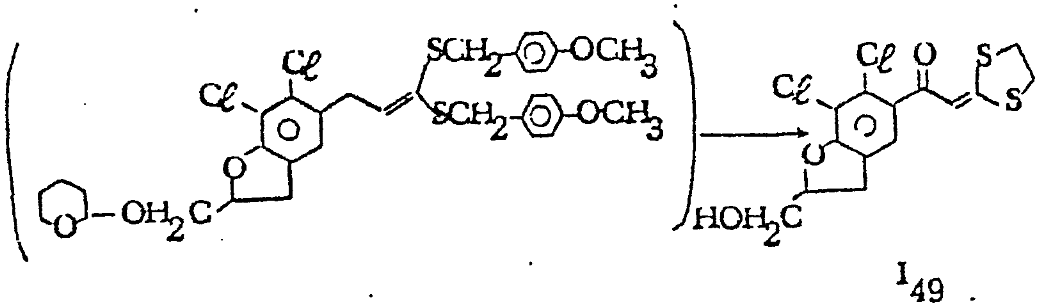

$I_{49}$.

In the same manner as in Example 19, 1.0 g of the compound prepared from the 1st step of the processes in Example 47 is treated with 0.28 g of ethanedithiol to give 0.15 g (yield 11.2%) of the compound $I_{49}$, m.p. 165-170°C. This is recrystallized from ethyl acetate to give 0.10 g (yield 7.5%) of pale yellow crystals, m.p. 170-171°C.

Anal. Calcd. (%) for $C_{14}H_{12}Cl_2O_3S_2$

: C 46.28, H 3.33, Cl 19.52, S 17.65,

Found (%): C 46.24, H 3.38, Cl 19.68, S 17.38.

IR $\nu$ max (Nujol): 3410, 1605, 1590 $cm^{-1}$.

NMR $\delta$ ppm ($CDCl_3$): 2.81 (1H, br-t), 3.07-4.25 (8H, m), 4.80-5.33 (1H, m), 6.93 (1H, s), 7.20 (1H).

Example 50

6,7-Dichloro-5-[2-(1,3-dithioran-2-ylidene)acetyl]-2,3-dihydro-1-benzofuran-2-yl-methanol acetate

$I_{49}$

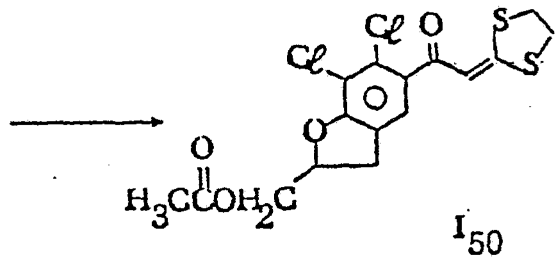

$I_{50}$

To a solution of 0.075 g (0.2 mmol) of the compound $I_{49}$ (Example 49), 0.042 g (0.4 mmol) of triethylamine, and a catalytic amount of 4-(N,N-dimethylamino)-pyridine in 2 ml of dry dichloromethane is added 0.035 g (0.4 mmol) of acetyl chloride while being stirred under ice-cooling, and the mixture is allowed to react for 0.5 hour. The reaction product is chromatographed on a Lober column (type A) with benzene/ethyl acetate (10/1) as an eluent to give 0.08 g (yield 95.2%) of the compound $I_{50}$, m.p. 126-128°C. This is recrystallized from ether/ ethyl acetate to give 0.05 g (yield 59.5%) of the compound $I_{50}$ as grayish white crystals, m.p. 128-129°C.

Anal. Calcd. (%) for $C_{16}H_{14}Cl_2O_4S_2$

: C 47.41, H 3.48, Cl 17.50, S 15.82,

Found (%): C 47.52, H 3.66, Cl 17.46, S 15.61.

IR $\nu$ max (Nujol): 1726, 1738, 1638, 1620, 1605 $cm^{-1}$.

NMR $\delta$ ppm ($CDCl_3$): 2.08 (3H, s), 2.95-3.72 (2H, m), 4.32 (2H, d), 4.98-5.43 (1H, m), 7.00 (1H, s), 7.28 (1H).

Example 51

1-{6,7-Dichloro-5-[3,3-bis(methylthio)-2-propenoyl]-2,3-dihydro-1-benzofuran-2-yl}-1-methyl ketone

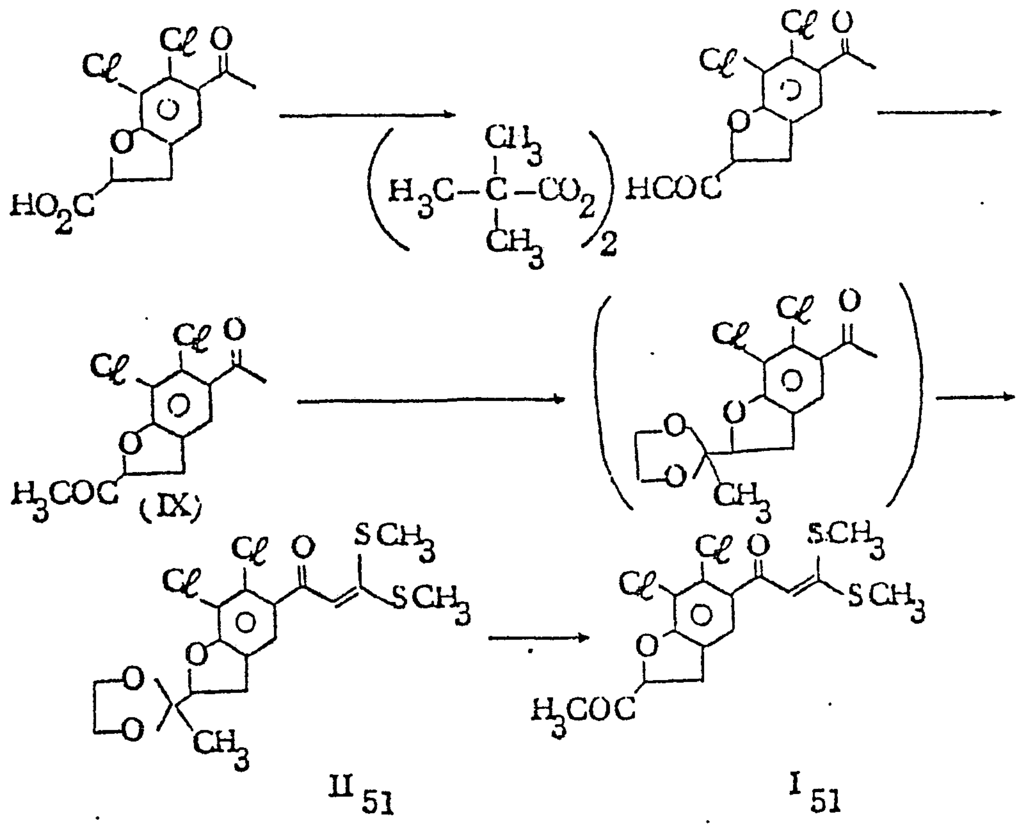

(IX)

$II_{51}$ $I_{51}$

With thionyl chloride is treated 0.70 g (2.5 mmol) of 5-acetyl-6,7-dichloro-2,3-dihydro-1-benzofuran-2-carboxylic acid (W. F. Hoffman *et.al* J. Med. Chem., *24*, 865-873 (1981)) to give the corresponding acid chloride, the solution of which dissolved in ether is treated with 1.1 g (5.1 mmol) of t-butyl malonate in ether in the presence of 0.18 g (4.9 mmol) of sodium hydride. The reaction product is, without isolation and purification, allowed to react with 3 ml of trifluoroacetic acid at room temperature for an hour, and then the trifluoroacetic acid is removed by evaporation. To the residue is added 15 ml of toluene and the mixture is refluxed under heating for 2.5 hours. The product is chromatographed on a Lober column (type B) with benzene/ethyl acetate (10/1) as an eluent to give 0.32 g (yield 46.0%) of the compound IX.

NMR: 2.35 (3H, s), 2.63 (3H, s), 3.27-3.63 (2H, m), 5.12-5.40 (1H, m), 7.37 (1H).

IR $\nu$ max ($CHCl_3$): 1715, 1680, 1690, 1605 $cm^{-1}$.

A mixture of 1.50 g (5.5 mmol) of the compound Ⅸ, 5.0 ml of ethylene glycol, and a catalytic amount of p-toluenesulfonic acid in benzene is refluxed under heating for 2 hours, during which time the water generated is removed as azeotrope. The crude product (1.80 g) is treated, isolated and purified in the same manner as in Example 1 to give 0.180 g (yield 7.8%) of the compound $II_{61}$ as an oil.

NMR $\delta$ ppm ($CDCl_3$): 1.37 (3H, s), [2.47 (s), 2.52 (s), 6H], 3.20-3.33 (2H, m), 4.00 (4H, br), 4.77-5.05 (1H, m), 6.47 (1H, s), 7.25 (1H).

The compound $II_{61}$ (0.180 g, 0.4 mmol) is allowed to react with 2 ml of trifluoroacetic acid at room temperature for 6 hours to give 0.120 g (yield 74.8%) of the compound $I_{61}$, m.p. 98-101°C. This is recrystallized from ether/ethyl acetate to give 0.097 g (yield 59.6%) of grayish white crystals, m.p. 102-103°C.

Anal. Calcd. (%) for $C_{16}H_{14}Cl_2O_3S_2$ 1/4$H_2O$

: C 47.20, H 3.83, Cl 18.57, S 16.80, $H_2O$ 1.18,

Found (%): C 47.10, H 3.74, Cl 18.51, S 16.59, $H_2O$ 1.00.

IR $\nu$ max (Nujol): 3420 (br), 1725, 1623, 1598, 1605 $cm^{-1}$.

NMR $\delta$ ppm ($CDCl_3$): 2.33 (3H, s), 2.53 (6H, s), 3.30-3.60 (2H, m), 5.08-5.37 (1H, m), 6.43 (1H, s), 7.27 (1H).

Example 52

(2R, 3S/2S, 3R)-6,7-Dichloro-5-[3,3-bis(methylthio)-2-propenoyl]-3-methyl-2,3-dihydro-1-benzofuran-2-carboxylic acid

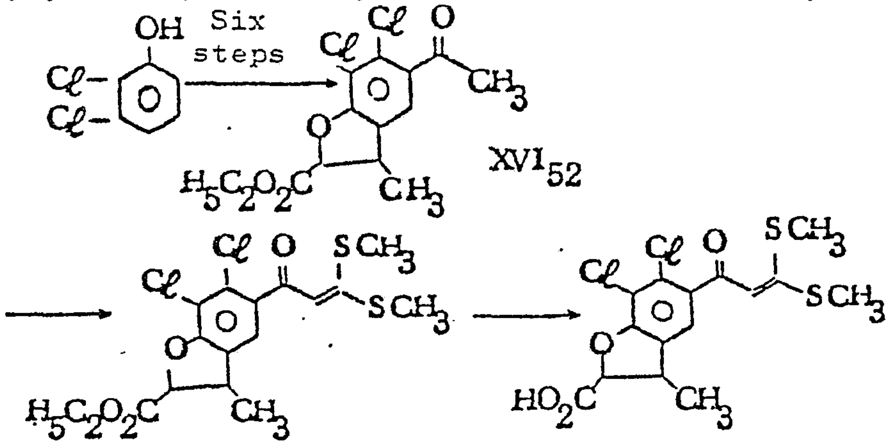


XVI$_{52}$

II$_{52}$ I$_{52}$

The starting material 2,3-dichlorophenol (made by Aldrich Chemical Co.) is allowed to react with crotyl bromide in place of allyl bromide in the same manner as disclosed in W. F. Hoffman *et al* J. Med. Chem. *24* 865 (1981) to give the compound XVI in 12.2% yield, m.p. 92-93°C.

NMR $\delta$ ppm ($CDCl_3$): 1.20-1.43 (6H, m), 2.62 (3H, s), 3.63-3.77 (1H, m), 4.28 (2H, q), 5.37 (1H, d, J=9.8Hz), 7.23 (1H).

In the same manner as in Example 1, 0.8 g (2.5 mmol) of the compound XVI is treated to give 0.187 g (yield 17.6%) of the compound II$_{52}$.

NMR $\delta$ ppm ($CDCl_3$): 1.17-1.43 (6H, m), 2.52 (6H, s), 3.48-4.45 (3H, m), 5.38 (1H, d, J=9.8Hz), 6.48 (1H, s), 7.28 (1H).

In the same manner as in Example 1, 0.187 g (0.4 mmol) of the compound II$_{52}$ is hydrolyzed to give 0.174 g (qu. yield) of the compound I$_{52}$, m.p. 236-239°C. This is recrystallized from ethyl acetate to give 0.137 g (yield 77.4%) of pale yellow crystals, m.p. 236-239°C.

Anal. Calcd. (%) for $C_{15}H_{14}Cl_2O_4S_2$ 1/4$H_2O$

: C 45.29, H 3.67, Cl 17.83, S 16.12, $H_2O$ 1.13,

Found (%): C 45.41, H 3.68, Cl 17.75, S 15.88, $H_2O$ 1.00.

Example 53

(2R, 3R/2S, 3S)-6,7-Dichloro-5-[3,3-bis(methylthio)-2-propenoyl]-3-methyl-2,3-dihydro-1-benzofuran-2-carboxylic acid

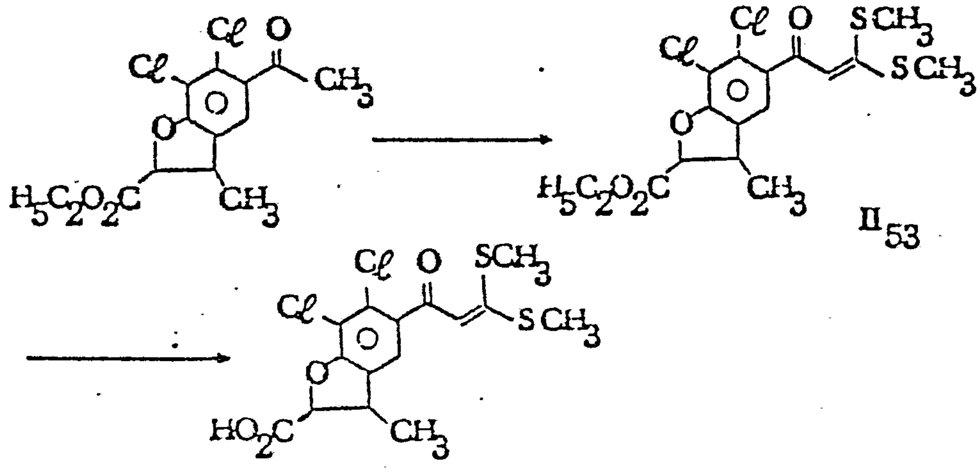

$II_{53}$

$I_{53}$

In the same manner as in Example 52, 0.80 g (2.5 mmol) of the compound $XVI_{53}$:

NMR δ ppm ($CDCl_3$): 1.20-1.56 (6H, m), 2.63 (3H, s), 3.35-4.00 (1H, m), 4.30 (2H, q), 4.90 (1H, d, J=6.4Hz), 7.33 (1H).

which is obtained in Example 52 as the *trans* isomer (yield 12.5%) of the compound $XVI_{52}$ (*cis* form),

is treated to give 0.085 g (yield 8.0%) of the compound $II_{53}$.

NMR δ ppm ($CDCl_3$): 1.17-1.53 (6H, m), 2.45 (3H, s), 2.56 (3H, s), 3.37-4.00 (1H, m), 4.25 (2H, q), 4.80 (1H, d, J=6.4Hz), 6.42 (1H, s), 7.17 (1H).

In the same manner as in Example 52, 0.085 g (0.2 mmol) of the compound $XVI_{53}$ is hydrolyzed to give 0.079 g (qu. yield) of the compound $I_{53}$, m.p. 122-124°C. This is recrystallized from ether to give 0.050 g (yield 63.3%) of pale yellow crystals, m.p.

124-125°C.

Anal. Calcd. (%) for $C_{15}H_{14}Cl_2O_4S_2$

: C 45.80, H 3.59, Cl 18.03, S 16.31,

Found (%): C 45.64, H 3.70, Cl 18.13, S 16.21.

Example 54

6,7-Dichloro-5-[3,3-bis(methylthio)-2-propenoyl]-benzo[b]thiophene-2-carboxylic acid

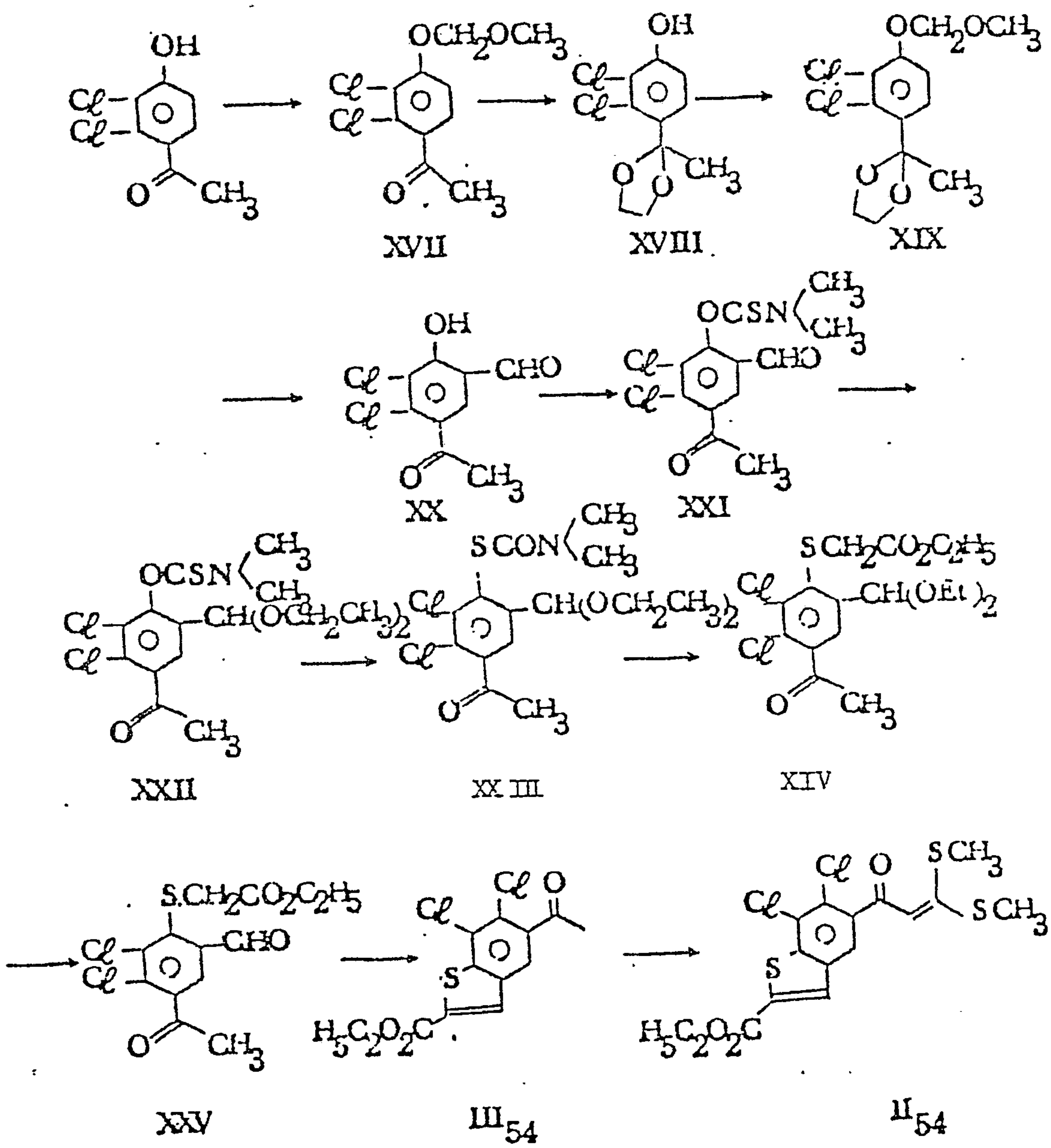

XVII XVIII XIX XX XXI XXII XXIII XIV XXV III54 II54

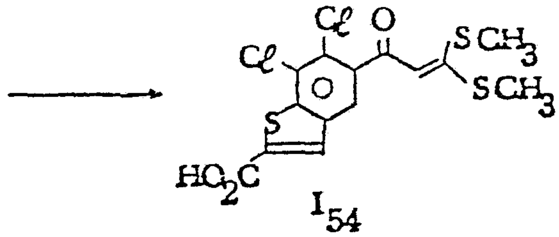

$I_{54}$

To 6.30 g (30.7 mmol) of 2, 3-dichloro-4-hydroxybenzophenone [Sprague, James, M. (Merck) U.S. 345, 312] are added 8.5 g (61.5 mmol) of anhydrous powderly potassium carbonate and 63 ml of acetonitrile, and then 3.7 g (46.0 mmol) of chloromethyl methyl ether is added at room temperature while being stirred, and the resulting mixture is allowed to react for 3 hours. The unpurified reaction product is dissolved in 300 ml of benzene, and 5 ml of ethylene glycol and catalytic amount of p-toluenesulfonic acid are added, and the mixture is refluxed for continuous dehydration for 8 hours while being stirred to give 5.90 g of the compound XVIII, yield 77.1 %, mp. 150-152 °C.

NMR ($CDCl_3$) $\delta$ ppm: 1.78(3H,s), 3.57-3.90(4H,m), 5.83(1H, brs disappeared by addition of $D_2O$), 6.85-7.43(2H,d-d).

A mixture of 5.90 g (23.7 mmol) of the compound XVIII, 6.55 g (47.4 mmol) of anhydrous powderly potassium carbonate, 2.10 g (26.1 mmol) of chloromethyl methyl ether and 60 ml of acetonitril is allowed to react at room temperature for 2 hours while being stirred to give 6.60 g of the compound XIX, quantitative yield.

NMR ($CDCl_3$) $\delta$ ppm: 1.78(3H,s), 3.50(3H,m), 3.50-4.10a(4H,m), 5.25(2H,s), 7.02-7.50(2H,d-d).

In the same manner as disclosed in the literure [Holdor, Christensen, Synth. Comm. 5(1), 65-78] is treated 6.60 g of the compound XIX to give 3.72 g of the compound XX, yield 67.5 %. To a solution of 1.30 g (5.6 mmol) of the compound XX in 6.5 ml

of N,N-dimethylformamide is added a suspension of 0.227 g (6.1 mmol) of 65 % sodium hydride in 3 ml of DMF over a 1/6 hour period, and then added 1.0 g (8.1 mmol) of dimethylthiocarbamoyl chloride (Made by Aldrich), and the resultant mixture is allowed to react at room temperature for 1/2 hours and then on an oil bath at 60-65 °C for 2 hours.

The reaction product is treated with methanol to give 0.922 g of the compound XXI, yield 51.6 %, mp. 121-122 °C.

NMR ($CDCl_3$) δ ppm: 2.63(3H,s), 3.38(6H,m), 7.65(1H,m), 9.92(1H,s).

To a solution of 1.70 g (5.3 mmol) of the compound XXI in 12 ml of dry ethanol are added 0.051 g (1 mmol) of aluminium chloride and 1.4 ml (8.5 mmol) of orthoethyl formate, and the mixture is refluxed for 2 hours while being stirred to give 2.10 g of the reaction product (yield qu). To this, without purification, is added 18 ml diphenyl ether and the mixture is allowed to react in nitrogen atmosphere on an oil bath at 225-230 °C for 0.5 hour. Diphenyl ether is removed by evaporation under reduced pressure and the residue is chromatographed on 40 g of alumina to give 1.65 g of the compound XXIII. (yield 79.0 %)

NMR ($CDCl_3$) δ ppm: 1.20(6H,t), 2.62(3H,s), 3.12(6H,brs), 3.58(4H,qu), 5.77(1H,s), 7.73(1H,s).

To a solution of 1.65 g (4.2 mmol) of the compound XXIII in 33 ml of methanol is added 3.7 ml (9.3 mmol) of 10 % sodium hydroxide in nitrogen atmosphere while being stirred. The mixture is refluxed under heating for 2.5 hours. The reaction mixture is evaporated to dryness. To a solution of the residue dissolved in 10 ml of acetonitril is added 0.55 ml (5.0 mmol) of ethyl bromoacetate in nitorgen atomsphere while being stirred, and the mixture is allowed to react at room temperature for 3 hours. The

reaction mixture is chromatographed on a Lober column (Type B) with a benzene/ethyl acetate (10/1) to give 1.20 g of the compound XXIV, yield 69.9 %.

NMR ($CDCl_3$) δ ppm: 1.62-1.35(9H,m), 2.62(3H,s), 3.48-4.25 (8H,m), 5.98(1H,s), 7.67(1H,s).

To 1.20 g (2.9 mmol) of the compound XXIV is added 3.6 ml of trifluoroacetice acid and the mixture is allowed to react for 1 hour in nitorgen atmosphere while being stirred. The reaction product is chromatographed on a Lober column (Type B) with benzene/ethyl acetate (10/1) as an eluent to give 0.650 g of the compound XXV, yield 66.0 %.

NMR ($CDCl_3$) δ ppm: 1.17(3H,t), 2.63(3H,s), 3.70(2H,s), 4.08(2H, q), 7.72(1H,s), 10.75 (1H,s).

To 0.28 g (0.8 mmol) of the compound XXV is added 0.3 ml of 1N-soudim hydroxide at room temperature in nitorgen atmoshpere and the mixture is allowed to react for 1 hour (or in 1.4 ml of pyridine and 0.4 ml of pyperidine at 100-105 °C for 1 hour.). The reaction product (0.27 g) is chromatographed on a Lober column (Type B) to give 0.150 g of the compound $III_{64}$, yield 56.6 %.

NMR ($CDCl_3$) δ ppm: 1.42(3H,t), 2.67(3H,s), 4.08(2H,q), 7.87(1H,s), 8.02(1H,s).

The compound $III_{64}$ (0.15 g, 0.5 mmol) is allowed to react in the same manner as in Example 1 to give 0.075 g of the compound $II_{64}$, yield 37.7 %.

NMR ($CDCl_3$) δ ppm: 1.40(3H,t), 2.48(3H,s), 2.53(3H,s), 4.38(2H,q), 7.80(1H,s), 7.93(1H,s).

In the same manner as mentioned in Example 5 is hydrolyzed 0.075 g (0.2 mmol) of the compound $II_{64}$ to give 0.066 g of the compound $I'_{64}$, quantitative yield, mp. 223-225 °C (dec.). This is recrystallized from ethyl acetate to give 0.038 g of grayish white

crystals, yield 57.8 %, mp. 225-227 °C (dec.).

Anal. Calcd. (%) for $C_{14}H_{10}Cl_2O_3S_3$ 1/4$H_2O$

: C 42.26, H 2.66, Cl 17.82, S 24.18, $H_2O$ 1.13,

Found (%): C 42.02, H 2.79, Cl 17.72, S 24.02, $H_2O$ 1.00.

Example 54-A

6,7-Dichloro-5-[3,3-bis(methylthio)-2-propenoyl]-2,3-dihydrobenzo[b]thiophene-2-carboxylic acid

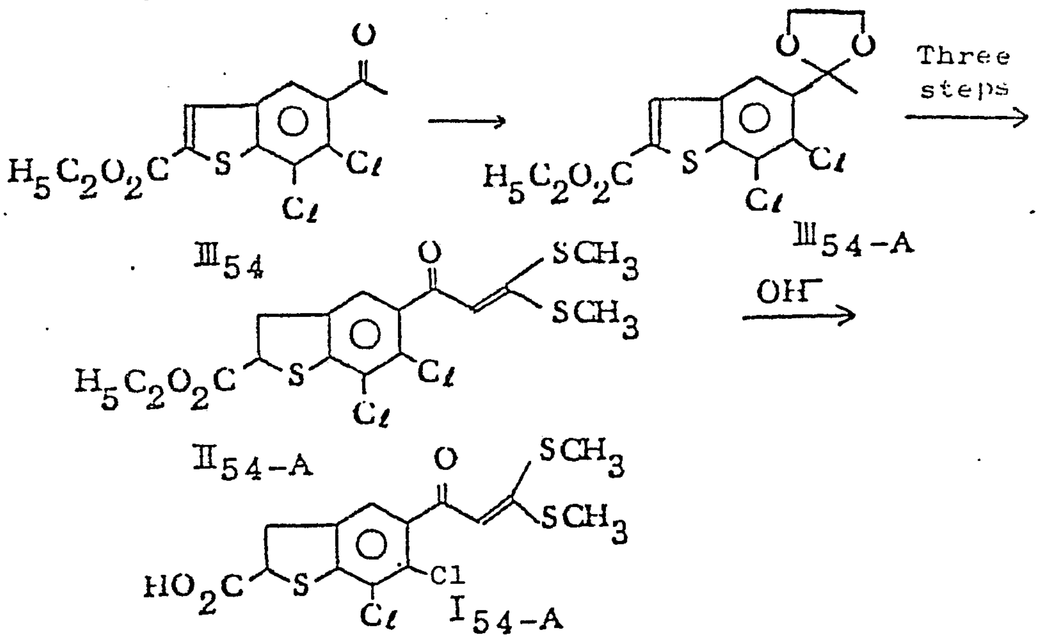


III54 III54-A II54-A I54-A

The mixture of 0.260 g (0.8 mmol) of the compound $III_{54}$, 1 ml of ethylene glycol, a catalytic amount of p-toluenesulfonic acid and 10 ml of benzene is refluxed for 48 hours while being stirred, during which time the water produced is removed continuously. The reaction mixture is cooled, poured into a mixture of ammonia water and ice and extracted with benzene. The organic layer is washed tiwce with water, dried over anhydrous magnesium sulfate and the benzene is removed to give 0.280 g of the compound $III_{54-A}$, yield 88.0 %, mp. 114-116 °C.

NMR $\delta$ ppm ($CDCl_3$): 1.42(3H,t), 1.83(3H,s), 3.53-4.38

(6H,m), 7.92(1H,s), 8.00(1H,s).

To 0.280 g (0.8 mmol) of the compound $III_{54-A}$ are added 5 ml of dioxane and 5 ml of 1N-sodium hydroxide, and the mixture is allowed to react for 3 hours at room temperature and then evaporated. To a solution of the residue dissolved in 10 ml of water is added sodium amalgam (perpared from 33 mg of sodium and 1.3 mg of mercury) in small portions over a 30 minute period and the mixture is allowed to react for further 6 hours. The precipitate is removed and resulting alkaline solution is acidfied (pH 3-4) with 10 % hydrochloric acid. This solution is extracted three times with ether. The organic layer is washed with water, dried over anhydrous magnesium sulfate and evaporated to give residue, to which are added 10 ml of ethanol and a catalytic amount of conc. sulfuric acid. The mixture is refluxed for 4 hours while being stirred. The reaction product (0.20 g) without purification is allowed to react in the same manner as in Example 1 and chromatographed on a Lober column (Type A) with ethyl acetate/benzene (1/15) as an eluent to give 0.04 g $II_{54-A}$, yield 12.2 %.

NMR $\delta$ ppm ($CDCl_3$): 1.28(3H, t), 2.52(3H, s), 2.55(3H, s), 3.50-3.85(2H, m), 4.20(2H, q), 4.37-4.60(1H, m), 6.60(1H, s), 7.63(1H).

The compound $II_{54-A}$ (0.04 g, 0.1 mmol) is hydrolyzed with an alkali to give the compound $I_{54-A}$ in quantitative yield, mp. 170-173 °C. This is recrystallized from ether to give 0.02 g of yellow crystals, mp. 173-174 °C, yield 53.5 %.

Anal. Calcd. (%) For $C_{14}H_{12}Cl_2O_3S_3$

: C 42.53, H 3.06, Cl 17.94, S 24.33,

Found (%): C 42.41, H 3.01, Cl 18.15, S 24.51.

Example 54-B

6,7-Dichloro-5-[2-methyl-3,3-bis(methylthio)propenoyl]-2,3-dihydro-benzo[b]thiophene-2-carboxylic acid

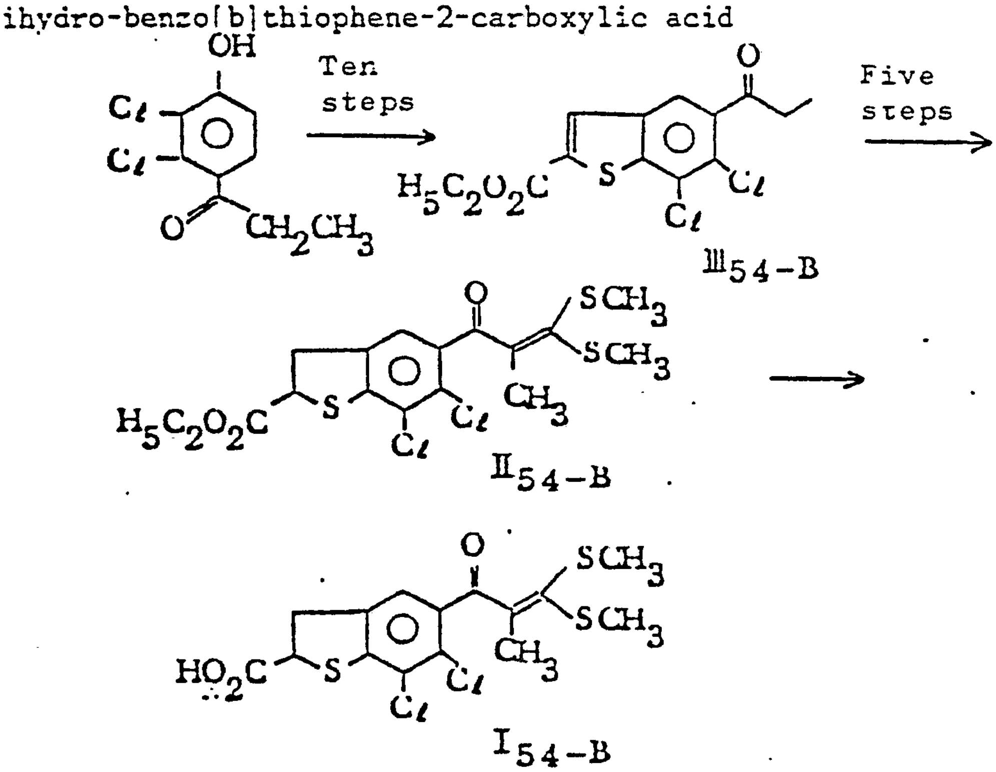


III54-B

II54-B

I54-B

A strating material, 2,3-dichloro-4-hydroxy-propiophenone, is allowed to react in the same manner as in Example 54 to yield the compound $III_{54-B}$, which is allowed to react in the same manner in as Example 54-A to yield $I_{54-B}$. The yield and physical constants of intermediates are listed as follows.

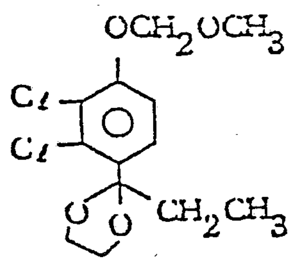

NMR : 0.87 (3H, t) 2.15 (2H, q)
3.52 (3H, s) 3.62~4.18 (4H, m)
5.25 (2H, s) 6.95~7.55 (2H, d-d)

XIX B
70.0 %

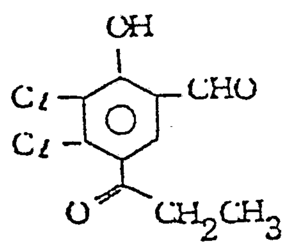

NMR : $^{CDCl_3}_{ppm}$ 1.20 (3H, t)
2.93 (2H, q) 7.63 (1H)
9.90 (1H, s) 11.73 (1H, br)
IR : $\nu^{CHCl_3}_{max}$ 3480, 3250 (br)
1695 (sh) 1660, 1603

XX
85.0 %

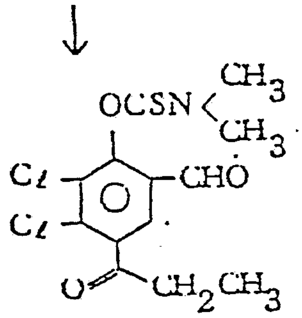

NMR : $^{CDCl_3}_{ppm}$ 1.22 (3H, t)
2.95 (2H, q) 3.45 (6H, s)
7.78 (1H, s) 9.95 (1H, s)

XXI B
58.5 %

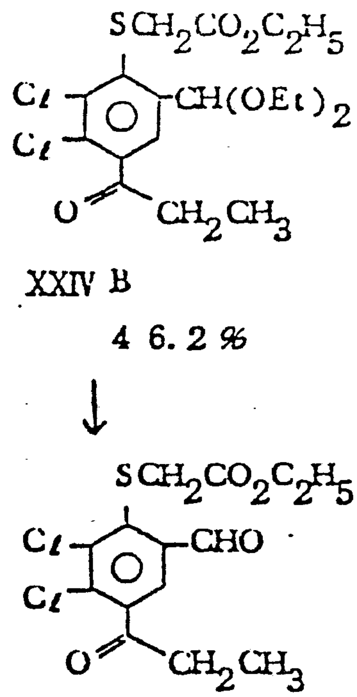

XXIV B

46.2 %

NMR : $\frac{CDCl_3}{ppm}$ 1.05~1.35(12H,m) 2.90(2H,q) 3.47~3.82(4H,m) 4.05(2H,q) 5.95(1H,s) 7.53(1H,s)

XXV-B

79.4 %

NMR : $\frac{CDCl_3}{ppm}$ 1.23, 1.42(6H, t×2) 2.96(2H,q) 4.08(2H,q) 4.00(2H,s) 7.58(1H,s) 10.67(1H,s)

Compound $III_{54-B}$: yield 0.680 g (yield 96.4 %), mp. 98-99 °C.

NMR δ ppm ($CDCl_3$): 1.12-1.55(6H,m), 2.97(2H,q), 4.42(2H,q), 7.75(1H,s), 7.98(1H,s).

Compound $II_{54-B}$: (yield 7.4 %)

NMR δ ppm ($CDCl_3$): 1.28(3H,t), 2.08(3H,s), 2.15(3H,s), 2.35(3H, s), 3.40-3.93(2H,m), 4.00-4.63(3H,m), 7.57(1H).

Compound $I_{54-B}$: (yield 91.2 %), mp. 131-132 °C.

Anal. Calcd. (%) for $C_{15}H_{14}Cl_2O_3S_3$

: C 44.01, H 3.45, Cl 17.41, S 23.50,

Found (%): C 44.05, H 3.24, Cl 17.35, S 23.41.

Example 55

Preparation of 6,7-dichloro-5-(3- ethylamino-3-methylthio-2-propenoyl)-2, 3-dihydro-benzofuran-2-carboxylic acid [3]

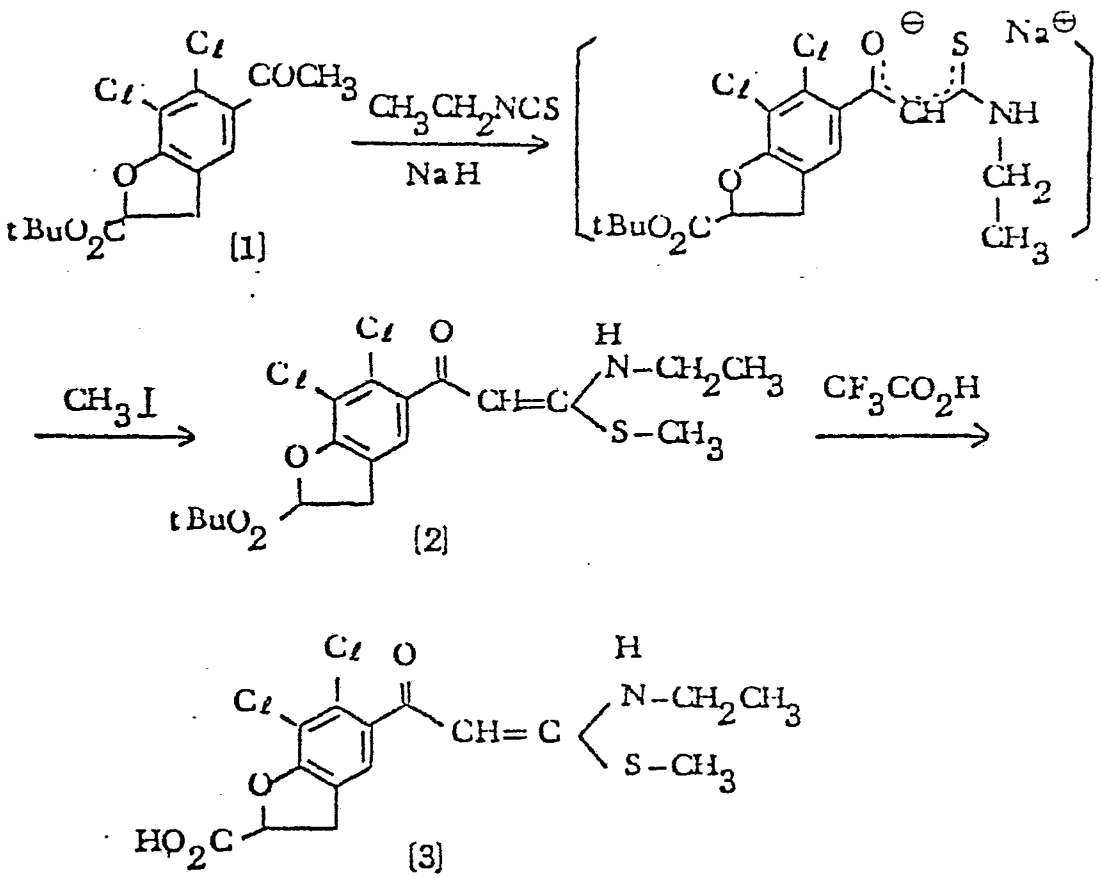


[Step-1]

A solution of 1.65 g (5 mmol) of tert-butyl 6,7-dichloro-5-acetyl-2,3-dihydro-benzofuran-2-carboxylate [1] in 4 ml of dry dimethylformamide is added to a mixture of 0.20 g (5 mmol) of 60 % oily sodium hydride, 0.53 g (6 mmol) of ethyl isothiocyanate and 1 ml of DMF in nitrogen atmosphere at 5-10 °C while being stirred, and the resultant mixture is kept at the same temperature for 2.5 hours. To the reaction mixture is added 0.85 g (6 mmol) of methyl iodide and the mixture is allowed to react for 2.5 hours. After addition of an ammonium chloride solution, the mixture is extracted with ether. The organic layer is washed with a saturated aqueous solution of sodium chloride, dried over anhydrous magnesium sulfate, and then evaporated under reduced pressuer. The residue is isolated and purified by medium pressure column

chromatography on silica gel to give 1.6 g of the compound [2] as an oil, yield 76.5 %.

IR: ν max ($CHCl_3$) 1750 (CO-O),
1609(sh)-1565(br) (aminopropenoyl portion) $cm^{-1}$.

NMR δ ppm ($CDCl_3$): 10.4(1H,br) , 7.12(1H,s), 5.26(1H,s), 5.18(1H,d-d), 3.80-3.32(4H,m), 2 .39(3H,s) 1.49, 1.32(12H,s + t).

[Step-2]

To 1.3 g of the compound [2] perpared in Step-1 is added 13 ml of trifluoroacetic acid and the mixture is stirred for 0.5 hour at room temperature. Trifluoroacetic acid is removed under reduced pressure to give residue which is crystallized from a small amount of ether. The resulting crystals are collected by filtration and washed with a small amount of ether to give 1.15 g of the titled compound [3]. This is recrystallized from acetone to give 0.81 g of yellowish white crystals, yield 69.8 % (Yield from compound [1] is 53.4 %), mp. 247-249 °C (dec.).

Anal. Calcd. (%) for $C_{15}H_{15}Cl_2NO_4S$
: C 47.88 H 4.02, Cl 18.85, N 3.72,
Found (%): C 47.76, H 3.90, Cl 19.03, N 3.80.

IR: ν max (Nujol) 3300-2200(br) 2200-1800(br) 1735, 1610, 1565 $cm^{-1}$.

NMR δ ppm (DMSO d-6): 11.30(1H,br), 7.29(1H,s), 5.41(1H, d-d), 5.21(1H,s), 3.80-3.20(4H,m), 2.42(3H,s), 1.23(3H,t).

## Example 56-66

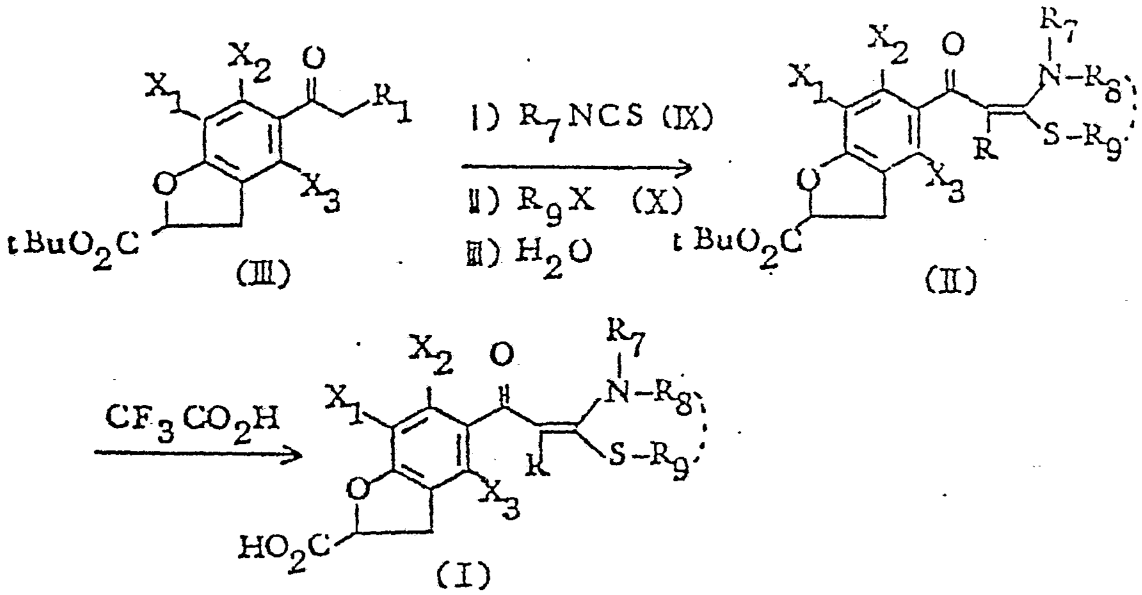


The compound (III) is allowed to react with the compound IX (1.2 equiv. mole) in a solution of 60 % oily sodium hydride (equiv. mole) in DMF or dimethylacetoamide (DMA) and tetrahydrofuran (THF) under nitrogen atmoshere at 5-15 °C, then, the compound (X) is added thereto, and the mixture is allowed to react at a temperature of 5 °C to room temperature. Ammonium chloride is added to the reaction mixture and the resulting mixture is extracted with ether. The ether residue is purified by column chromatography on silica gel to give the compound (II) which is allowed to react with 10 equivalent amount of trifluoroacetic acid at room temperature for 0.5-1.0 hour. The reaction mixture is evaporated under reduced pressure and the residue is crytallized from ether to give the aimed compound [ I ]. The compound [ I ] is recrystallized from an appropriate solvent as occasion demands for the porpose of further purification. The respective examples are shown in table 5 (Nos. 1-4).

Table 5 ( No. 1 )

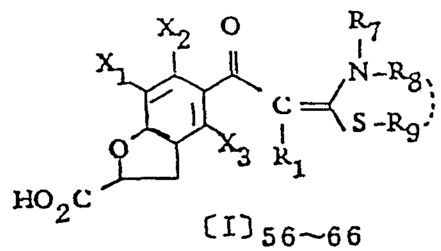

〔I〕56~66

| Example Nos. | $X_1 \sim X_3$ | $R_1$ | $R_7$ | $R_8$ | $R_9$ | Yield (from III) (%) |
|---|---|---|---|---|---|---|
| 56 | 6,7-di-Cl | H | $CH_3$ | H | $CH_3$ | 44.6 |
| 57 | 〃 | H | $CH_3$ | H | $C_2H_5$ | 39.5 |
| 58 | 〃 | H | 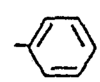 | H | $CH_3$ | 62.1 |
| 59 | 〃 | $CH_3$ | $C_2H_5$ | H | $CH_3$ | 67.5 |
| 60 | 〃 | $CH_3$ | $CH_3$ | H | $CH_3$ | 60.3 |
| 61 | 〃 | H | $CH_3$ | H | $-CH_2-CH=CH_2$ | 46.7 |
| 62[-1)] | 〃 | H | $CH_3$ | $-CH-(OCH_3)-CH_2$ | | 29.4 |
| 63[-2)] | 〃 | H | 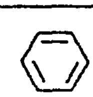 | $-CH_2-CH_2-$ | | 34.6 |
| 64[-3)] | | H | Me | $-\overset{O}{\overset{\|}{C}}-CH_2-$ | | 60.5 |
| ※ 65 | $X_1$=Me $X_3$=Cl | H | Me | H | Me | 21.7 |
| ※ 66 | $X_1$=Me $X_2$ $X_3$=H | H | Me | H | Me | 6.2 |

m ( ※ Starting materials were prepared according to the method of William F. Hoffman et al. J. Med. Chem. (1981) 24 865~873 )

-1) $R_9X=BrCH_2CH<^{OMe}_{Br}$ -2) $R_9X=BrCH_2CH_2Br$ -3) $R_9X=BrCH_2CO_2Et$

Table5 ( No. 2 )

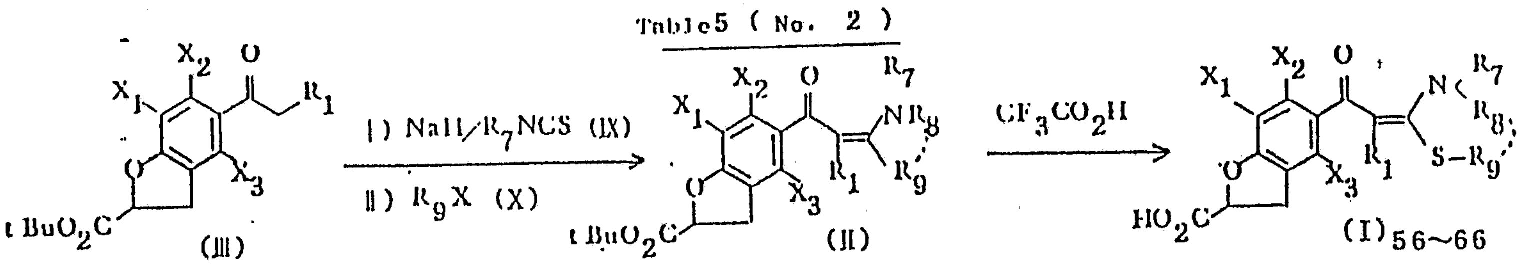


| Example Nos. | Amount Used g (mmol) Compd (III) | Solvent (ml) | Reaction Temp. | Reaction Time | Compds (X) $R_9X$ | Reaction Temp. | Reaction Time | Yield of (II) (%) | Yield of (I) (%) |
|---|---|---|---|---|---|---|---|---|---|
| 56 | 0.994 (3) | DMF (4) | 5~10° | 2 | Me J | 5~r.t. | 1 | 57.3 | 77.8 |
| 57 | 〃 | 〃 | 〃 | 1 | Et J | 〃 | 1 | 46.0 | 85.9 |
| 58 | 1.65 (5) | 〃 (6) | 〃 | 2 | Me J | 〃 | 1 | 70.8 | 87.7 |
| 59 | 1.04 (3) | 〃 (4) | 〃 | 2 | Me J | 〃 | 1 | 77.6 | 87.0 |
| 60 | 1.04 (3) | DMF－THF (1:3) | 〃 | 2 | Me J | 〃 | 1 | 62.2 | 96.9 |
| 61 | 0.994 (3) | DMF－THF (〃) | 〃 | 2 | $CH_2$=CH-$CH_2$Br | r.t. | 2 | 51.0 | 91.5 |
| 62 | 1.32 (4) | DMF－THF (〃) | 5~8° | 3 | $CH_3O$ Br >-$CH_2$Br -a) | -50~-10° | 2 | 40.7 | 72.3 |
| 63 | 0.994 (3) | DMF－THF (〃) | 5~10° | 2 | Br$CH_2CH_2$Br -a) | 5~r.t. | 18 | 61.0 | 56.7 |
| 64 | 0.994 (3) | DMF－THF (〃) | 〃 | 2 | Br$CH_2CO_2$Me | 5~15° | 1.5 | 62.0 | 97.5 |
| 65 | 0.90 (3) | DMF (4) | 〃 | 2 | Me J | 〃 | 1 | 22.7 | 95.6 |
| 66 | 1.38 (5) | DMF (6) | 5~25° | 1 | Me J | 〃 | 1 | 7.7 | 80.1 |

-a) Equivalent mole of NaH is added

Table 5 (No. 3)

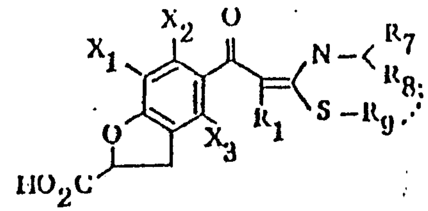

(I)

| Example Nos. | Recrystallized from | m.p. (℃) | Molecular formula | Elementary Analysis Calcd. C | H | Cl | N | S | Found C | H | Cl | N | S |
|---|---|---|---|---|---|---|---|---|---|---|---|---|---|
| 56 | DMF - ethanol | 263~265(d) | $C_{14}H_{13}Cl_2NO_4S$ | 46.40 | 3.62 | 19.57 | 3.87 | 8.85 | 46.44 | 3.70 | 19.32 | 3.96 | 8.66 |
| 57 | 〃 | 249~251(d) | $C_{15}H_{15}Cl_2NO_4S$ | 47.88 | 4.02 | 18.84 | 3.72 | 8.52 | 47.79 | 4.04 | 18.57 | 3.82 | 8.30 |
| 58 | acetone | 223~225(d) | $C_{19}H_{15}Cl_2NO_4S$ | 53.78 | 3.63 | 16.71 | 3.30 | 7.55 | 53.93 | 3.83 | 16.49 | 3.15 | 7.70 |
| 59 | acetone | 194~196(d) | $C_{16}H_{17}Cl_2NO_4S$ | 49.24 | 4.39 | 18.17 | 3.59 | 8.21 | 49.12 | 4.45 | 18.28 | 3.59 | 8.48 |
| 60 | DMF - water | 206~207(d) | $C_{15}H_{15}Cl_2NO_4S$ | 47.88 | 4.02 | 18.85 | 3.72 | 8.52 | 47.66 | 4.00 | 18.56 | 3.88 | 8.44 |
| 61 | ethanol | 178~180(d) | $C_{16}H_{15}Cl_2NO_4S$ | 49.50 | 3.89 | 18.26 | 3.61 | 8.26 | 49.44 | 3.86 | 18.33 | 3.64 | 8.10 |
| 62 | ethanol | 258~260(d) | $C_{16}H_{15}Cl_2NO_5S$ | 47.53 | 3.74 | 17.54 | 3.47 | 7.93 | 47.40 | 3.90 | 17.84 | 3.59 | 7.80 |
| 63 | ethanol | 225~228(d) | $C_{20}H_{15}Cl_2NO_4S \cdot 1-C_2H_5OH$ | 54.78 | 4.39 | 14.70 | 2.90 | 6.65 | 54.42 | 4.35 | 15.05 | 3.03 | 6.60 |
| 64 | ethanol | 253~256(d) | $C_{15}H_{11}Cl_2NO_5NS$ | 46.41 | 2.86 | 18.26 | 3.61 | 8.26 | 41.31 | 2.96 | 18.12 | 3.54 | 8.07 |
| 65 | ethanol | 248~250(d) | $C_{15}H_{16}Cl\ NO_4S$ | 52.71 | 4.72 | 10.37 | 4.10 | 9.38 | 52.66 | 4.89 | 10.04 | 4.03 | 9.17 |
| 66 | ethanol | 219~220(d) | $C_{15}H_{17}NO_4S$ | 58.62 | 5.57 | | 4.56 | 10.43 | 58.30 | 5.67 | | 4.61 | 10.21 |

Table 5 ( No. 4 )

| Example Nos. | IR ( $\nu_{max}^{Nujol}$ $cm^{-1}$ ) | NMR ( $\delta_{ppm}^{DMSO}$ ) |
|---|---|---|
| 56 | 3200~2300, 2100~1800, 1730, 1569 | 11.15(1H, br) 7.30(1H, s) 5.42(1H, d-d) 5.22(1H, s) 3.85~3.20(2H, m) 3.0(3H, d) 2.43(3H, s) |
| 57 | 3200~1800(br), 1735, 1610, 1570 | 11.2(1H, br) 7.28(1H, s) 5.42(1H, d-d) 5.25(1H, s) 3.85~3.15(2H, m) 3.1~2.9(5H, m) 1.29(3H, t) |
| 58 | 3200~1800(br), 1738, 1608, 1592, 1530 | 13.03(1H, br) 7.38(6H, m) 5.53(1H, s) 5.42(1H, d-d) 3.85~3.10(2H, m) 2.40(3H, s) |
| 59 | 3200~1800(br), 1741, 1608, 1570 | (※) t-Bu ester 12.03(1H, br) 6.93(1H, s-like) 5.18(1H, d-d) 3.8~3.2(4H, m) 2.39(3H, s) 1.87(3H, s) 1.48(9H, s) |
| 60 | 3200~1800(br), 1725, 1610, 1565 | 12~11(1H, br) 7.08(1H, s) 5.45(1H, d-d) 3.85~3.16(1H, m+s) 2.42(3H, s) 1.80(3H, s) |
| 61 | 3200~1800(br), 1728, 1565 | 11.20(1H, br) 7.26(1H, s) 6.1~5.65(1H, m) 5.5~5.1(4H, m) 3.83~3.2(4H, s) 3.0(3H, d) |
| 62 | 3200~1800(br), 1730(br), 1608, 1523 | 7.27(1H, s-like) 5.56~5.2(3H, s+m) 3.82~3.0(10H, m) |
| 63 | 3140(br)~1800(br), 1728, 1500, 1490 | 7.45~7.19(6H, m) 5.09(1H, s) 5.42(1H, d-d) 4.10(2H, t) 3.8~3.2(8H, m) 1.06(3H, t) |
| 64 | ~2580, 1763, 1732, 1600, 1570 | 7.40(1H, s) 6.46(1H, s) 5.49(1H, d-d) 3.90~3.17(7H, m) |
| 65 | 3140(br)~1800(br), 1712, 1567, 1500 | 7.28(1H, s) 5.38(1H, d-d) 5.22(1H, s) 3.80~3.15(2H, m) 3.0(3H, d) 11.35(1H, br) 2.43(3H, s) 2.23(3H, s) |
| 66 | 3200~2200, ~1950(br), 1732, 1570 | 11.50(1H, br) 7.10(2H, br) 5.66(1H, s) 5.27(1H, d-d) 3.80~3.10(2H, m) 2.98(3H, d) 2.50(3H, s) 2.20(3H, s) |

Example 67

Preparation of 6,7-dichloro-5-[[N-methyl(thiocarbamoyl)]-acetyl]-2,3-dihydrobenzofuran-2-carbaxylic acid [6]

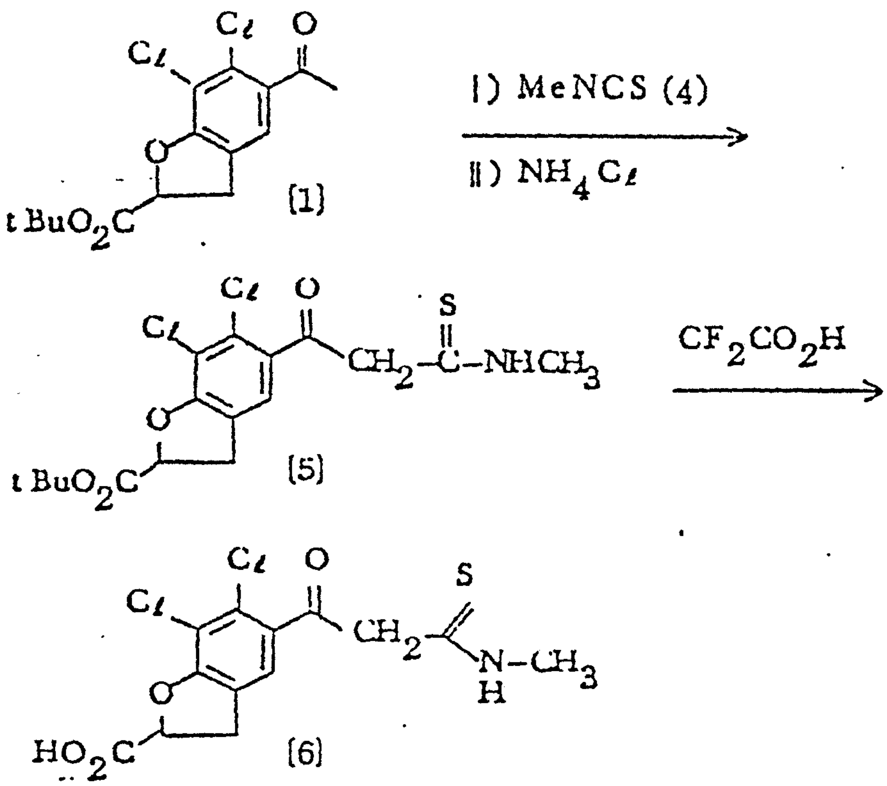


[1]

[5]

[6]

The compound [1] (0.993 g, 3 mmol) is allowed to react with methyl isothiocyanate [4] in the same manner as in Example 56. To the resulting solution of the sodium salt [5] is added a saturated ammonium chloride solution and the mixture is extracted with ether. The organic layer is washed with a saturated solution of sodium chloride, dried over anhydrous magnesium salfate, and evaporated. The residue is chromatographed on silica gel to give 0.65 g of the compound [5] as a resinous product. (By IR and NMR spectra, the compound [5] is comfirmed as a mixture of keto-form and enol-form)

IR: $\nu$ max ($CHCl_3$) 3420(NH), 3330(br: hydrogen bond -OH), 1750(COO), 1683(CO-N-), 1620, 1610 $cm^{-1}$.

NMR δ ppm ($CDCl_3$): (Keto+enol mixture) 14.45(0.5H,brs), 8.9(0.5H,br), 5.60(0.5 H, s), 4.39(1H,s), 3.8-3.1(5H,m), 1.49(9H,s).

A mixture of 0.65 g (1.61 mmol) of the compound [5] with 6.5 ml trifluoroacetic acid is stirred for 0.5 hour at room temperature. The reaction mixture is treated in the same manner as in Example 56 and the product is recrystallized from benzene to give 0.34 g of the titled compound [6], yield 60.7 %, mp.121-124 °C.

Anal. Calcd. (%) for $C_{13}H_{11}Cl_2NO_4S$
: C 44.84 H 3.18, Cl 20.36, N 4.02, S 9.21,
Found (%): C 44.92, H 3.29, Cl 20.14, N 4.10, S 8.96.
IR: ν max(Nujol) 3240, 3400-2400(br), 1725, 1615, 1535$cm^{-1}$;
NMR δ ppm (DMSO d-6) [a mixture of the keto-form and enol(thiol)-form (1/2)] : 14.3(2/3H,br), 10.2-9.83(1H,br), 7.4-7.66(1H), 5.82(2/3H,s), 4.30(2/3H,s), 4.0-3.20(2H,m), 3.0(3H,d).

Example 68

Preparation of 6,7-dichloro-5-(3-methylamino-3-propargylthio-2-propenoyl)-2,3-dihydrobenzofuran-2-carboxylic acid [8]

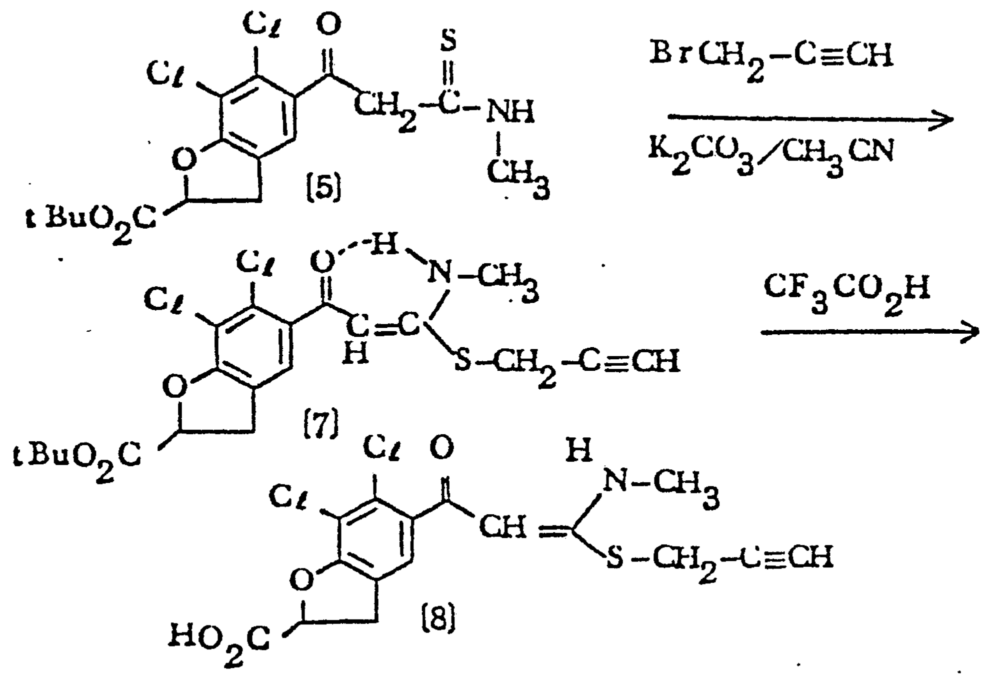


[5] [7] [8]

A mixture of 0.3 g (0.74 mmol) of the compound [5] prepared in Example 67, 0.097 g (0.82 mmol) of propargyl bromide, 150 mg of dry potassium carbonate powder and 6 ml of dry acetonitrile is stirred at room temperature for 2 hours. The reaction mixture is concentrated under reduced perssure, and the residue is extracted with ether. The organic layer is washed with water, dried over anhydrous magnesium sulfate and evaporated. The residue is purified by column chromatography on silica gel to give 0.30 g of the titled compound tert-butyl ester [7] as a resinous product, yield 91.6 %.

IR: $\nu$ max($CHCl_3$) 3320(acetylenic hydrogen), 1748, 1573 $cm^{-1}$.

NMR $\delta$ ppm ($CDCl_3$): 11.45(1H,br), 7.20(1H), 5.44(1H,s), 3.64(2H,d), 3.8-3.2(2H,m), 3.06(3H,d), 2.32(1H,m), 1.48(9H, s).

Subsequently, 0.61 g (1.38 mmol) of the compound [7] is allowed to react in the same manner as in Example 55 Step-2, and worked up and is recrystallized from ethanol to give 0.37 g of the titled compound, yield 69.5 %, mp. 180-183 °C (dec.).

Anal. Calcd. (%) for $C_{18}H_{13}Cl_2O_4NS$

: C 49.75  H 3.39, Cl 18.36, N 3.63, S 8.30,

Found (%): C 49.50, H 3.58, Cl 18.51, N 3.57. S 8.15.

IR: ν max (Nujol) 3260(acetylenic hydrogen), -2500-(br)-1950(br), 1725, 1572 $cm^{-1}$.

NMR δ ppm (DMSO d-6): 11.16(1H,br), 7.30(1H,sbr), 5.40-5.42(2H,s,d-d), 3.90(2H,d), 3.70-3.20(3H,m), 2.99(3H,d).

## Example 69

Preparation of 6,7-dichloro-5-[(3,4-dimethyl-4-thiazolin-2-yliden)acetyl]-2,3-dihydro-benzofuran-2-carboxylic acid [10]

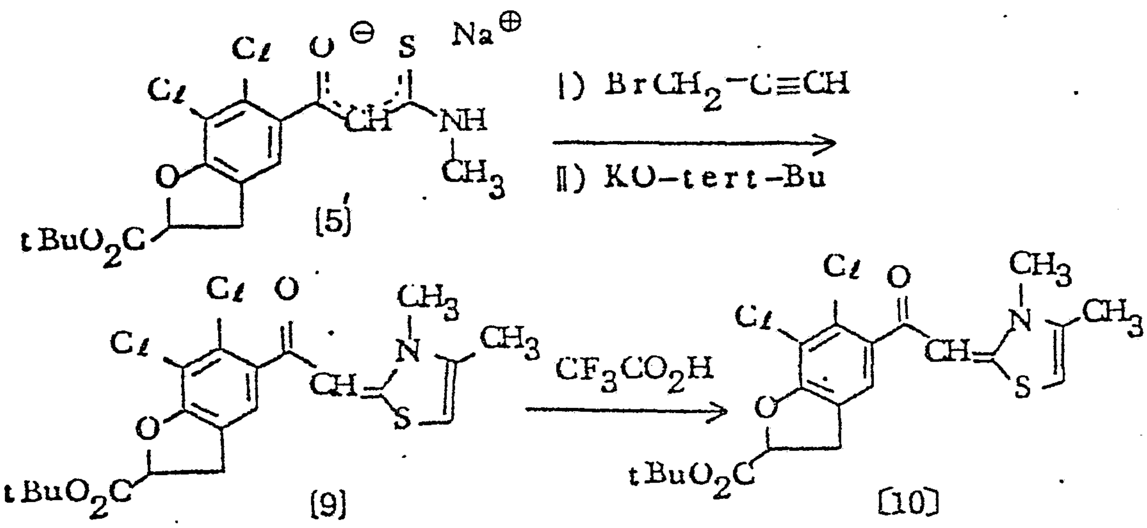


In the same manner as in Example 67, a mixture of 0.993 g (3 mmol) of the compound [1], 0.12 g (3 mmol) of 60 % oily sodium hydride and 0.263 g (3.6 mmol) of methyl isothiocyanate in DMA-THF (1/3) is allowed to react at 5-10 °C for 2 hours. To the resulting sodium salt [5] is added 0.29 ml (3.6 mmol) of propargyl bromide. The mixture is allowed to react at 10 °C to room temperature for 3

hours, then, 0.07 g (0.6 mmol) of tert-butoxide is added thereto, and the resulting mixture is allowed to react at room temperature overnight. Saturated ammoniun chloride solution is added and the mixture is extracted with ether. The ether extract is purified by column chromatography on silica gel and crystallized from a small amount of ether to give 0.50 g of the compound [9], yield 37.7 %, mp. 152-153 °C.

IR: ν max ($CHCl_3$) 1750, 1604, 1563, 1482 $cm^{-1}$.

NMR δ ppm ($CDCl_3$): 7.33(1H,s-like), 6.18(1H,s-like), 6.04(1H,s), 5.20(1H,d-d), 3.8-3.20+3.46(s)(6H), 2.26(3H,s), 1.49(3H,s).

In the same manner as in Example 55 (Step-2), 0.8 g of the compound [9] is allowed to react and worked up, and the product is recrystallized from DMF-ethanol to give 0.6 g of the titled compound, yield 85.9 %, mp. 277-279 °C (dec.).

Anal. Calcd. (%) for $C_{16}H_{13}Cl_2O_4NS$

: C 49.75, H 3.39, Cl 18.36, N 3.63, S 8.30,

Found (%): C 49.51, H 3.61, Cl 18.09, N 3.78. S 8.04.

IR: ν max(Nujol) 3120, -2480-(br), 1740(br), 1663, 1513$cm^{-1}$.

NMR δ ppm (DMSO, d-6): 6.00(1H,s), 5.44(1H,d-d), 3.85-3.3(5H, m+s), 2.26(3H,s).

Example 70

Preparation of 6,7-dichloro-5-[(4-thiazolin-2-ylidene)acetyl]-2,3-dihydro-benzofuran-2-carboxylic acid [25]

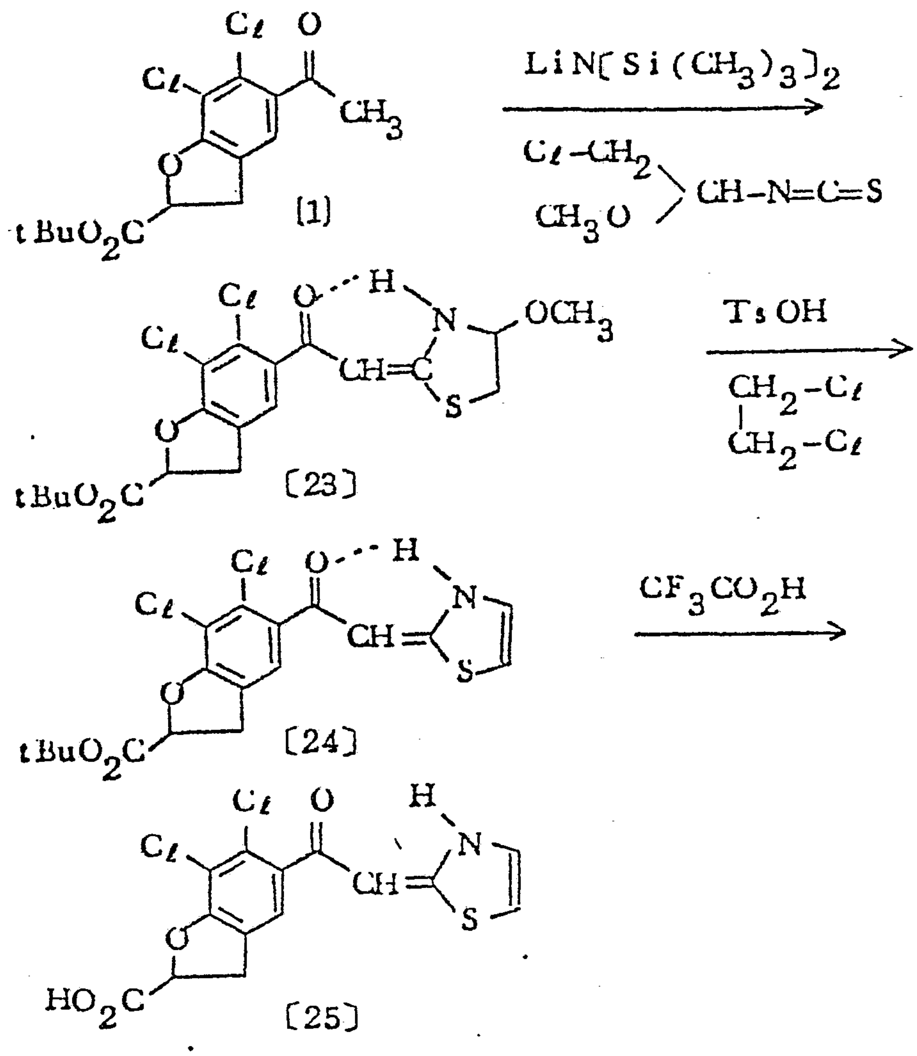


[1] [23] [24] [25]

A solution of 1.99 g (6 mmol) of the compound [1] in 6 ml of dry tetrahydrofuran (hereinafter abbreviated to as THF) is added to a solution of hexamethyl disilazane lithiumamine prepared from 1.3 ml (6.3 mmol) of hexamethyl disilazane and 4.2 ml (6.3 mmol) of a hexane solution of n-butyl lithium (1.5 N) at -78 °C. The mixture is allowed to react at -70 to -78 °C for 0.5 hour and a solution of 1.0 g (6.6 mmol) of 2-chloro-1-methoxy ethyl

isothiocyanate [22]* in 2 ml of THF is added thereto. The temperature of the reaction mixture is raised slowly and kept at 5-7 °C for 3 hours, further at 10-15 °C for 2 hours, and then, an ammonium chloride solution is added thereto. The resulting mixture is extracted with ether and the organic layer is a washed with a saturated sodium chloride solution, dried over anhydrous magnesium sulfate and evaporated at below 0 °C under reduced pressure. The residue is chromatographed on silica gel to give a mixture of the compound [23] and the strating material. The mixture is dissolved in 10 parts by volume of dichloromethane and 50 mg of anhydrous p-toluenesulfonic acid (hereinafter abbreviated to as p-TsOH) is added thereto. The mixture is heated under refluxing for 10 minutes, then, after cooling, washed with sodium dicarbonate, dried over anhydrous magnesium sulfate and evaporated under reduced pressure. The residue is purified by chromatography on silica gel to give 0.85 g (42 %) of the starting material and 0.78 g of the objected compound [24], yield 31.3 %.

IR: $\nu$ max ($CHCl_3$) 1750-1700(br), 1630, 1607 $cm^{-1}$

NMR $\delta$ ppm ($CDCl_3$): 7.72(d), 7.66(d), 7.4-7.23(m), 7.07(d), (total 3H) 6.10(s), 4.68(s) 5.2(1H,m) 3.8-3.2(2H,m) 1.50(9H, s) [NMR spectra indicated that the product was a mixture of thiazolin-form and thiazol-form in a solution.]

In the same manner as in Example 55 Sept-2, 0.75 g of the compound [24] is allowed to react and treated to give 0.60 g of the titled compound. Crystallized from acetone, and subsequent recrystallization from ethanol gave 0.3 g of the crystals, yield 46.3 %, (yield from [1] 14.5 %), mp. 214-216 °C.

Anal. Calcd. (%) for $C_{14}H_{9}Cl_2NO_4S$

: C 46.94 H 2.53, Cl 19.80, N 3.91, S 8.95.

Found (%): C 46.85, H 2.70, Cl 19.95, N 3.96, S 8.87.

IR: ν max (Nujol) 2800-2000, 2000-1800, 1715, 1683 $cm^{-1}$.

NMR δ ppm (DMSO d-6): 7.8-7.1(3H,m) 6.13(s,0.75H) 5.45(1H, m), 4.75(0.5H,br), 3.85-3.2(2H,m). [a mixture of 75 % thiazolin-form and 25 % thiazol-form]

* The compound [22] can be synthesized as follows.

A mixture of 4 g (32 mmol) of chlorodimethylacetal and 4.4 g (16.8 mmol) of silicon tetraisothiocyanate [$Si(NCS)_4$] is allowed to react at 80-85 °C for 6 hours. The reaction mixture is poured into ice and extracted with ether. The ether layer is washed with a sodium dicabonate solution, dried over anhydrous magnesium sulfate, and evaporated under reduced pressure. The residue is distilled to give 4.34 g of the compound [22], yield 90.5 %, bp. 92-93 °C (28 mmHg)

IR: ν max ($CCl_4$) 2000(br) $cm^{-1}$ (N=C=S) $cm^{-1}$.

## Example 71

Preparation of 6,7-dichloro-5-[2-(4-thiazolin-2-ylidene)-2-(carbethoxy)acetyl]-2,3-dihydro-benzofuran-2-carboxylic acid [30]

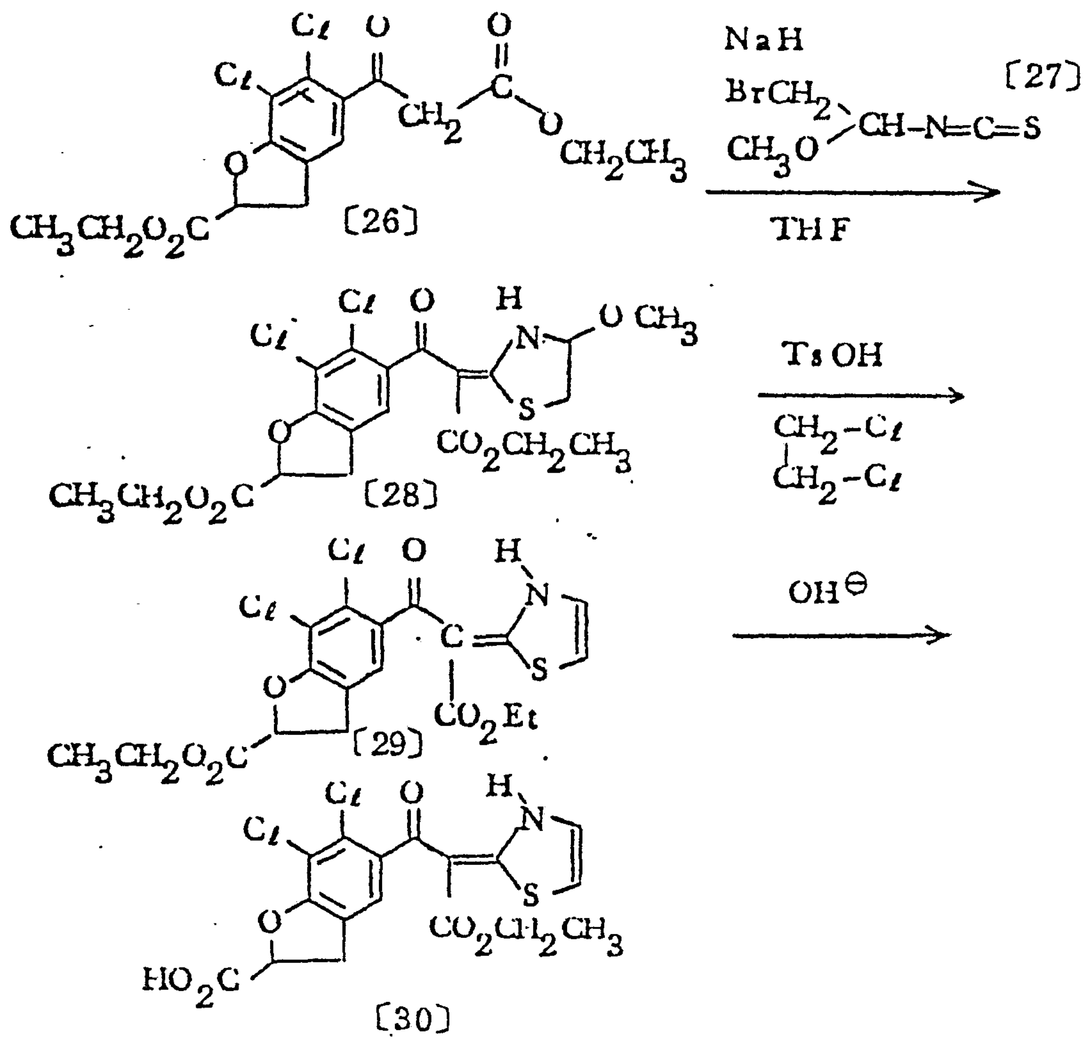


[26]

[28]

[29]

[30]

A solution of 1.0 g (2.67 mmol) of the compound [26] in 3 ml of a mixture of dimethylacetamide (hereinafter abbreviated to as DMA) and THF (1/3) is added to a solution of 0.107 g (2.67 mmol) of 60 % oily sodium hydride in 1 ml of DMA-THF (1/3) at 5-7 °C in nitrogen atmosphere. The mixture is stirred for 15 minutes, and 0.575 g (2.95 mmol) of the compound[27] is added thereto at -30 °C. The mixture is kept at 0-10 °C for 3 hours, then at 5 °C overnight, and then at 20-25 °C for 3 hours. The reaction mixture is worked up in the same manner as in Example 70 to give 0.86 g of

the compound [28], yield 65.6 %. Further, this is treated with p-TsOH in the same manner as in Example 70 to give 0.773 g of the compound [29], yield 96.3 % (yield from the compound [26], 63 %)

IR: ν max ($CHCl_3$) 3280(bs), 1755, 1738, 1645(br), 1610, 1561, 1540 $cm^{-1}$.

NMR δ ppm ($CDCl_3$): [15.5(br)+13.3(br)](1H), 7.4(1H,br), 7.0(2H,br+s), 5.30(1H,d-d), 4.4-3.2(6H,m), 1.30(3H,t), 0.86(3H, t).

The compound [29] is hydrolyzed with NaOH to give 0.4 g of the titled compound [30], yield 58.5 %. This is recrystallized from acetone to give crystalls having mp. 212-215 °C.

Anal. Calcd. (%) for $C_{17}H_{13}Cl_2NO_6S$

: C 47.46 H 3.05, Cl 16.48, N 3.25, S 7.45,

Found (%): C 47.47, H 3.33, Cl 16.34, N 3.22, S 7.18.

IR: ν max (Nujol) 3150, 3120, 1727, 1644 $cm^{-1}$.

NMR δ ppm (DMSO d-6): 13.7(1H,br), 7.16(1H,d), 7.28(1H,d), 7.04(1H,s-like), 5.40(1H,d-d), 3.9-3.15(5H,m), 0.73(3H,t).

○ The strating compound [26] is synthesized as follows.

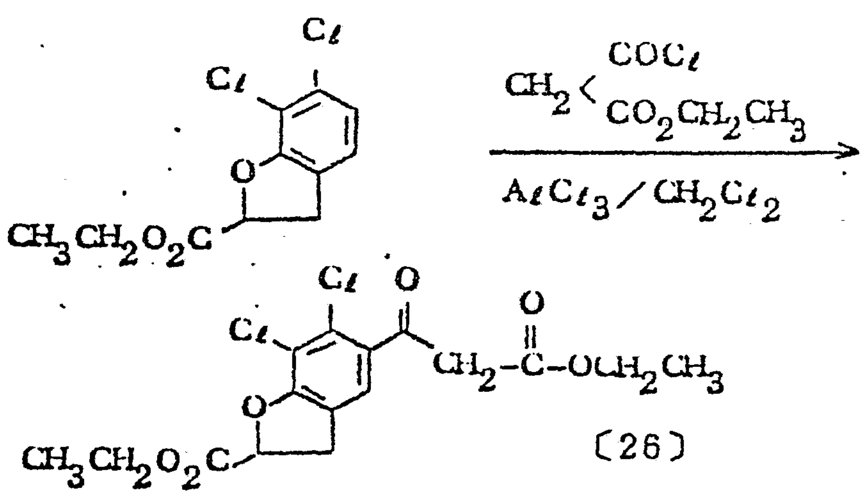


[26]

A mixture of 2.6 g (10 mmol) of ethyl 6,7-dichloro-2,3-dihydro-benzofuran-2-carboxylate, 1.96 g (13 mmol) of ethylmalonyl chloride, 4.8 g (36 mmol) of anhydrous aluminium chloride and 30 ml of dry dichloromethane is allowed to react at room temperature overnight and then poured into water. The resulting mixture is extracted with dichloromethane, and the organic layer is washed with water, dried over anhydrous magnesium sulfate and evaporated under reduced pressure. The residue is chromatographed on silica gel to give 0.75 g of the objected compound [26], yield 20 % and 0.81 g of the starting material(31 %).

IR: $\nu$ max ($CCl_4$) 1765, 1743, 1650-1628(br), 1610 $cm^{-1}$.

NMR $\delta$ ppm ($CDCl_3$): 7.40(1H,s-like), 5.45(0.5H,s), 5.3(1H,m), 4.9-3.25(7H,m), 1.4-1.15(12H,s,t), [a mixture of keto-enol forms(1/1)].

Further the reagent [27] can be synthesized from bromodimethylacetal in the same manner as the compound [22] mentioned in Example 70, yield 85 %, bp.86-86 °C (10 mmHg).

IR: $\nu$ max ($CCH_4$) 2000 $cm^{-1}$ (br) (N=C=S) $cm^{-1}$.

Example 72

Production of 6,7-dichloro-5-[(4-oxo-perhydro-1,3-thiazin-2-ylidene)acetyl]-2,3-dihydro-benzofuran-2-carboxylic acid [14]

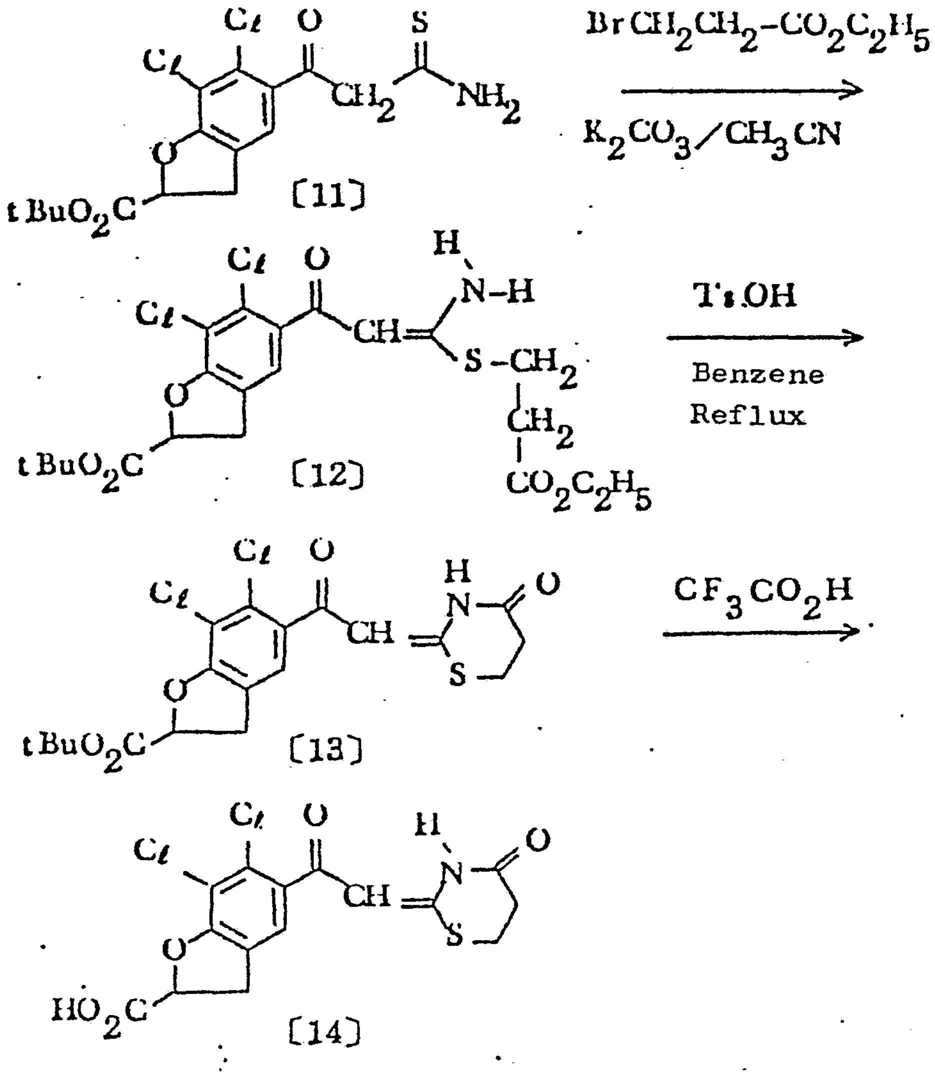


[11]

[12]

[13]

[14]

STEP 1.

A mixture of 0.39 g (1mmol) of t-butyl 6,7-dichloro-5-[(thiocarbamoyl)acetyl]-2,3-dihydrobenzofuran-2-carboxylate, 0.21 g (1.2 mmol) of ethyl bromopropionate, 0.21 g (1.5 mmol) of powdery anhydrous potassium carbonate, 0.017 g (0.1 mmol) of potassium iodide, and 3 ml of dry acetonitrile is allowed to react at room temperature for 5 hours and concentrated in vacuo. The crude residue is extracted with methylene chloride, and the material soluble in the methylene chloride is purified by silica gel chromatography to give the compound [12] as an oily product. For the purpose of cyclization, this is dissolved in 10 ml dry benzene containing 0.009 g (0.05 mmol) of toluenesulfonic acid (anhydrous) and refluxed azeotropically under heating for an hour in a vessel equipped with a water-separator in which 3A-Molecular sieves are placed. The reaction mixture is washed with an aqueous solution of sodium hydrogencarbonate, concentrated in vacuo, and purified by silica gel chromatography to give 0.32 g (yield 72%) of the compound [13].

IR $\nu$ max ($CHCl_3$): 1741, 1701, 1583, 1545 $cm^{-1}$.

NMR $\delta$ ppm ($CDCl_3$): 12.55 (1H, br), 7.23 (1H, s), 5.87 (1H, s), 5.22 (1H, d-d), 3.8-2.8 (6H, m), 1.48 (9H, s).

STEP 2

A mixture of 0.3 g of the compound [13] and 3 ml of trifluoroacetic acid is stirred at room temperature for 1 hour and then treated in the same manner as in STEP 2 of Example 55 to give 0.235 g (yield 92.5%) of the titled compound [14], which is recrystallized from ethanol to give crystals, m.p. 226-228°C.

Anal. Calcd.(%) for $C_{16}H_{11}Cl_2O_6NS$: C, 46.41; H, 2.86; Cl, 18.26; N, 3.61; S, 8.26. Found (%): C, 46.19; H, 3.02; Cl, 18.32; N, 3.52; S, 8.12.

IR $\nu$ max (Nujol): 3300-2300 (br), 1747, 1642, 1595, 1561 $cm^{-1}$.

NMR $\delta$ ppm (DMSO,d-6): 12.45 (br) + 11.02 (1H, keto+enol), 7.4 + 7.26 (1H, s), 0.63 + 0.6 (1H, s), 5.45 (1H, m), 3.8-2.8 (6H, m).

The starting material, t-butyl-6,7-dichloro-5-[(thiocarbamoyl)acetyl]-2,3-dihydro-benzofuran-2-carboxylate [11] can be produced in the following manner.

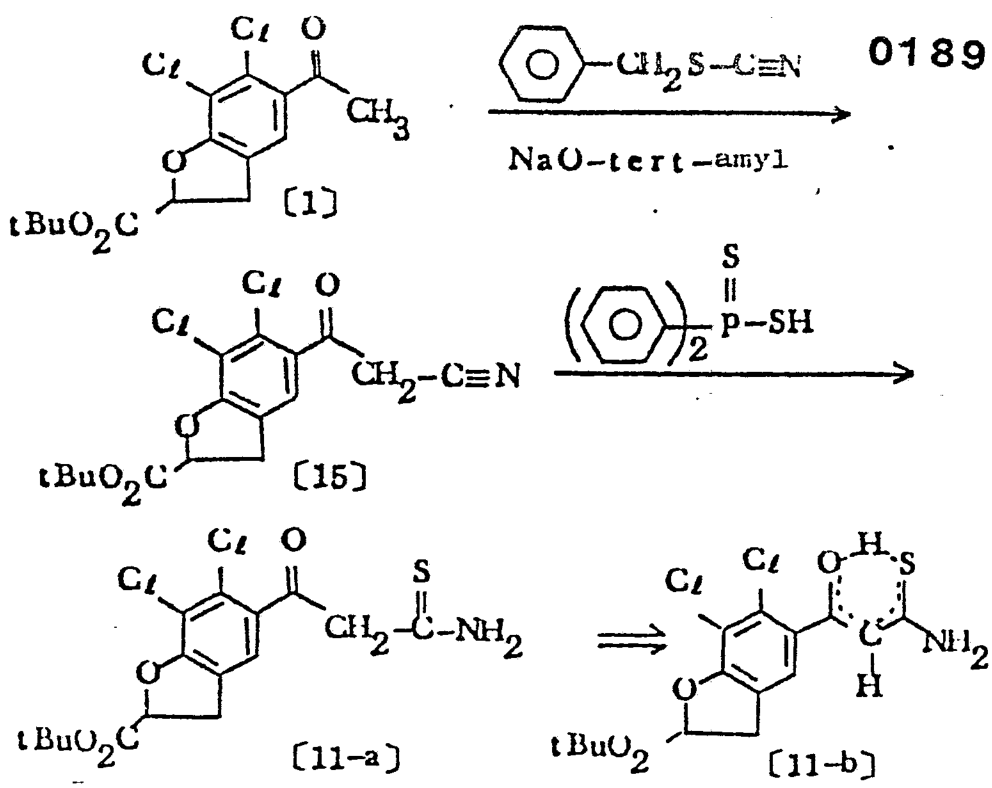


A solution of 3.03 g (10 mmol) of t-butyl 6,7-dichloro-5-acetyl-2,3-dihydro-benzofuran-2-carboxylate [1] in 5 ml of benzene is added under ice-cooling to a solution of sodium tert-amylate which is prepared by refluxing 0.48 g (12 mmol) of 60% oily sodium hydride, 1.06 g (12 mmol) of tert-amyl alcohol, and 25 ml of dry benzene, and the mixture is stirred for 0.5 hour. A solution of 2.24 g (15 mmol) of benzyl thiocyanate in 10 ml of benzene is added thereto under ice-cooling. The reaction mixture is allowed to react at room temperature overnight, to which an aqueous solution of ammonium chloride is then added. The benzene layer is separated, washed with water, dried over anhydrous magnesium sulfate, and concentrated in vacuo to give a residue, which is purified by silca gel chromatography to give 1.08 g (yield 30.3%) of the compound [15]. This is recrystallized from a small amount of isopropyl ether to give crystals, m.p. 73-74°C.

IR $\nu$ max ($CHCl_3$): 2260 (CN), 1750, 1700, 1605 $cm^{-1}$.

NMR $\delta$ ppm ($CDCl_3$): 7.46 (1H, s), 5.30 (1H, d-d), 4.17 (2H,

s), 3.85-3.24 (2H, m), 1.50 (9H, s).

A mixture of 1.21 g (3.39 mmol) of the compound [15] with 1.87 g (7.46 mmol) of diphenyldithiosulfonic acid (prepared from benzene and phosphorus pentasulfide in the same manner as in W. A. Higgins *et al.*, J. Am. Chem. Soc., 77, 1867 (1955)) and 50 ml of isopropanol is allowed to react at 40°C overnight. The precipitating crystals are removed by filtration under ice-cooling and the filtrate is concentrated in vacuo to give a residue, which is purified by silica gel chromatography to give 1.1 g (yield 83.1 %) of resinous objective compound [11] (the compound is in a mixture of keto- and enol-form in a solution.).

IR $\nu$ max ($CHCl_3$): 3560, 3500, 3385, 2540, 1748, 1603 $cm^{-1}$.

NMR $\delta$ ppm ($CDCl_3$): [enol-form: 14.54 (s), 6.70 (br), 5.76 (s)], [keto-form: 8.45, 7.8 (bs), 4.39 (s)], 7.27 (1H, m), 5.76 (1H, m), 3.8-3.2 (2H, m), 1.49 (9H, s).

Example 73-77

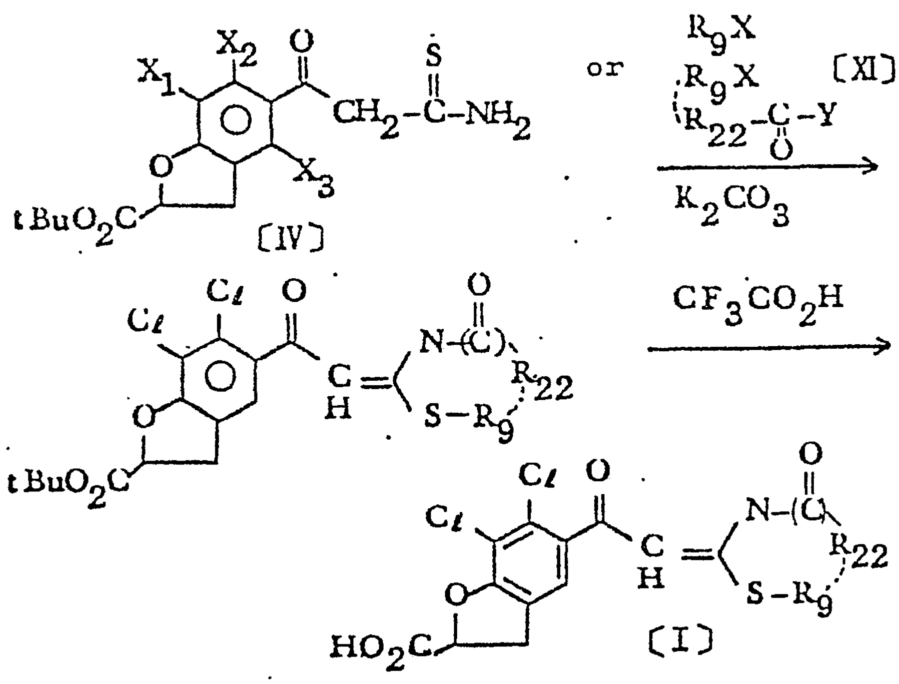


[IV] [XI] [I]

A solution of the compounds (Ⅳ) and (X I) dissolved in acetonitrile is allowed to react in the presence of anhydrous

potassium carbonate. After filtration, the filtrate is concentrated in vacuo to give a residue, which is purified by silica gel chromatography. In Example 77, the reaction is carried out in benzene under refluxing in the presence of a catalytic amount of p-toluenesulfonic acid, and the reaction product is washed with sodium hydrogencarbonate, concentrated in vacuo, and then worked up in the same manner as above. The obtained compound ( II ) is allowed to react with a ten-fold amount of trifluoroacetic acid at room temperature for an hour, the mixture is concentrated in vacuo to give a residue, which is recrystallized from ether to give the objective compound ( I ).

This may be purified by recrystallization, if necessary.

Examples are more specifically explained in Table 6 (Nos. 1-4).

Table 6 ( No. 1 )

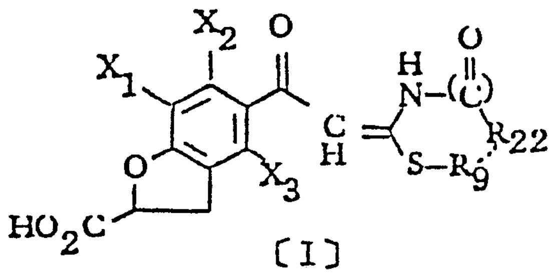

〔I〕

| Example Nos. | $X_1 \sim X_3$ | 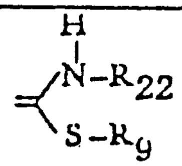 | Yield (%) from (IV) |
|---|---|---|---|
| 73 | 6,7 - di-chloro | 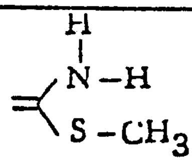 | 72.3 |
| 74 | 〃 | 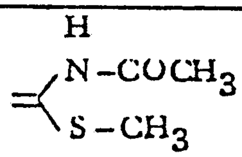 | 38.9 |
| 75 | 〃 | 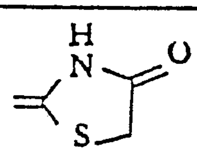 | 44.2 |
| 76 | 〃 | 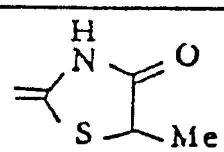 | 31.5 |
| 77 | 〃 | 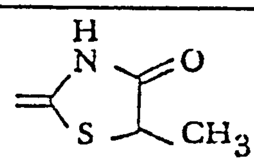 | 46.3 |

Table6 ( No. 2 )

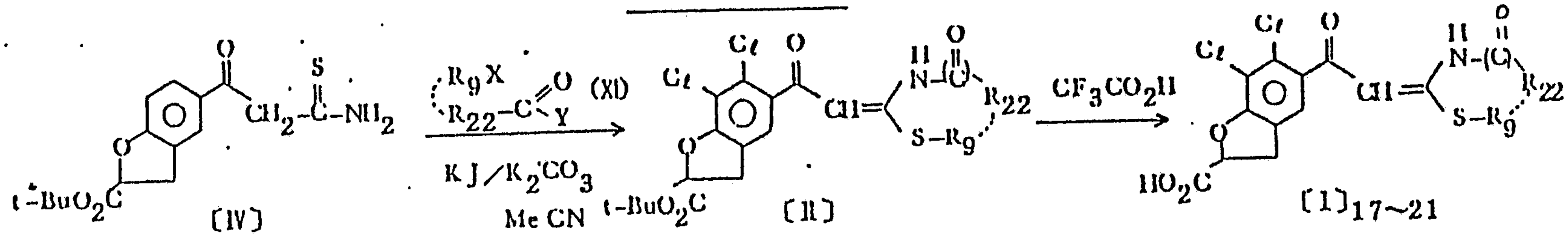


[IV] [II] $[I]_{17\sim21}$

| Example Nos. | Amount Used g (mmol) (IV) | (XI) | $R_9X$ / $R_{22}$–C(O)Y | $K_2CO_3$ | $CH_3CN$ | Reaction Temp. | Time (hr) | [II] Yield (%) | [I] Yield (%) |
|---|---|---|---|---|---|---|---|---|---|
| 73 | 0.16 (0.41) | $CH_3J$ | 0.07 (0.5) | 0.085 (0.62) | 3 ml | rt | 1.0 | 96.4 | 75.0 |
| 74 | | $CH_3J$ (2) (0.07)<br>$CH_3COCl$ | (1) (0.5)<br>0.32 (4.1) | 0.85 (6.2) | 3 ml | rt | 16 | 66.7 | 58.3 |
| 75 | 0.14 (1.025) | $BrCH_2COOMe$ | 0.188 (1.23) | 0.212 (1.54) | 3 ml | rt | 3 | 88.4 | 50.0 |
| 76 | 0.50 (1.28) | $Br-CH(CH_3)COOC_2H_5$ | 0.28 (1.54) | 0.265 (1.92) | 4 ml | rt | 2 | 92.0 | 34.2 |
| 77 | 0.60 (1.54) | $Br-CH_2-CH(CH_3)-CO_2C_2H_5$ -a) | 0.36 (1.85) | (1) 0.32 (0.23)<br>2) TsOH 0.0065 g/benzene 12 ml refluxed for 1.5 hours | 5 ml | rt | 16 | 84.2 | 55.0 |

-a) which is prepared according to the method disclosed in pickard, JACS 69 14 (1947)

Table 6 ( No. 3 )

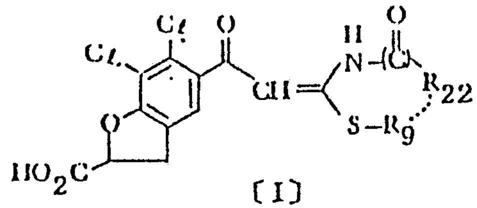

[I]

| Example Nos. | Recrystal from | m.p. (℃) | Molecular formula | Elemantary Analysis Calcd. (%) C | H | Cl | N | S | Found (%) C | H | Cl | N | S |
|---|---|---|---|---|---|---|---|---|---|---|---|---|---|
| 73 | ethanol | 254~257(d) | $C_{14}H_{11}Cl_2NO_4S$ | 44.84 | 3.18 | 20.36 | 4.02 | 9.21 | 44.67 | 3.26 | 20.19 | 3.97 | 9.09 |
| 74 | ethanol | 217~220(d) | $C_{15}H_{13}Cl_2NO_5S$ | 46.17 | 3.36 | 18.17 | 3.59 | 8.21 | 46.01 | 3.49 | 18.14 | 3.61 | 8.07 |
| 75 | ethanol | 242~258(d) | $C_{14}H_{9}Cl_2NO_5S$ | 44.94 | 2.42 | 18.95 | 3.74 | 8.57 | 44.83 | 2.58 | 19.18 | 3.66 | 8.47 |
| 76 | ethyl acetate | 202~206 | $C_{15}H_{11}Cl_2NO_5S$ | 46.41 | 2.86 | 18.26 | 3.61 | 8.26 | 46.18 | 2.98 | 18.11 | 3.66 | 8.14 |
| 77 | ethanol | 213~217 | $C_{16}H_{13}Cl_2NO_5S$ | 47.78 | 3.26 | 17.63 | 3.48 | 7.97 | 47.50 | 3.25 | 17.50 | 3.46 | 7.48 |

Table 6 ( No. 4 )

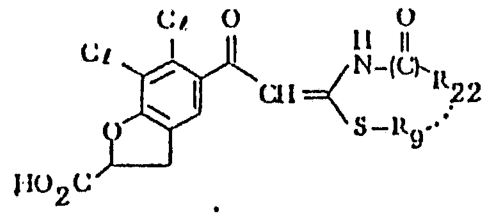

| Example Nos. | IR ( $\nu_{max}^{Nujol}$ $cm^{-1}$ ) | NMR ( $\delta_{ppm}^{DMSO_{d-6}}$ ) |
|---|---|---|
| 73 | 3410, 3240, 1900(br), 1730, 1596, 1494 | 1.0~7.5(2H, br) 7.26(1H, s) 5.42(1H, d-d) 5.23(1H, s) 3.8~3.2(2H, m) 2.40(3H, s) |
| 74 | 3000~2000(br), 1748, 1665, 1565(br) | 13.30(1H, s(br)) 7.46(1H, s) 5.76(1H, s) 5.45(1H, d-d) 3.8~3.25(2H, m) 2.35(3H, s) 2.20(3H, s) |
| 75 | 3120, 3080, 1750, 1690, 1623, 1601, 1510 | 11.85(1H, br) 7.32(1H, s) 6.33(1H, s) 5.45(1H, d-d) 3.80~3.20(4H, s+m) |
| 76 | 3280, 1730, 1630, 1620, 1570, 1525 | (※) d-6 acetone 10.5(1H, br) 7.39+7.33(1H, s) 6.50+6.04(1H, s) 5.48(1H, d-d) 4.3~3.25(3H, m) 1.55(3H, d) |
| 77 | 3300~2300(br), 1755, 1720, 1561, 1515 | 12.46+11.0(1H, br) 7.40+7.27(1H, s) 6.30+5.86(1H, s) 5.46(1H, m) 3.85~2.70(5H, m) 1.2(m, 3H) |

Example 78

6,7-Dichloro-5-[3-methylamino-3-(methylthio)-2-propenoyl]-2,3-dihydro-1-benzofuran-2-yl-methanol

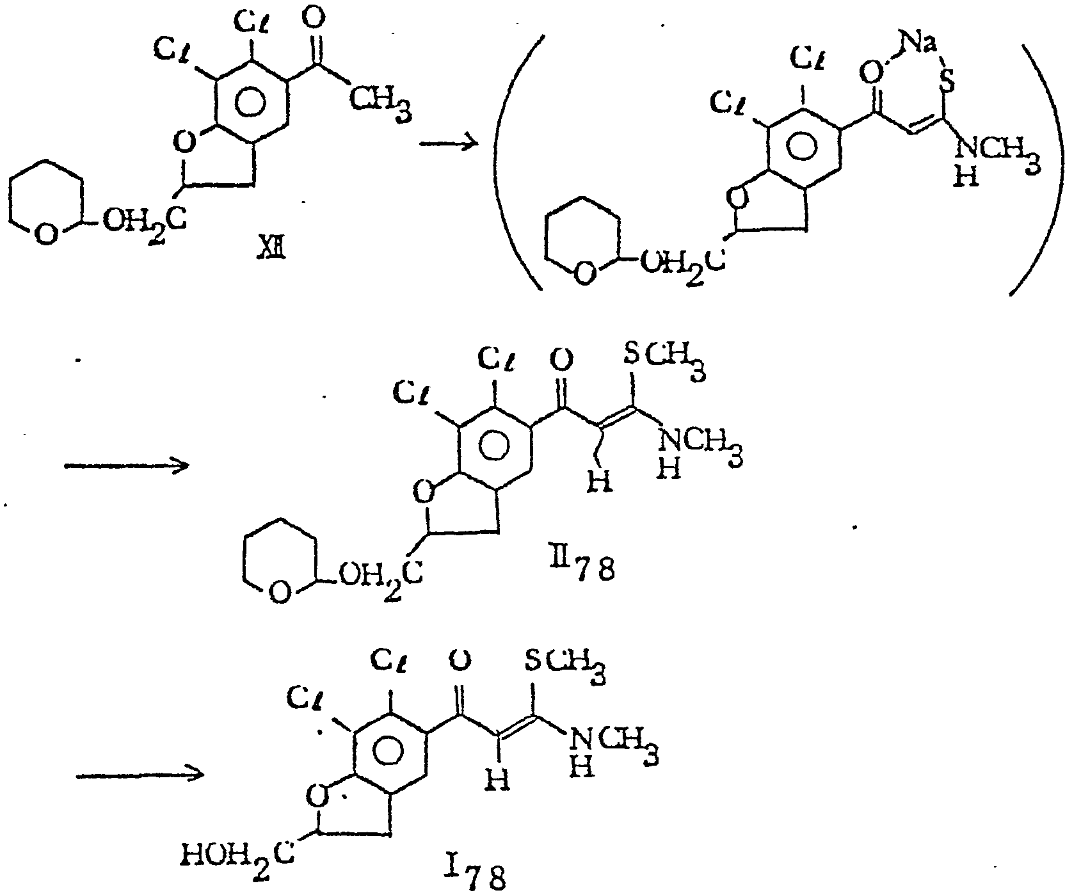

XII

$II_{78}$

$I_{78}$

A mixture of 1.0 g (2.9 mmol) of the compound XII (Example 44) and 0.112 g (3.0 mmol) of 65% sodium hydride is allowed to react for 2/3 hour in 5 ml of N,N-dimethylformamide while being stirred under nitrogen atmosphere, then combined with 0.233 g (3.2mmol) of methyl isothiocyanate, and reacted for 2 hours. To the mixture is added 0.5 g (3.5 mmol) of methyl iodide, and reacted for further 14 hours. The reaction product is treated with n-hexane to give crude crystals, which are recrystallized from ethyl acetate to give 0.42 g (yield 33.6 %) of pale yellow crystals, m.p. 176-177°C.

NMR $\delta$ ppm ($CDCl_3$): 1.63 (6H, br), 2.38 (3H, s), 2.95-4.13

(9H, m), 4.65 (1H, br), 4.83-5.40 (1H, m), 7.10 (1H), 11.38 (1H, br).

A mixture of 0.42 g (1.0 mmol) of the compound $II_{78}$ with 5 ml of trifluoroacetic acid is allowed to react at room temperature for 0.5 hour. The reaction product is chromatographed on a Lober column with an ethyl acetate/dichloromethane mixture(95:5) as eluent to give 0.24 g (yield 70.8%) of the compound $I_{78}$, m.p. 170-173°C. This is recrystallized from ethyl acetate to give 0.185 g (yield 54.6%) of pale yellowish crystals, m.p. 170-173°C.

Anal. Calcd.(%) for $C_{14}H_{15}Cl_2NO_3S_2$: C, 48.28; H, 4.34; Cl, 20.36; N, 4.02; S, 9.21. Found (%): C, 48.09; H, 4.34; Cl, 20.09; N, 4.16; S, 8.92.

IR $\nu$ max (Nujol): 3350, 3140, 1602, 1565 $cm^{-1}$.

NMR $\delta$ ppm (DMSO,d-6): 2.42 (s), 2.73-3.43 (5H, m), 3.50-3.82 (2H, m), 4.73-5.17 (2H, m), 5.22 (1H, s), 7.25 (1H), 11.2 (1H, br).

Example 79

6,7-Dichloro-5-[3-mercapt-3-(methylamino)-2-propenoyl]-2,3-dihydro-1-benzofuran-2-yl-methanol

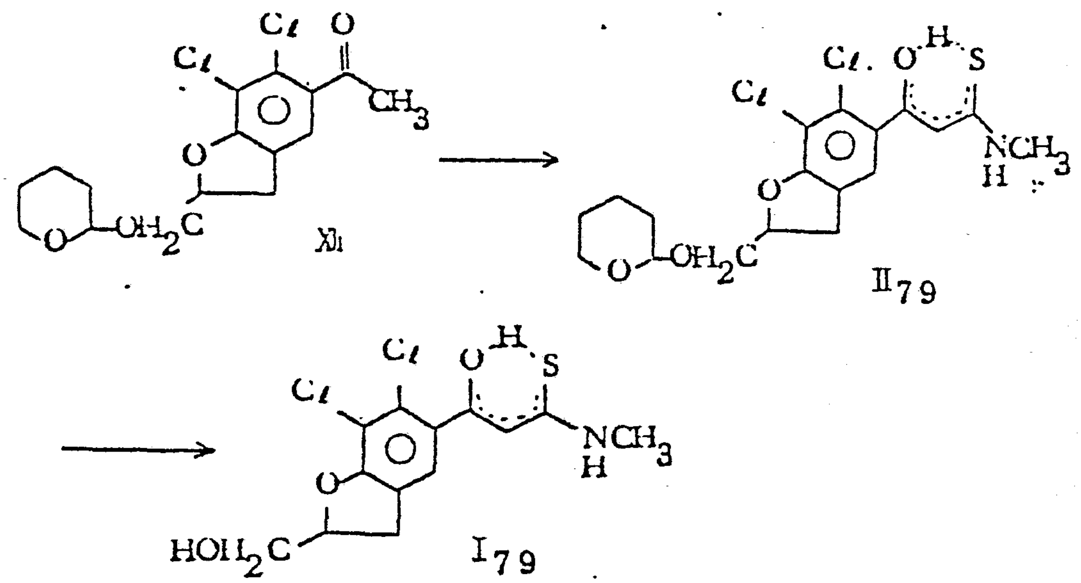

XI

$II_{79}$

$I_{79}$

The same procedure as in Example 78 is applied to this example, using 0.5 g (1.4 mmol) of the compound X II (Example 44), 0.056 g (1.5 mmol) of 65% sodium hydride, 0.116 g (1.6 mmol) of methyl isothiocyanate, and 4 ml of N,N-dimethylformamide. Then the reaction product is chromatographed on a Lober column (type B) with an ethyl acetate/dichloromethane mixture (3:97) as an eluent to give 0.26 g (yield 42.9%) of the compound $II_{79}$.

NMR δ ppm ($CDCl_3$): 1.58 (6H, br), 3.07-4.15 (9H, m), 4.60 (1H, br), 4.83-5.33 (1H, m), 4.35 (1H, s), 7.15 (1H).

The product which is prepared by treating 0.25 g (0.6 mmol) of the compound $II_{79}$ in the same manner as in Example 79 is crystallized from ether to give the compound $I_{79}$, m.p. 121-125°C. This is recrystallized from ether to give 0.022 g (yield 2.9%) of pale yellowish crystals, m.p. 126-127°C.

Anal. Calcd.(%) for $C_{13}H_{13}Cl_2NO_3S$ $1/3(C_2H_5)_2O$: C, 47.96; H, 4.59; Cl, 19.75; N, 3.90; S, 8.93. Found (%): C, 48.17; H, 4.37; Cl, 19.66; N, 3.85; S, 8.64.

IR ν max (Nujol): 3590, 3245, 3360, 1616, 1606 $cm^{-1}$.

NMR δ ppm ($Me_2CO$,d-6): 1.13 (t), 2.08 (1H, br), 2.67-3.50 (5H, m), 3.63-4.20 (2H, m), 4.70-5.30 (1H, m), 5.77 (1H, s), 7.22 (1H).

Example 80

Production of 6,7-dichloro-5-[3-(methylthio)-3-propargylamino-2-propenoyl]-2,3-dihydro-benzofuran-2-carboxylic acid and 6,7-dichloro-5-[(5-methylene-thiazolidin-2-ylidene)acetyl-2,3-dihydro-benzofuran-2-carboxylic acid [21]

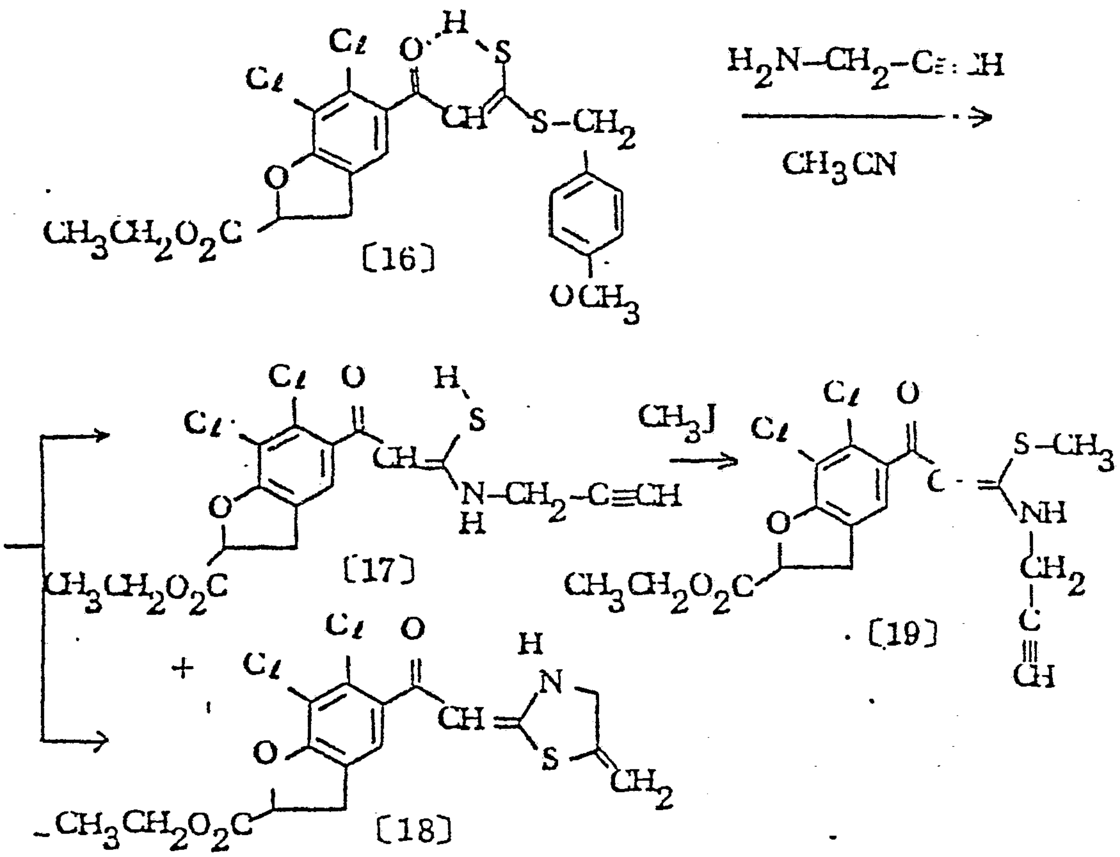


[16]

[17]

[18]

[19]

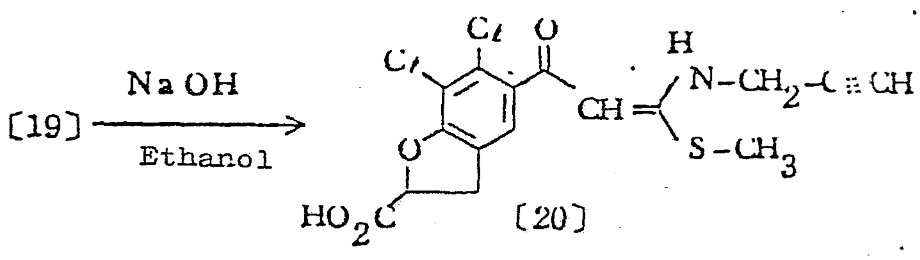


[19] [20]

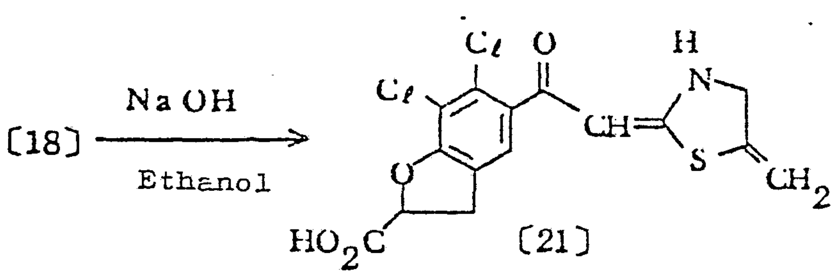


[18] [21]

A solution of 0.93 g (1.86 mmol) of ethyl 6,7-dichloro-5-[3-(4-methoxybenzylthio)-3-mercapto-2-propenoyl]-2,3-dihydrobenzofuran-2-carboxylate [16] (see, Example 35) and 0.113 g (2.05 mmol) of propargylamine dissolved in 2.3 ml of dry acetonitrile is allowed to react at room temperature overnight. The compound [18] precipitated as crystals are collected by filtration and washed with a small amount of ether to give 0.195 g of the compound [18], m.p. 135-136°C. The filtrate and the ether layer are combined and concentrated in vacuo to give a residue, to which 0.35 g of powdery anhydrous potassium carbonate, 0.317 g of methyl iodide, and 5 ml of dry acetonitrile are added, and the mixture is reacted at room temperature for 2 hours. The reaction mixture is concentrated in vacuo, and the residue extracted with dichloromethane, and purified by silica gel chromatography to give 0.105 g (total yield 0.3 g: 40.3%) of the compound [18] and 0.08 g (yield 10.7%) of the compound [19], m.p. 151-154°C.

IR $\nu$ max ($CHCl_3$): 3310 (-CCH), 1753, 1735, 1608, 1550 (br) $cm^{-1}$.

NMR $\delta$ ppm ($CDCl_3$): 11.50 (1H, br), 7.22 (1H, s-like), 5.34, 5.30 (2H, s, d-d), 4.27, 4.18 (4H, q+m), 3.8-3.25 (2H, m), 2.42, 2.35 (4H, s+m), 1.30 (3H, t).

A solution of 0.15 g of the compound [19] dissolved in 3 ml of a dichloroethane/ethanol (1:1) mixture is treated with 0.56 ml of 1N-sodium hydroxide for an hour for hydrolysis. The reactionm mixture is concentrated in vacuo, neutralized with 1N-hydrochloric acid to precipitate crystals, which are collected by filtration and washed with a small amount of ethanol to give 0.08 g of the titled compound [20]. This is recrystallized from ethanol to give 0.07 g (yield 45.6%: 4.9% from [16]) of crystals, m.p. 202-204°C (dec.).

Anal. Calcd.(%) for $C_{16}H_{13}Cl_2NO_4S$ 1/2$C_2H_5OH$: C, 48.93; H, 3.87; Cl, 16.99; N, 3.36; S, 7.68. Found (%): C, 49.15; H, 4.06; Cl, 16.76; N, 3.41; S, 7.66.

IR $\nu$ max ($CHCl_3$): 3300, 3260, 1744, 1610(br), 1550(br) $cm^{-1}$.

NMR $\delta$ ppm (DMSO,d-6): 11.27 (1H, t-br), 7.30 (1H, s), 5.5-5.27 (2H, s+d-d), 4.20 (2H, d-d), 3.8-3.2 (2H, m), 2.48-2.40 (4H, m+s).

On the other hand, the compound [18] is recrystallized from benzene to give crystals, m.p. 135-136°C.

Anal. Calcd.(%) for $C_{17}H_{15}Cl_2NO_4S$: C, 51.01; H, 3.78; Cl, 17.71; N, 3.50; S, 8.01. Found (%): C, 50.81; H, 3.73; Cl, 17.93; N, 3.48; S, 8.29.

IR $\nu$ max (Nujol): 3200 (br), 1755, 1735, 1591, 1523 $cm^{-1}$.

NMR $\delta$ ppm ($CDCl_3$): 10.40 (1H, br), 7.20 (1H, s), 5.50 (1H, s), 5.4-5.2 (3H, m), 4.64 (2H, t-like), 4.26 (2H, q), 3.8-3.2 (2H, m), 1.29 (3H, t).

In the same manner as on the compound [19], 0.2 g of the compound [18] is hydrolyzed to give 0.137 g of the titled compound [21], m.p. 141-143°C. This is recrystallized from ethanol/water to give 0.095 g (yield 48.7%; 19.6% from [16]) of crystals, m.p. 145-147°C.

Anal. Calcd.(%) for $C_{15}H_{11}Cl_2NO_4S$ $H_2O$: C, 46.16; H, 3.35; Cl, 18.17; N, 3.59; S, 8.21. Found (%): C, 46.36; H, 3.48; Cl, 18.38; N, 3.78; S, 8.27.

IR $\nu$ max (Nujol): 3570, 3200, 3000-1800 (br), 1715, 1590$cm^{-1}$

NMR $\delta$ ppm (DMSO d-6) as a mixture of keto and enol forms: 8.75 (br), 7.23 (1H, br), 5.76 (s), 5.5-5.25 (3H, m), 4.63 (m), 4.37 (2H, m), 3.85-3.2 (2H, m).

Example 81-82

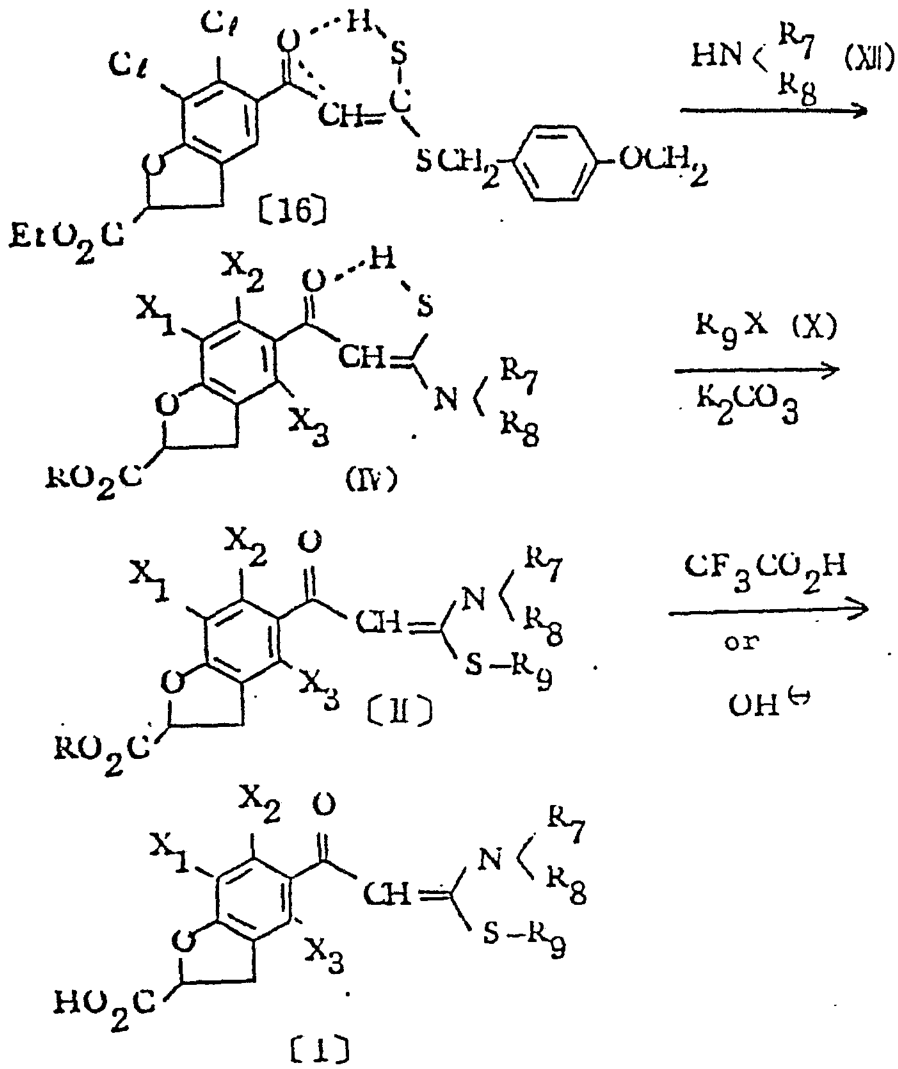


[16] (IV) [II] [I]

The compound [16] are reacted with an amine ( X II ) at room temperature in acetonitrile. The reaction mixture is concentrated in vacuo, chromatographed on silica gel to give the compound ( IV ), to which 1.5 eq. of powdery potassium carbonate and 1.2 eq. of R,X ( X ) are added, and the mixture is kept at room temperature in acetonitrile, then concentrated in vacuo to give a residue, which is purified by silica gel chromatography to give the compound ( II ). The compound ( II ) is hydrolyzed with sodium hydroxide and then neutralized with a diluted hydrochloric acid to precipitate the objective compound ( I ) as crystals, which is collected by

filtration and purified by recrystallization.

Some examples carried out in the manner as metioned above are shown in Table 7 (Nos. 1-4).

Table 7 ( No. 1 )

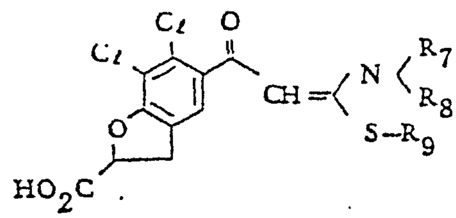

[I]81~82

| Example Nos. | $N<^{R_7}_{R_8}$ | $R_9$ | Yield (%) from [16] |
|---|---|---|---|
| 81 | $-N<^{Me}_{Me}$ | $CH_3$ | 18.1 |
| 82 | $-N<^{H}_{CH_2-CH=CH-CH_3}$ | $CH_3$ | 27.3 |

Table 7 ( No. 2 )

| Exa Nos | Amount Used g ( mmol ) 16 | [XI] | Reaction Temp. Time | Yield (%) [IV] | $R_9X$ | Reaction Temp. Time | Yield (II) |
|---|---|---|---|---|---|---|---|
| 81 | 0.51 (1.024) | $HN<^{Me}_{Me}$ 0.07 (1.54) | r.t. 18 | 39.0 | $CH_3J$ | rt 2 | ~100 |
| 82 | 0.705 (1.41) | $H_2-CH_2-CH=CH-CH_3$ 0.12 (1.7) | 13~25℃ 20 | 58.7 | $CH_3J$ | rt 2 | 95.6 |

Table 7 ( No. 3 )

| Ex. No. | Yield(%) [I]←[II] | [I] m.p. (℃) | Re-crystal from | Melecular formula | Anal. : Calcd./Found C | H | Cl | N | S |
|---|---|---|---|---|---|---|---|---|---|
| 81 | 46.5 | 230~232(d) | ethanol | $C_{15}H_{15}Cl_2NO_4S$ | 47.88<br>47.64 | 4.02<br>4.02 | 18.85<br>18.81 | 3.72<br>3.69 | 8.52<br>8.21 |
| 82 | 48.7 | 213~216 | ethanol | $C_{19}H_{21}Cl_2NO_4S$ | 50.76<br>50.71 | 4.26<br>4.28 | 17.63<br>17.75 | 3.48<br>3.49 | 7.97<br>7.74 |

Table 7 ( No. 4 )

| Exa. Nos. | IR ( $\nu^{Nujol}_{max}$ $cm^{-1}$ ) | NMR ( $\delta^{DMSO-d_6}_{ppm}$ ) |
|---|---|---|
| 81 | ~2500~(br) ~1900(br) 1730(br), 1610, 1560 | 7.22(1H, s) 5.40(1H, d-d) 5.14(1H, s) 3.8~3.1(8H, m+s) 2.40(3H, s) |
| 82 | 3200~2100, 2000~1800 1739, 1610, 1575, 1564 | 11.35(1H, br) 7.30(1H, s) 5.8~5.22(4H, m) 3.9~3.2(4H, m) 2.40(3H, s) 1.67(3H, s) |

Example 83

6,7-Dichloro-5-[3-cyclopropylamino-3-(methylthio)-2-propenoyl]-2,3-dihydro-1-benzofuran-2-carboxylic acid

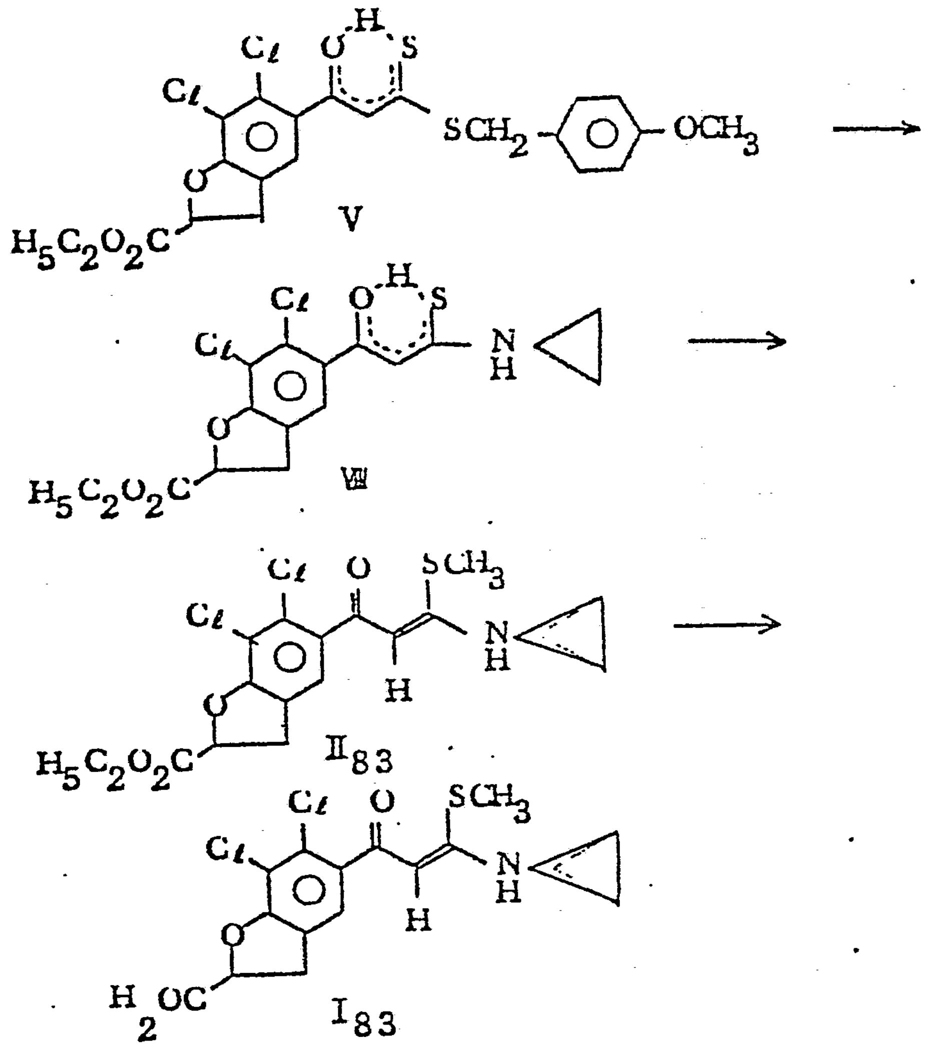

V

VIII

$II_{83}$

$I_{83}$

To 0.39 g (0.8 mmol) of the compound V (Example 35) are added 0.134 g (2.3 mmol) of cyclopropylamine and 2 ml of acetonitrile, and the mixture is allowed to react for 5 hours while being stirred at room temperature. The reaction product is applied to high performance liquid chromatography on Lober column (type B) with a benzene/ethyl acetate mixture (10:1) as an eluent to give 0.23 g (yield 73.0%) of the compound VIII as a pale yellow oil.

IR $\nu$ max ($CHCl_3$): 3430, 3330, 1750, 1770 (sh), 1610 $cm^{-1}$.

A mixture of 0.26 g (0.6 mmol) of the compound Ⅷ, 0.179 g (1.3 mmol) of dry powdery potassium carbonate, 0.14 g (1.0 mmol) of methyl iodide, and 4 ml of acetonitrile is reacted for an hour while being stirred at room temperature. The reaction product is subjected to liquid chromatography on a Lober column (type A) with a dichloromethane/ethyl acetate mixture (49:1) as an eluent to give 0.238 g (yield 88.5%) of the compound $II_{a3}$ as a pale yellowish oil.

IR $\nu$ max ($CHCl_3$): 3420, 1745, 1760 (sh), 1608, 1575, 1540 $cm^{-1}$.

NMR $\delta$ ppm ($CDCl_3$): 0.60-1.07 (4H, m), 1.32 (3H, t), 2.37 (3H, s), 2.40 (1H, bro), 3.03-3.77 (2H, m), 4.25 (2H, q), 5.13-5.47 (2H, m), 7.13 (1H).

In the same manner as in the above-mentioned Example, 0.230 g (0.6 mmol) of the compound $II_{a3}$ is hydrolyzed to give 0.207 g (yield 96.3%) of the compound $I_{a3}$, m.p. 240-245°C (dec.). This is recrystallized from a acetone/ethyl acetate mixture to give 0.20 g (yield 93.0%) of grayish white crystals, m.p. 242-246°C (dec.).

Anal. Calcd.(%) for $C_{16}H_{15}Cl_2NO_4S$: C, 49.44; H, 3.89; Cl, 18.26; N, 3.61; S, 8.26. Found (%): C, 49.26; H, 3.96; Cl, 18.15; N, 3.66; S, 8.20.

IR $\nu$ max (Nujol): 3130, 2690, 2580, 2490, 1732, 1608 $cm^{-1}$

Example 84

6,7-Dichloro-5-[3-morpholino-3-(methylthio)-2-propenoyl]-2,3-dihydro-1-benzofuran-2-carboxylic acid

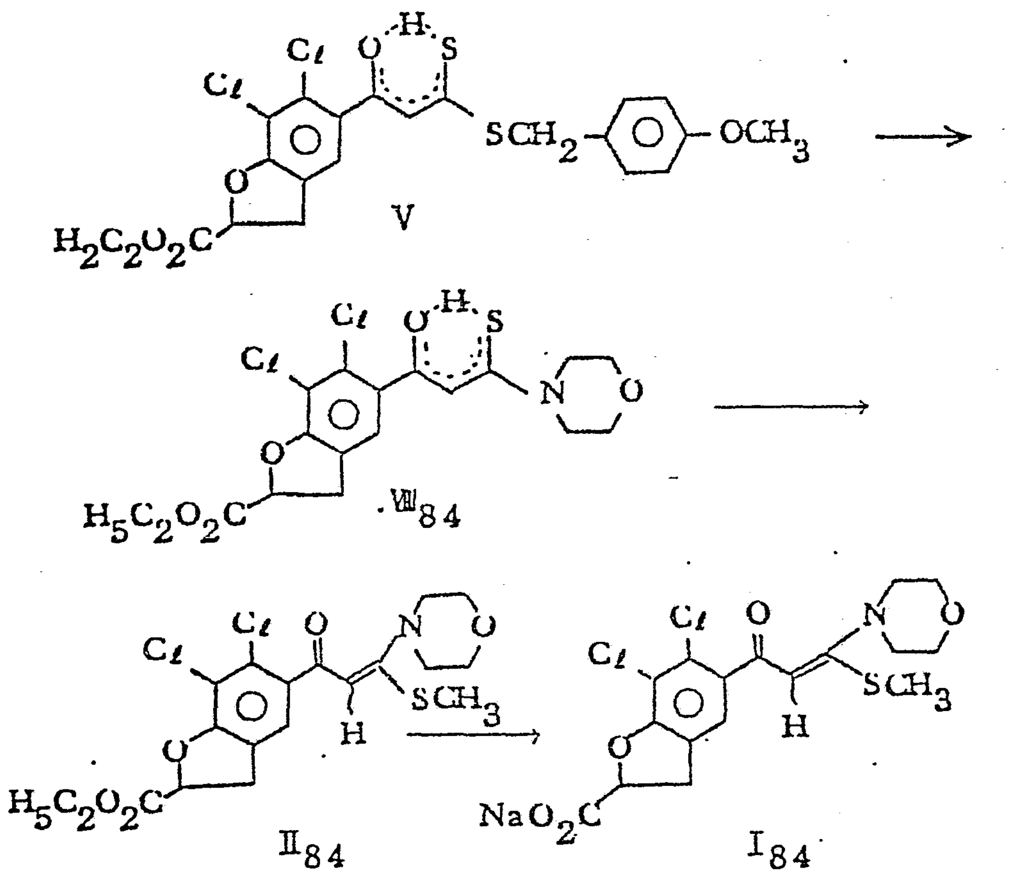

V

VIII$_{84}$

II$_{84}$

I$_{84}$

In the same manner as in Example 83, 0.29 g (0.6 mmol) of the compound V (Example 35) and 3 ml of morpholine are treated to give 0.22 g (yield 84.6%) of the compound VIII$_{84}$ as an oil.

NMR $\delta$ ppm ($CDCl_3$): 1.30 (3H, t), 3.13-4.53 (12H, m), 4.67 (2/3H, s), 5.17-5.58 (1H, m), 5.88 (1/3H, s), 7.25, 7.50 (1H), 15.02 (2/3H, s).

In the same manner as in Example 83 are treated 0.22 g (0.5 mmol) of the compound VIII$_{84}$, 0.136 g (1.0 mmol) of powdery potassium carbonate, 0.14 g (1.0 mmol) of methyl iodide, and 2.4 ml of acetonitrile to give 0.225 g (yield 86.5%) of the compound II$_{84}$ as an oil.

NMR $\delta$ ppm ($CDCl_3$): 1.30 (3H, t), 2.40 (3H, s), 3.10-3.93

(10H, m), 4.23 (2H, q), 5.12-5.45 (2H, m), 7.15 (1H).

In the same manner as in Example 83, 0.210 g (0.5 mmol) of the compound $II_{84}$ is hydrolyzed to give 0.150 g (yield 75.8%) of the compound I , m.p. 215-216°C (dec.). This is reacted with 13.86 mg of sodium hydroxide in 3.3 ml of water for 30 minutes, and insoluble substances are removed by filtration (twice). The filtrate is evaporated to dryness, then treated with ethyl acetate, and recrystallized from an ethanol/ethyl acetate mixture to give 0.140 g (yield 66.4%) of grayish white crystals, m.p. 230-232°C (dec.).

Anal. Calcd.(%) for $C_{17}H_{16}Cl_2NO_6S_2Na$ 1/2$H_2O$: C, 45.44; H, 3.81; Cl, 15.78; N, 3.12; S, 7.13; $H_2O$, 2.00. Found (%): C, 45.47; H, 3.84; Cl, 15.88; N, 3.19; S, 7.38; $H_2O$, 2.12.

IR $\nu$ max (Nujol): 3300, 1621 (1615) $cm^{-1}$.

Example 85

6,7-Dichloro-5-[3-(methylthio-3-pyrrolidino)-2-propenoyl]-2,3-dihydro-1-benzofuran-2-pyrrolidinamide

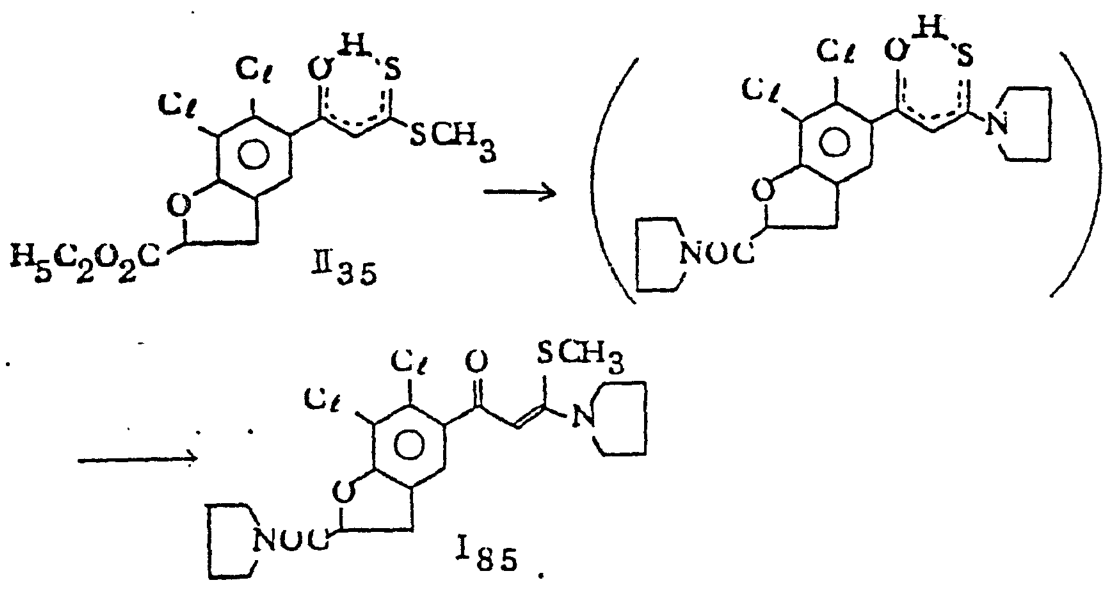

$II_{35}$

$I_{85}$.

A mixture of 0.194 g (0.5 mmol) of the compound $II_{35}$ (Example 35) with 2 ml of pyrrolidine is reacted at room temperature for an hour, then azeotropically distilled with

toluene to remove an excess of pyrrolidine. To the residue are added 0.140 g (1.0 mmol) of powdery potassium carbonate, 0.08 g (0.6 mmol) of methyl iodide, and 2 ml of N,N-dimethylformamide, and the mixture is allowed to react for an hour while being stirred at room temperature. The product is chromatographed on a Lober column (type B) with a dichloromethane/ethyl acetate mixture (9:1) as an eluent to give 0.190 g (yield 84.4%) of the compound $I_{85}$, m.p. 110-114°C. This is recrystallized from isopropyl ether/ethyl acetate to give 0.085 g (yield 37.8%) of compound $I_{85}$ as grayish white crystals, m.p. 115-116°C.

Anal. Calcd.(%) for $C_{21}H_{24}Cl_2N_2O_3S$: C, 55.38; H, 5.31; Cl, 15.57; N, 6.15; S, 7.04. Found (%): C, 55.10; H, 5.15; Cl, 15.65; N, 6.11; S, 6.79.

IR $\nu$ max (Nujol): 1650, 1603, 1590 $cm^{-1}$.

NMR $\delta$ ppm ($CDCl_3$): 1.63-2.28 (8H, m), 2.47 (3H, s), 3.07-4.05 (10H, m), 5.15 (1H, s), 5.23-5.58 (1H, m), 7.20 (1H).

Example 86

6,7-Dichloro-5-[2-(1,3-thiazolidin-2-ylidene)acetyl]-1-benzofuran-2-carboxylic acid

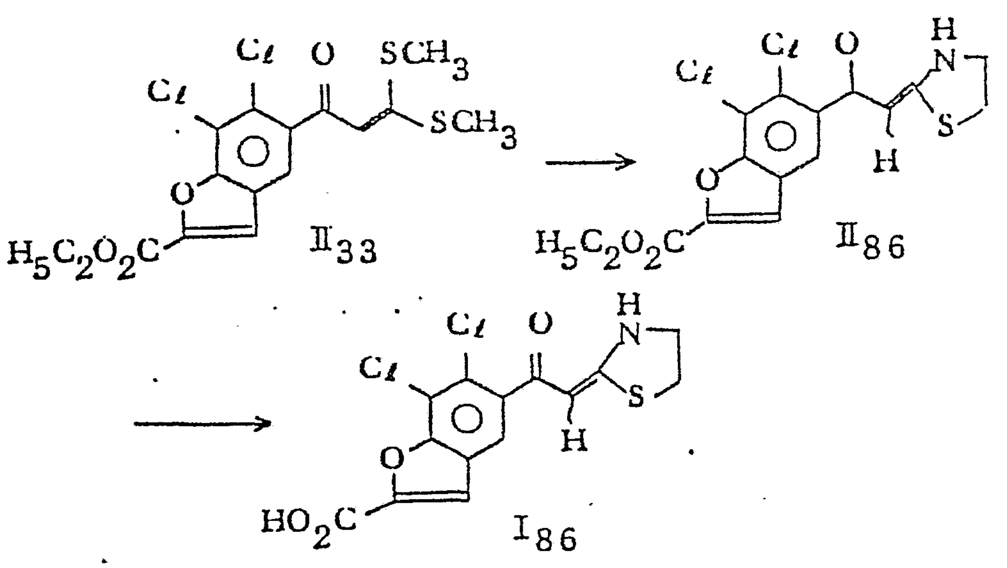

$II_{33}$ $II_{86}$ $I_{86}$

In the same manner as in Example 19 or 34, 0.173 g (0.4

mmol) of the compound $II_{33}$ (Example 33) is subjected to the reaction to give 0.107 g (yield 64.2%) of the compound $II_{86}$, m.p. 226-223°C.

IR $\nu$ max (Nujol): 3230, 3100, 1715, 1610 $cm^{-1}$.

NMR $\delta$ ppm ($CDCl_3$): 1.43 (3H, t), 3.13-4.23 (4H, m), 4.43 (2H, q), 5.57 (1H, s), 7.50 (1H, s), 7.63 (1H, s).

In the same manner as in Example 33, 0.100 g (0.3 mmol) of the compound $II_{86}$ is hydrolyzed to give 0.093 g (yield 100%) of the compound $I_{86}$, m.p. 265-271°C (dec.). This is recrystallized from acetone to give 0.087 g (yield 87.0%) of grayish white crystals, m.p. 268-272°C (dec.).

Anal. Calcd.(%) for $C_{14}H_9Cl_2NO_4S$ 1/2$(CH_3)_2CO$: C, 48.07; H, 3.12; Cl, 18.31; N, 3.62; S, 8.28. Found (%): C, 48.35; H, 3.20; Cl, 18.55; N, 3.75; S, 8.51.

IR $\nu$ max (Nujol): 3235, 2675, 2540, 2460, 1705, 1612 $cm^{-1}$.

Example 87

Production of 6,7-dichloro-5-[3-(furfurylamino-3-(methylthio)-2-propenoyl]-2,3-dihydrobenzofuran-2-carboxylic acid [33]

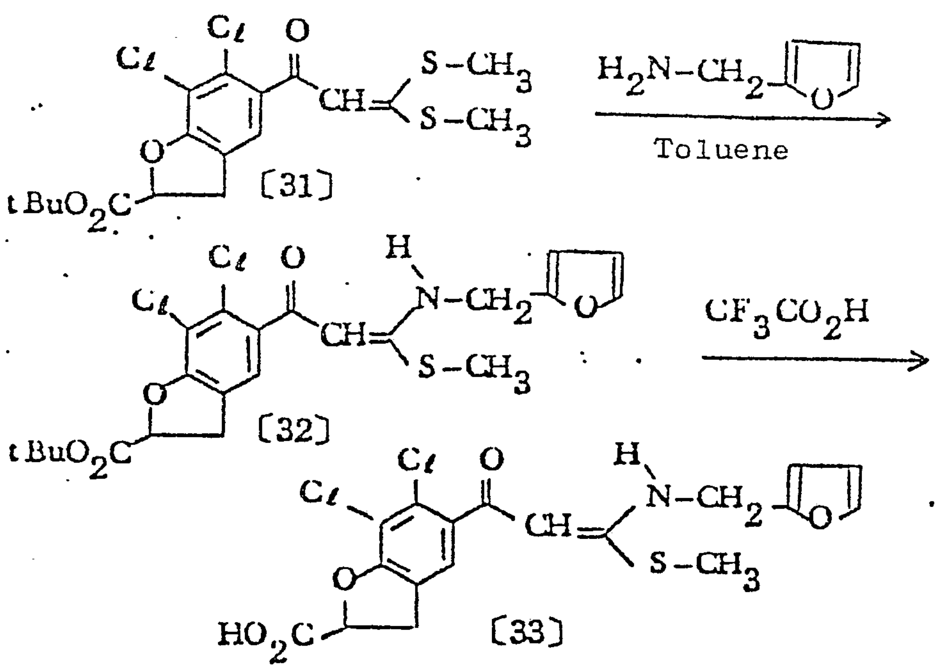

STEP 1

A mixture of 0.435 g (1 mmol) of tert-butyl 6,7-dichloro-5-(3,3-bismethylthio-2-propenoyl)-2,3-dihydrobenzofuran-2-carboxylate [31], 0.117 g (1.2 mmol) of furfurylamine, and 1 ml of dry toluene is refluxed for 9 hours under heating. After condensation in vacuo, the residue is purified by silica gel chromatography to give 0.365 g (yield 75.3%) of the resinous compound [33].

IR $\nu$ max ($CHCl_3$): 1749 (br), 1562 (br) $cm^{-1}$.

NMR $\delta$ ppm ($CDCl_3$): 11.65 (1H, br: $D_2O$ exchange), 7.38 (1H, m), 7.19 (1H, m), 6.31 (2H, d-like), 5.31 (s), 5.15 (m), 4.55 (2H, d), 3.73-3.15 (2H, m), 2.40 (3H, s), 1.48 (9H, s).

STEP 2

A mixture of 0.36 g (0.74 mmol) of the compound [32] and 3.6 ml of trifluoroacetic acid is stirred at room temperature for 0.5 hour. After condensation in vacuo, the residue is recrystallized from a small amount of ether to give 0.30 g of the titled compound [33]. This is recrystallized from ethanol to give 0.22 g of crystals (yield 69.4%; 52.3% from [31]), m.p. 214-216°C (dec.).

Anal. Calcd.(%) for $C_{18}H_{15}Cl_2NO_5S$: C, 50.48; H, 3.53; Cl, 16.55; N, 3.27; S, 7.48. Found (%): C, 50.29; H, 3.61; Cl, 16.38; N, 3.22; S, 7.39.

IR $\nu$ max (Nujol): 3200-2200(br)-1950(br), 1740, 1560 $cm^{-1}$.

NMR $\delta$ ppm (DMSO d-6): 11.53 (1H, t-br: $D_2O$ exchange), 7.65 (1H, m), 7.32 (1H, s-like), 6.42 (2H, m) [5.42 (s) + 5.15 (d) 2H], 4.10 (2H, d), 3.84-3.2 (2H, m), 2.45 (3H, s).

Anal. Calcd.(%) for $C_{15}H_{11}Cl_2NO_4S$: C, 48.40; H, 2.98; Cl, 19.05; N, 3.76; S, 8.61. Found (%): C, 48.31; H, 3.26; Cl, 18.80; N, 3.64; S, 8.47.

IR ν max (Nujol): 3365, 1752, 1602, 1585, 1570 $cm^{-1}$.

NMR δ ppm (DMSO d-6): 11.60 (1H, br), 7.23 (1H, s), 6.85 (1H, m), 6.18 (1H, m), 5.50 (1H, d-d), 3.9-3.2 (2H, m), 2.48 (3H, s).

Example 88-97

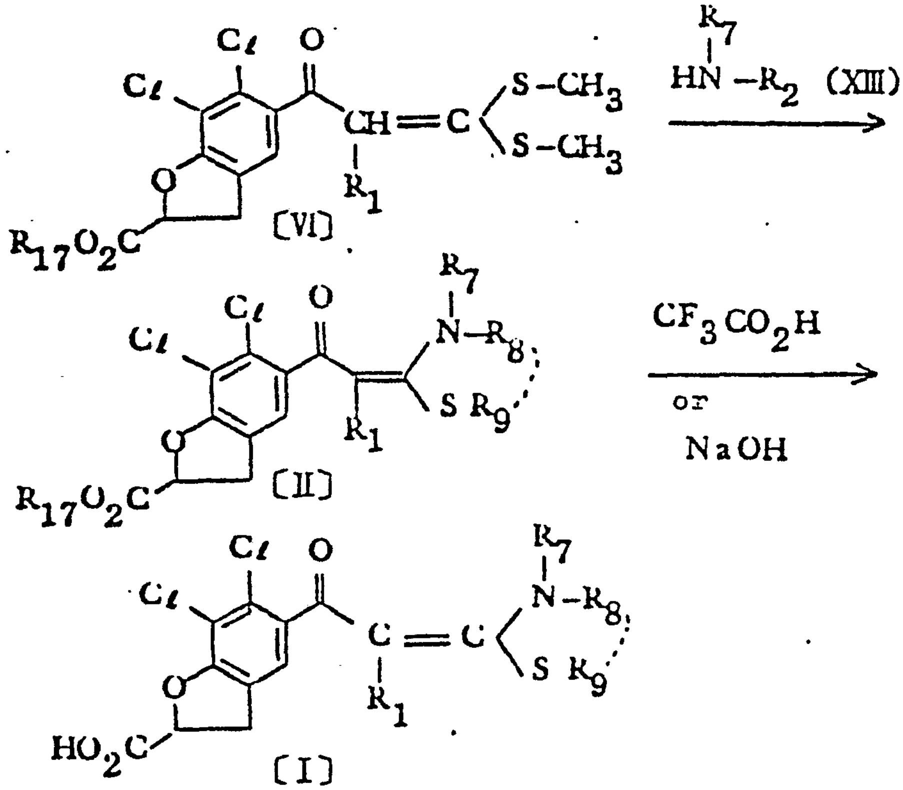


[VI] [II] [I]

A mixture of a compound (VI) and 1.2 eq. amine compound (XIII) in a solvent is refluxed under heating for 3.5 to 72 hours.

After condensation in vacuo, thus obtained residue is chromatographed on silica gel to give a compound (II), which is treated with trifluoroacetic acid (Method A) or with sodium hydroxide (Method B) for hydrolysis to give a compound (I). This is refined by recrystallization. Some examples are shown in the following Table 8 (Nos. 1-4).

Table 8 (No. 1)

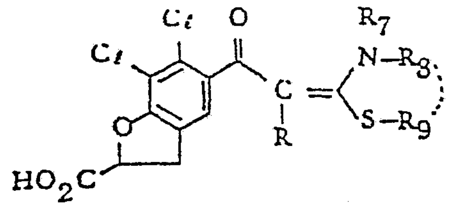

[I]88~97

| Example Nos. | R | $R_7$ | $R_8$ | $R_9$ | Yield (%) from [VI] |
|---|---|---|---|---|---|
| 88 | H | H | 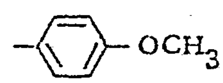 | Me | 34.0 |
| 89 | H | H | 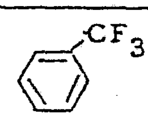 | Me | 17.6 |
| 90 | H | H | 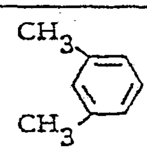 | Me | 29 -a) |
| 91 | H | H | 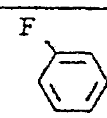 | Me | 35.5 |
| 92 | H | H | 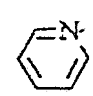 | Me | 28.9 |
| 93 | H | H | 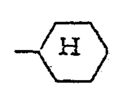 | Me | 49.8 |
| 94 | H | H | $-CH_2-CH_2-$ | | 55.4 |
| 95 | Me | H | $-CH_2-CH_2-$ | | 22.8 |
| 96 | H | Me | $-CH_2-CH_2-$ | | 69.7 |
| 97 | H | H | 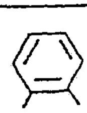 | | 24.6 |

a) hydrolysies with NaOH (Method B)

Table 8 ( No. 2 )

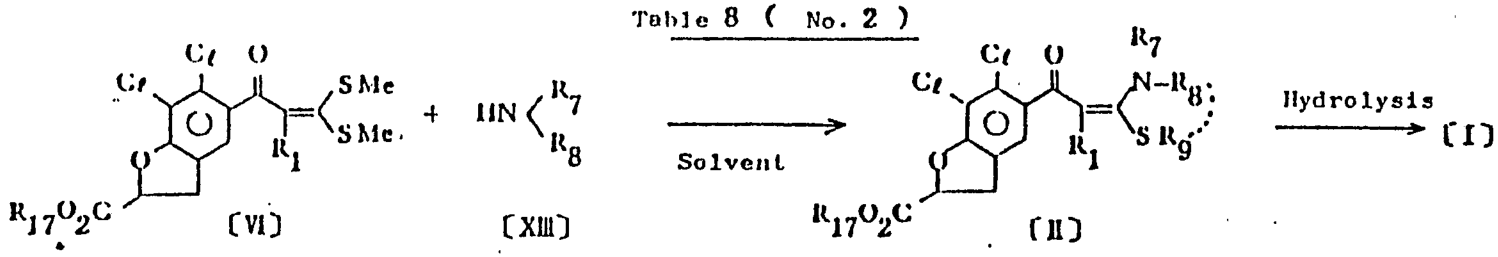


| Exampl Nos. | Amount Used g (mmol) [IX] $R_7$ | [X] $-N<^{R_7}_{R_8}$ | Reaction Solvent(ml) | Temp. | Time | [II] Yield(%) | [I] Yield(%) |
|---|---|---|---|---|---|---|---|
| 88 | t-Bu 0.435(1) | $-\overset{H}{N}C_6H_4-OMe$ (4) | xylene (3) | reflux temp. | 3.5 | 70.5 | 48.2 |
| 89 | 〃 | $-\overset{H}{N}C_6H_4-CF_3$ (3) | toluene (3) | 〃 | 72 | 43.9 | 40.0 |
| 90 | Et 0.53 (1.3) | $-\overset{H}{N}C_6H_3-(Me)_2$ (2.6) | xylene (3) | 〃 | 20 | 40.0 | 72.3 |
| 91 | t-Bu 0.57 (1.3) | $-\overset{H}{N}C_6H_4-F$ (2) | toluene (1.5) | 〃 | 30 | 42.0 | 84.5 |
| 92 | 〃 0.485(1.1) | 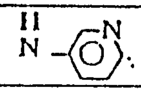 | xylene (2.5) | 〃 | 20 | 51.0 | 56.7 |
| 93 | 〃 0.52 (1.2) | 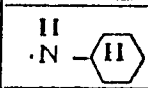 | xylene (3) | 〃 | 22 | 93.0 | 53.5 |
| 94 | 〃 1.31 (3) | $-\overset{H}{N}-CH_2-CH_2-SH$ | toluene (10) | 〃 | 15 | 72.0 | 77.0 |
| 95 | 〃 2.0 (4.6) | 〃 | toluene (20) | 〃 | 24 | 25.1 | 91.0 |
| 96 | 〃 1.0 (2.3) | $-\overset{H}{N}-CH_2-CH_2-SH$ | toluene (10) | 〃 | 16 | 91.0 | 76.6 |
| 97 | 〃 1.0 (2.3) | 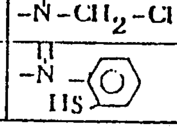 | toluene (10) | 〃 | 72 | 35.7 | 68.9 |

Table 6 ( No. 3 )

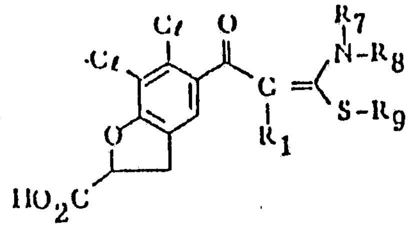

(I)88~97

| Exm. Nos. | Recrystal from | m.p. (℃) | Molecular formula | Elementary Analysis Calcd. (%) C | H | Cl | N | S | Found (%) C | H | Cl | N | S |
|---|---|---|---|---|---|---|---|---|---|---|---|---|---|
| 88 | dioxane | 240~243 (d) | $C_{20}H_{17}Cl_2NO_5S$ | 52.87 | 3.77 | 15.61 | 3.08 | 7.06 | 52.74 | 3.77 | 15.95 | 2.84 | 7.21 |
| 89 | ethanol | 226~228 (d) | $C_{20}H_{14}Cl_2F_3NO_4S$ | 48.79 | 2.87 | 14.40 | 2.85 | 6.51 | 48.74 | 3.04 | 14.13 | 2.80 | 6.65 |
| 90 | 〃 | 252~255 (d) | $C_{21}H_{19}Cl_2NO_4S$ | 55.76 | 4.23 | 15.67 | 3.10 | 7.09 | 55.60 | 4.32 | 15.47 | 3.11 | 6.94 |
| 91 | 〃 | 237~242 (d) | $C_{19}H_{14}Cl_2NO_4S$ | 51.60 | 3.19 | 16.03 | 3.17 | 7.25 | 51.38 | 3.33 | 16.06 | 3.08 | 7.48 |
| 92 | 〃 | 227~232 (d) | $C_{18}H_{14}Cl_2N_2O_4S$ | 50.84 | 3.32 | 16.67 | 6.59 | 7.54 | 50.63 | 3.57 | 16.67 | 6.58 | 7.30 |
| 93 | 〃 | 167~168 | $C_{19}H_{21}Cl_2NO_4S$ | 53.03 | 4.92 | 16.48 | 3.25 | 7.45 | 52.92 | 4.95 | 16.37 | 3.30 | 7.38 |
| 94 | 〃 | 252~254 (d) | $C_{14}H_{11}Cl_2NO_4S$ | 46.68 | 3.08 | 19.68 | 3.89 | 8.90 | 46.50 | 3.21 | 19.58 | 3.88 | 8.69 |
| 95 | DMF - water | 264~266 (d) | $C_{15}H_{13}Cl_2NO_4S$ | 48.13 | 3.50 | 18.95 | 3.74 | 8.57 | 47.99 | 3.64 | 18.89 | 4.00 | 8.26 |
| 96 | DMF-ethanol | 272~275 (d) | $C_{15}H_{13}Cl_2NO_4S$ | 48.14 | 3.50 | 18.95 | 3.74 | 8.57 | 47.93 | 3.57 | 18.55 | 3.79 | 8.45 |
| 97 | 〃 | 259~261 (d) | $C_{18}H_{11}Cl_2NO_4S$ | 52.96 | 2.72 | 17.37 | 3.43 | 7.85 | 52.66 | 2.91 | 17.37 | 3.31 | 7.98 |

Table 8 ( No. 4 )

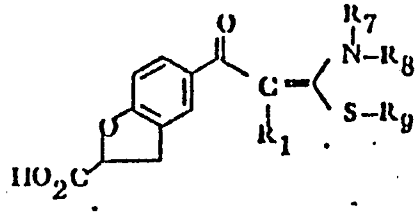

[I]$_{88\sim97}$

| Example Nos. | IR ( $\nu_{max}^{Nujol}$ $cm^{-1}$ ) | NMR ( $\delta_{ppm}^{DMSO d-6}$ ) |
|---|---|---|
| 88 | 3200~2100~1800, 1737, 1610, 1555, 1510 | 12.85(1H, br) 7.39(1H) 7.25(2H, d) 6.95(2H, d) [5.48(s)+5.43(m), 2H)] 3.85~3.20(5H, m) 2.38(3H, s) |
| 89 | 3200~2400, 1737, 1604, 1571 | 13.0(1H, s, br) 7.63~7.28(5H, m) 5.60(1H, s) 2.40(3H, s) |
| 90 | 3200~2300(br), 1775, 1733, 1612, 1528, 1493 | 12.40(1H, brs) 7.43(1H, s) 7.17(3H, s) 5.49(1H, s) 5.45(1H, dd), 3.9~3.2(2H, m) 2.33(3H, s) 2.20(6H, s) |
| 91 | 3200~2400(br), 1743, 1618, 1603, 1565 | 12.81(1H, s; br) 7.7~7.2(5H, m) 5.61(1H, s) 2.43(3H, s) |
| 92 | 3200~2450(br), 1720(br) 1605~1550(br) | 12.87(1H, s, br) 8.6~7.4(5H, m) 5.61(1H, s) 2.44(3H, s) |
| 93 | 3200~2400(br), 2000~1850 (br), 1743, 1608, 1562 | 11.55(1H, d) 7.30(1H, s) 5.20(1H, s) 3.8~3.18(3H, m) 2.40(3H, s) 2.1~1.2(10H, br) |
| 94 | 3240~2500(br), 1730(br), 1614, 1567 | 10.2(br)+8.55(br): 1H 7.25(1H, s, br) [5.75(br)+5.3(m)]: 2H 4.0~3.0(6H, m) |
| 95 | 3220~2720~2400, 1730, 1610, 1570 | 10.50+7.75(br, 1H) 7.01(1H, br) 5.40(1H, m) 4.0~3.0(6H, m) 1.61(3H, br) |
| 96 | ~2420~(br), 2000~1800, 1730(br), 1609, 1565. | 7.25(1H, s) 5.56(1H, s) 5.42(1H, m) 3.8~2.8(9H) |
| 97 | ~2500(br)~1800~(br) 1719, 1688, 1606 | 13~12(1H, br) 7.9~7.1(m, 5H) 6.27(1H, s) 5.45(1H, dd) 3.85~3.2(2H, m) |

Example 98

Production of 6,7-dichloro-5-[2-(methylthio)pyrrol-3-yl-carbonyl]-2,3-dihydrobenzofuran-2-carboxylic acid [36]

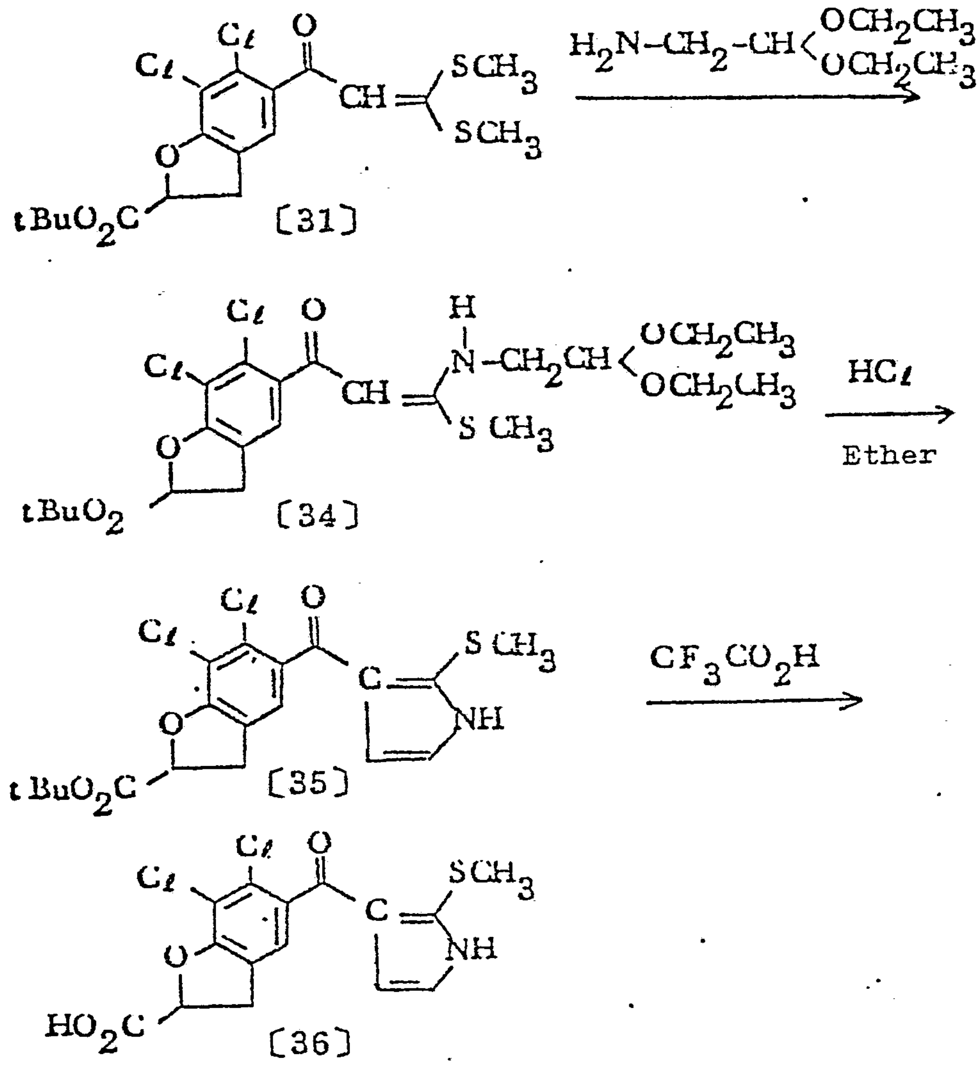


[31]

[34]

[35]

[36]

In the same manner as in Example 97, 1.09 g (2.5 mmol) of the compound [31] is reacted with 0.33 g (2.5 mmol) of 2,2-diethoxyethylamine in toluene for 16 hours to give 1.02 g (yield 80.9%) of the compound [34].

NMR $\delta$ ppm ($CDCl_3$): 11.45 (1H, t-like), 7.22 (1H, s), 5.30 (1H, s), 5.19 (1H, d-d), 4.69 (1H, t), 3.85-3.2 (8H, m), 2.40 (3H, s), 1.48 (9H, s), 1.26 (6H, t).

To a solution of 0.9 g (1.78 mmol) of the compound [34] in

an ether (15 ml) : dichloromethane (15 ml) mixture is added 8 ml (8 mmol) of 1N-hydrochloric acid in ether anhydrous, and the mixture is allowed to react at 5-25°C overnight. After condensation in vacuo, the residue is dissolved in dichloromethane, washed with an aqueous solution of sodium hydrogencarbonate, and then purified by silica gel chromatography to give 0.445 g (yield 58.2%) of the compound [35], m.p. 187-188°C.

NMR $\delta$ ppm ($CDCl_3$): 11.58 (1H, br), 7.20 (1H, s), 6.82 (1H, m), 6.15 (1H, m), 5.42 (1H, d-d),, 3.85-3.15 (2H, m), 2.45 (3H, s), 1.47 (9H, s).

The compound [35] (0.445 g) is allowed to react with 5 ml of trifluoroacetic acid at room temperature for 1 hour, the reaction mixture is concentrated in vacuo, crystallized from ether, washed with a small amount of 50% ethanol, and recrystallized from 95% ethanol to give 0.21 g (yield 54.3%) of the titled compound [36], m.p. 232-234°C.

Example 99

Production of 6,7-dichloro-5-[(3-methyl-4-thiazolin-2-ylidene)acetyl]-2,3-dihydrobenzofuran-2-carboxylic acid [41] and the sodium salt [42]

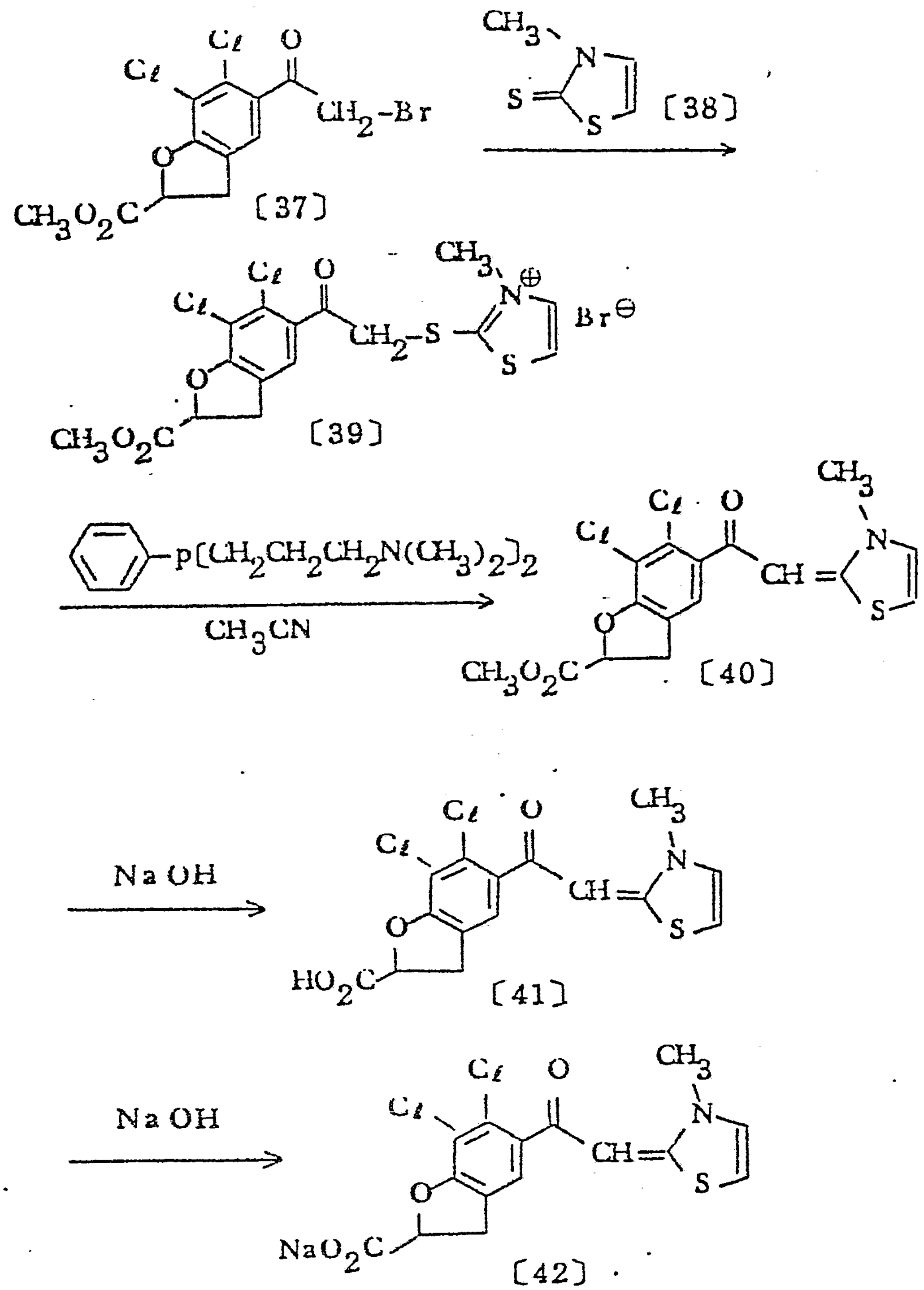


[37] [38] [39] [40] [41] [42]

In 10 ml of dry acetone, 3.68 g (10 mmol) of methyl 6,7-dichloro-5-bromoacetyl-2,3-dihydrobenzofuran-2-carboxylate [37] is treated, at 40-45°C for 5 hours, with 1.31 g (10 mmol) of N-methylthiazole-2-thione [38] (prepared according to M.O. Kolosova *et al.*, J. Gen. Chem.(USSR) *33* (8), 2706 (1963)). To the reaction

mixture is added 10 ml of benzene, and the precipitating crystals are collected by filtration and washed with a small amount of acetone to give 4.73 g (yield 94.8%) of the compound [39], m.p. 126-128°C.

NMR $\delta$ ppm (DMSO d-6): 8.42 (1H, d), 8.15 (1H, d), 7.96 (1H, s), 5.68 (1H, d-d), 5.40 (2H, s), 4.06 (s)-3.73 (s)-3.20 (8H, m).

To a suspension of 1.50 g (3 mmol) of the compound [39] in 10 ml of acetonitrile is added a solution of 0.308 g (3.3 mmol) of phenyl-bis[N,N-dimethylaminopropyl]phosphine (prepared according to Loeliger P. Org. Synthesis *55*, 127 (1976)) in 1 ml of acetonitrile while being stirred under ice-cooling, and then the mixture is allowed to react for 0.5 hour while being stirred at room temperature. After condensed in vacuo, the residue is dissolved in dichloromethane, washed with 1N-sodium dihydrogenphosphate ($NaH_2PO_4$) solution and with water, dried over anhydrous magnesium sulfate, concentrated in vacuo, and then purified by silica gel chromatography (Lober column type B) to give 0.94 g (yield 81.1%) of an oily material, which is the methyl ester [40] of the titled compound.

IR $\nu$ max ($CHCl_3$): 1760, 1743, 1609, 1564 $cm^{-1}$.

NMR $\delta$ ppm ($CDCl_3$): 7.31 (1H, s-like), 6.88 (1H, d), 6.48 (1H, d), 6.04 (1H, d), 3.80 (3H, s), 3.7-3.2 (5H, m+s).

To a solution of 1.2 g (2.75 mmol) of the compound [40] dissolved in 15 ml of an ethanol/dichloromethane (2/1) mixture is added 4.7 ml (4.7 ml) of 1N-sodium hydroxide, and the mixture is subjected to hydrolysis at room temperature for an hour. After condensed in vacuo, the residue is adjusted to pH 3 with dil. hydrochloric acid and acetic acid to precipitate crystals, which are collected by filtration and washed with water and with ethanol

to give 1.09 g (yield 95%) of the titled compound, m.p. 280-283°C (dec.).

This is recrystallized from DMF/ethanol to give yellowish crystals, m.p. 280-283°C (dec.).

Anal. calcd.(%) for $C_{16}H_{11}Cl_2NO_4S$: C, 48.40; H, 2.98; Cl, 19.05; N, 3.76; S, 8.61. Found (%): C, 48.28; H, 3.11; Cl, 18.98; N, 3.77; S, 8.71.

IR $\nu$ max (Nujol): 3140, 3120, 2000 (br), 2000-1800, 1733, 1607, 1520 $cm^{-1}$.

NMR $\delta$ ppm (DMSO d-6): 7.40-7.35 (2H, m), 6.30 (1H, d), 6.00 (1H, s-like), 5.43 (1H, d-d), 3.85-3.2 (5H, m+s).

The starting material, *i.e.*, methyl 6,7-dichloro-5-(bromoacetyl)-2,3-dihydrobenzofuran-2-carboxylate [37] can be prepared according to the following reaction scheme.

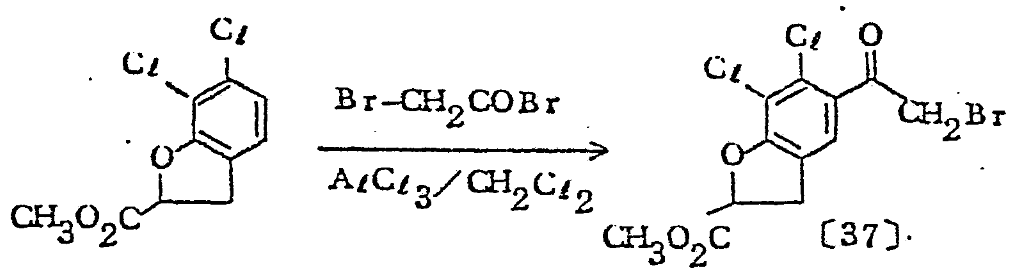


[37].

To a solution of 6.2 g (25 mmol) of methyl 6,7-dichloro-2,3-dihydrobenzofurancarboxylate (m.p. 113-114°C: prepared according to Willam F. Hoffman J. Med. Chem., *24*, 865 (1981)) and 6.56 g (32.5 mmol) of bromoacetyl bromide dissolved in 62 ml of dry dichloromethane is added 8.6 g (65 mmol) of anhydrous aluminium chloride under ice-cooling, and then the mixture is allowed to react at room temperature for 3 hours. The reaction mixture is poured into a mixture of ice and hydrochloric acid, then extracted with dichloromethane, and washed with water. The organic layer is dried over anhydrous magnesium sulfate, and concentrated in vacuo

to give 8.5 g of crystals. This is recrystallized from benzene/cyclohexane to give 7.5 g (yield 81.5%) of the objective compound [37], m.p. 108-111°C.

NMR $\delta$ ppm ($CDCl_3$): 7.35 (1H, s-like), 5.38 (1H, d-d), 4.48 (2H, s), 3.88-3.3 (5H, m+s).

※ Production of sodium salt [42]:

In 9.9 ml (99% molar ratio) of 0.1N-sodium hydroxide is dissolved 0.372 g (1 mmol) of the carboxylic acid [41]. The insoluble carboxylic acid is removed by filtration and the filtrate is concentrated in vacuo to give a residue, which is rcrystallized from a small amount of water, collected by filtration under cooling, and washed with a small amount of ethanol to give 0.255 g (63.3%) of the sodium salt [42] containing 1/2 molecule of $H_2O$.

Anal. calcd.(%) for $C_{18}H_{10}Cl_2NNaO_4S$ 1/2$H_2O$: C, 44.68; H, 2.75; Cl, 17.59; N, 3.47; Na, 5.70; S, 7.95. Found (%): C, 44.83; H, 2.89; Cl, 17.84; N, 3.56; Na, 5.60; S, 8.17.

IR $\nu$ max (Nujol): 3400, 3150, 1622, 1568, 1492 $cm^{-1}$.

Example 100-102

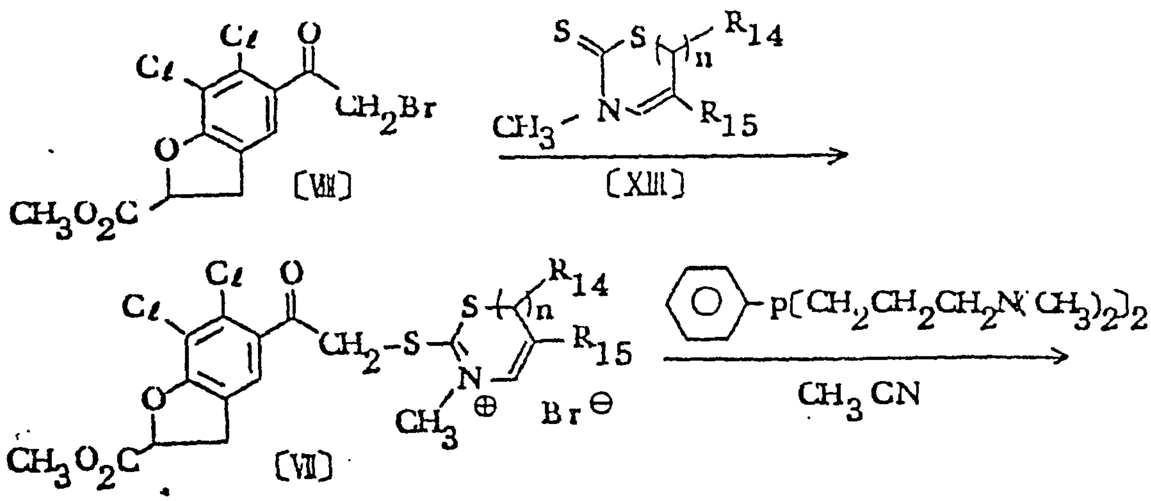

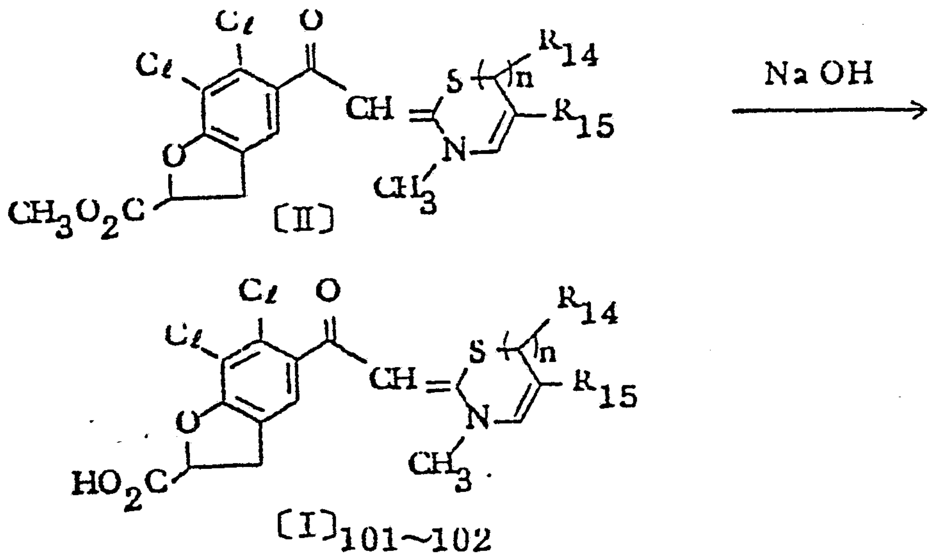


(II)

(I)101~102

STEP 1

A solution of the compound (Ⅷ) and a compound (XⅢ) (1.1 molar eq. each) dissolved in dichloromethane or acetone is kept at room temperature for 2-3 days while being stirred. Ether is added to the reaction mixture to precipitate crystals, which is washed with ether to give a compound (Ⅶ). This may be used for the following step without purification.

STEP 2

To a suspension of a compound (Ⅶ) in dry acetonitrile is added 1.1 molar eq. of phenyl bis-(N,N-dimethylpropyl)phosphine, and the mixture is allowed to react at room temperature for an hour. After condensation in vacuo, the residue is dissolved in dichloromethane. The solution is washed with 1N-sodium hydrogenphosphate, then with water, dried over anhydrous magnesium sulfate, concentrated in vacuo, and purified by silica gel chromatography to give a compound (Ⅱ).

STEP 3

To a solution of a compound (Ⅱ) dissolved in ethanol or an ethanol/dichloromethane mixture is added 1.5 eq. of 1N-sodium

hydroxide for hydrolysis at room temperature. The reaction mixture is neutralized with dil. hydrochloric acid to precipitate crystals, which are recrystallized from a proper solvent to give a compound ( I ). Some examples are shown in Table 9 (Nos. 1-4).

Table 9 ( No. 1 )

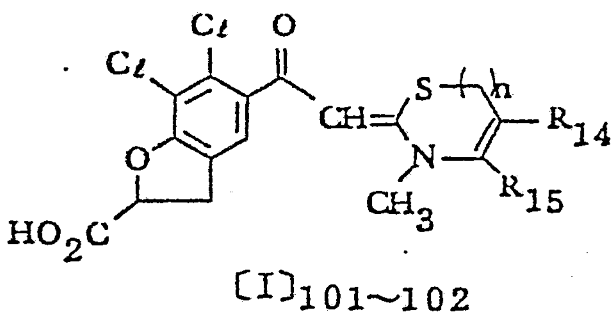

〔I〕101〜102

| Example Nos. | 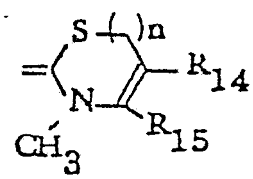 | Yield from [37] |
|---|---|---|
| 100 | 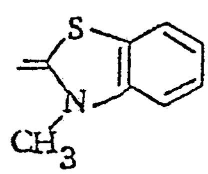 | 44.2% |
| 101 | 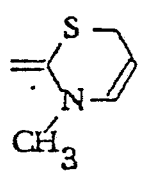 | 43.2% |
| 102 | 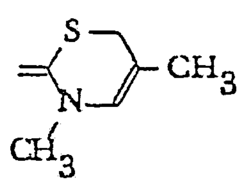 | 60.4% |

Table 9 ( No. 2 )

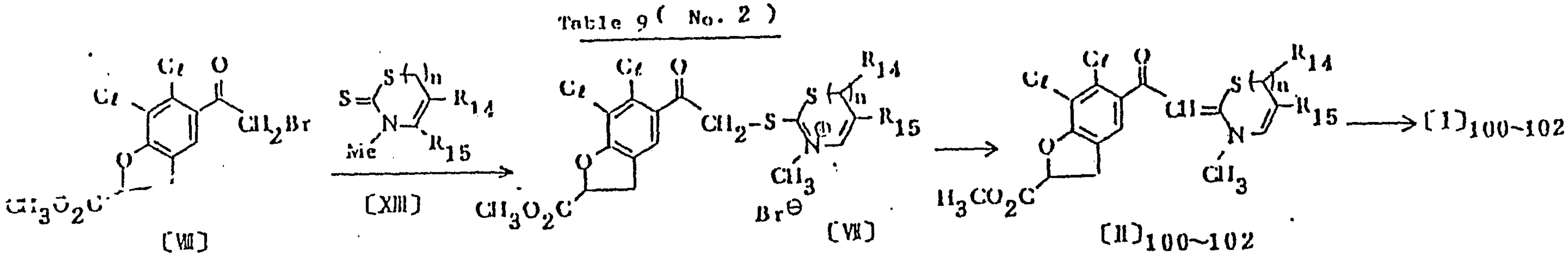

[VIII] [XIII] [VII] [II]100~102 → [I]100~102

| Example Nos. | Amount Used g ( mmol ) [VIII] | 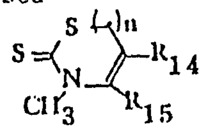 [XIII] | Solvent (ml) | Temp. | Time | [VI] Yield (%) | [VI] m.p. (℃) | [II] Yield(%) | [I] Yield(%) |
|---|---|---|---|---|---|---|---|---|---|
| 100 | 1.47 (4) | 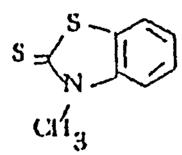 0.8 (4.4) | $CH_2Cl_2$ | r.t. | 3 Days | 89.5 | 121~122 | 81.3 | 60.7 |
| 101 | 0.368 (1.0) | 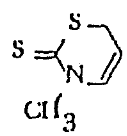 ※-b) 0.16 (1.1) | $CH_2Cl_2$ (2) | r.t. | 3 Days | 79.8 | 115~117(d) | 63.3 | 85.6 |
| 102 | 0.48 (1.3) | 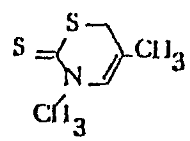 ※-b) 0.23 (1.44) | acetone (3) | r.t. | 2 Days | 92.0 | 134~136 | 69.9 | 94.0 |

b) prepared according to the method disclosed in Garraway JCS 4004-4010 (1964)

Table9 ( No. 3 )

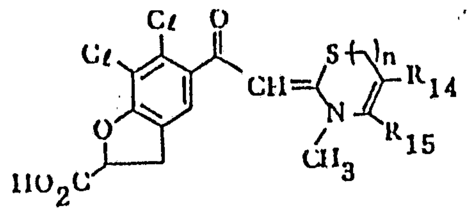

$[I]_{100\sim102}$

| Example Nos. | IR ( $\nu_{max}^{Nujol}$ $cm^{-1}$ ) | NMR ( $\delta_{ppm}^{DMSO d-6}$ ) |
|---|---|---|
| 100 | ~2500(br) ~1900~(br) 1747, 1605, 1554 | 7.9~7.1 (5H, m) 6.30 (1H, s) 5.45 (1H, d-d) 3.9~3.2 (5H, m+s) |
| 101 | 3200~2300, ~1900~(br) ~1760(br), 1664, 1607, ~1530(br) | 7.30 (1H, s like) 6.35 (1H, d) 5.84 (1H, s) 5.5~5.1 (2H, m) 3.83~3.15 (7H, m+s) |
| 102 | 3200~1800(br), 1730, 1604, 1540 | 7.29 (1H, s like) 6.18 (1H, br) 5.77 (1H, s) 3.84~3.13 (7H, m+s) 1.78 (3H, s) |

Table 9 ( No. 4 )

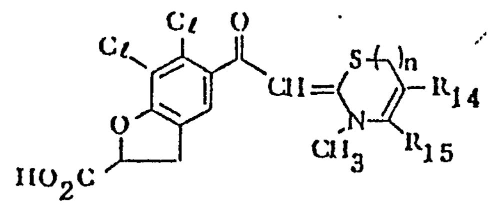

(I)100~102

| Exa. Nos. | Recrystal from | m.p. (℃) | Molecular formula | Elementary Analysis Calcd. (%) C | H | Cl | N | S | Found (%) C | H | Cl | N | S |
|---|---|---|---|---|---|---|---|---|---|---|---|---|---|
| 100 | DMF-ethanol | 283~285(d) | $C_{19}H_{13}Cl_2NO_4S$ | 54.04 | 3.10 | 16.79 | 3.82 | 7.59 | 53.67 | 3.37 | 16.74 | 3.54 | 7.41 |
| 101 | DMF-ethanol | 224~227(d) | $C_{16}H_{13}Cl_2NO_4S$ | 49.75 | 3.39 | 18.36 | 3.63 | 8.30 | 49.71 | 3.49 | 18.35 | 3.77 | 8.00 |
| 102 | DMF-ethanol | 219~221(d) | $C_{17}H_{15}Cl_2NO_4S$ | 51.01 | 3.78 | 17.71 | 3.50 | 8.01 | 50.80 | 3.89 | 17.69 | 3.58 | 7.91 |

## Effect of the Invention

Compounds prepared in Examples above are evaluated by the following pharmacological test.

I. Test Method:

Experiments with rats and mice were carried out according to Assay Programs #27-104 and #27-106, respectively. The outline is as follows.

1. Bioassay for Diuretic Effect on Rats

*Slc:SD* 8-week-old rats (male, about 250g body-weight each) were used for the test. A few lumps of sugar in place of ordinary diets were given on the morning of the day before the test day and 5% glucose solution was given orally at a rate of 20 ml/kg in the evening (approximately at 4 p.m.) of the test day. In the morning for the test, a sample which was prepared by suspending or dissolving a test compound in 2% gum arabic was orally administered to each at a dose of 20 ml/kg. On the other hand, mere by 2% gum arabic was orally administered to the control group at 20 ml/kg. Immediately after the administration, the test animals were put in a plastic cage for the metabolic tests and their urine samples were collected for 5 hours. The cumulative urine volume, urinary sodium ($Na^+$), and urinary potassium ($K^+$) were quantitatively determined.

2. Bioassay for Diuretic Effect on Mice

*Slc:ddy* 5-week-old mice (female, about 20g body-weight each) were used for the test. From the morning of the day before the test day, the mice were fasted but water. In the morning of the test day, a sample which was prepared by suspending or dissolving a test compound in 2% gum arabic was orally administered to each at 30 ml/kg. On the other hand, mere by 2% gum arabic was orally administered to the control group at 30 ml/kg. Immediately

after the administration, 5 mice employed were put in a plastic cage for the metabolic tests and their urine samples were collected for 4 hours. The cumulative urine volume, urinary sodium ($Na^+$), and urinary potassium ($K^+$) were quantitatively determined.

Ⅱ. Test Results

Results on some typical compounds are shown in Table 10.

Results regarding the urine volume are shown by percentages to control (100%). Also, results regarding the urinary sodium ($Na^+$) and the urinary potassium ($K^+$) are shown by percentages to control (100%). The asterisk * indicates that the compounds are recognized to be significantly effective.

Table 10

| Example Nos. | Structural formula of Test Compound | Rat Dose (mg) | Rat Urine Vol. (%) | Rat Urinary $Na^+$ (%) | Rat Urinary $K^+$ (%) | Mouse Dose (mg) | Mouse Urine Vol. (%) | Mouse Urinary $Na^+$ (%) | Mouse Urinary $K^+$ (%) |
|---|---|---|---|---|---|---|---|---|---|
| 1 | 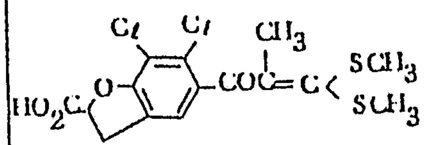 | 30<br>50<br>100 | 111<br>139※<br>162※ | 236※<br>492※<br>570※ | 143<br>236※<br>450※ | 3<br>30 | 117<br>324※ | 183※<br>1000※ | 121<br>262※ |
| 3 | 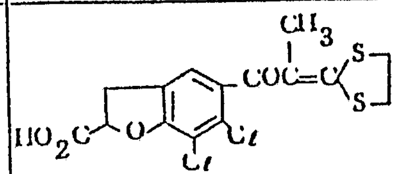 | 50 | 153※ | 291※ | 217※ | 30 | 270※ | 1370※ | 304※ |
| 14 | 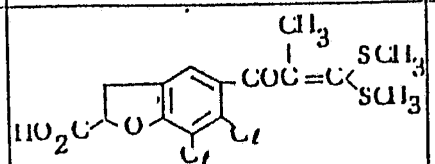 | – | – | – | – | 30 | 296※ | 743※ | 330※ |
| 17 | 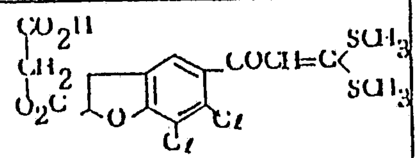 | 50 | 144※ | 478※ | 285※ | 30 | 216※ | 970※ | 277※ |
| 21 | 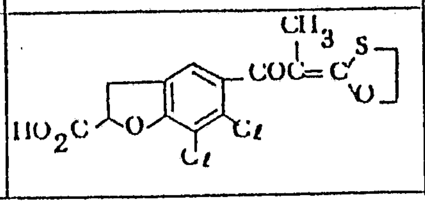 | 50 | 104 | 179※ | 178※ | 30 | 155※ | 704※ | 273※ |

Table 1 0 (Continued)

| Example Nos. | Structural formula of Test Compound | Rat | | | | Mouse | | | |
|---|---|---|---|---|---|---|---|---|---|
| | | Dose (mg) | Urine Vol. (%) | Urinary $Na^+$ (%) | Urinary $K^+$ (%) | Dose (mg) | Urine Vol. (%) | Urinary $Na^+$ (%) | Urinary $K^+$ |
| 28 | 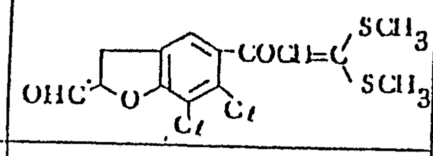 | | | | | 30 | 222※ | 905※ | 260※ |
| 67 | 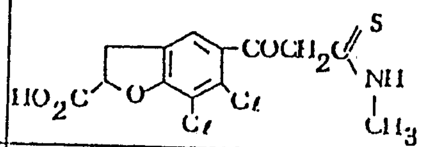 | 50 | 148※ | 377※ | 306※ | 30 | 192※ | 610※ | 232※ |
| 70 | 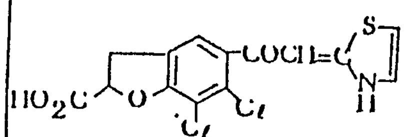 | 50 | 154※ | 459※ | 374※ | 30 | 144※ | 410※ | 178※ |
| 80 | 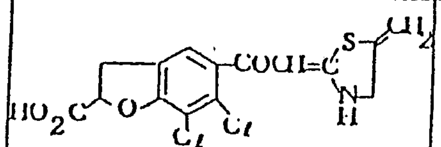 | 10 | 101 | 211※ | 161※ | 30 | 264※ | 738※ | 344※ |
| 98 | 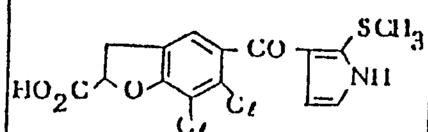 | 10 | 157※ | 445※ | 254※ | 30 | 222※ | 810※ | 292※ |

What is claimed is:

1. A compound of the following formula ( I ), or the permissible salts thereof:

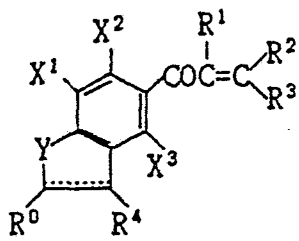

( I )

wherein $X^1$, $X^2$, and $X^3$ each is hydrogen, halogen, or $CH_3$; Y is an atom O or S; $R^1$ is hydrogen, alkyl, alkenyl, aryl, aralkyl, or alkoxycarbonyl; $R^2$ is $SR^5$, $OR^6$, or $NR^7R^8$,

wherein $R^5$ is hydrogen, alkyl, alkenyl, alkynyl, aryl, aralkyl, or carbamoylmethyl, $R^6$ is alkyl, $R^7$ and $R^8$ each is hydrogen, alkyl, alkenyl, cycloalkyl, aryl, furylmethyl, or acyl where $R^7$ and $R^8$ taken together with the adjacent nitrogen atom may form pyrrolidino, piperidino, or morpholino or one of $R^7$ and $R^8$ is hydrogen and the other is $-C(O)R^{22}$ where $R^{22}$ is alkyl, substituted alkyl, alkylene or substituted alkylene;

$R^3$ is $SR^9$ or $S(O)R^{10}$,

wherein $R^9$ is hydrogen, alkyl, alkenyl, alkynyl, or aralkyl and $R^{10}$ is alkyl;

$R^4$ is hydrogen or alkyl; $R^0$ is CHO, $COCH_3$, $COOCH_2COOH$, CN, CH=NOH, $COOR^{17}$, $CH_2OR^{18}$, $CONR^{19}R^{20}$, or $CH_2OC(O)-CH_2R^{21}$,

wherein $R^{17}$ is hydrogen, alkali metal, or alkyl, $R^{18}$ is hydrogen, alkyl, or acyl, $R^{19}$ and $R^{20}$ each is hydrogen or alkyl, $R^{21}$ is hydrogen or lower alkyl where $R^{19}$ and $R^{20}$ may

form pyrrolidino together with the adjacent nitrogen atom;

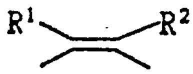 may be 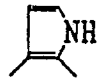 ; and,

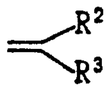 may be any one of the followings:

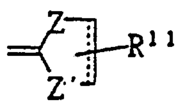 , 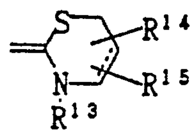 , and 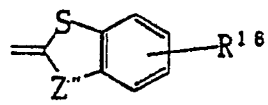

wherein Z is O, S, or NH, Z' is S or N-$R^{12}$, Z" is S, NH, or N-$CH_3$, $R^{11}$ is hydrogen, alkyl, alkoxy, carbonyl, or methylene, $R^{12}$, $R^{13}$, $R^{14}$, and $R^{16}$ each is hydrogen or alkyl, $R^{15}$ is hydrogen or carbonyl, and the dotted line shows the presence or absence of a double bond.

2. A compound as claimed in claim 1 and having the formula as given in example 1, 3, 14, 17, 21, 28, 67, 70, 80 or 98.

3. Use of a compound of formula (I) for the manufacture of a medicament having antihypertensive and diuretic activities.

European Patent Office

0189596

Application number

## EUROPEAN SEARCH REPORT

EP 85116616.5

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (Int. Cl.4) |
| A | DE - B2 - 1 927 452 (CIBA-GEIGY AG)<br>* Claim 1; column 2, lines 24-33 *<br>-- | 1,3 | C 07 D 307/80<br>C 07 D 307/85<br>C 07 D 333/52<br>C 07 D 405/06<br>C 07 D 407/06<br>C 07 D 411/06<br>C 07 D 417/06<br>A 61 K 31/34<br>A 61 K 31/38 |
| A | DE - A1 - 2 635 064 (KAKENYAKU KAKO CO)<br>* Claim 1 *<br>-- | 1 | |
| A | US - A - 3 674 810 (ZER GENYI et al.)<br>* Abstract; examples; column 1, lines 30-39 *<br>---- | 1,3 | |
| | | | TECHNICAL FIELDS SEARCHED (Int. Cl.4) |
| | | | C 07 D 307/00<br>C 07 D 333/00<br>C 07 D 405/00<br>C 07 D 407/00<br>C 07 D 411/00<br>C 07 D 417/00 |

The present search report has been drawn up for all claims

| Place of search | Date of completion of the search | Examiner |
|---|---|---|
| VIENNA | 03-04-1986 | BRUS |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82